(19) Europäisches Patentamt
European Patent Office
Office européen des brevets

(11) EP 2 952 585 A1

(12) **EUROPEAN PATENT APPLICATION**

(43) Date of publication:
**09.12.2015 Bulletin 2015/50**

(51) Int Cl.:
***C12N 15/67*** *(2006.01)* ***C12N 15/81*** *(2006.01)*

(21) Application number: **14190469.8**

(22) Date of filing: **27.10.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **04.06.2014 EP 14171146**

(71) Applicants:
- **Boehringer Ingelheim RCV GmbH & Co KG**
  **1121 Wien (AT)**
- **SANDOZ AG**
  **4056 Basel (CH)**
- **VTU Technology GmbH**
  **8074 Grambach (AT)**
- **BIOMIN Holding GmbH**
  **3130 Herzogenburg (AT)**
- **Lonza Ltd**
  **3930 Visp (CH)**

(72) Inventors:
- **Gasser, Brigitte**
  **1050 Wien (AT)**
- **Mattanovich, Diethard**
  **1120 Wien (AT)**
- **Sauer, Michael**
  **1040 Wien (AT)**
- **Russmayer, Hannes**
  **4560 Kirchdorf (AT)**

(74) Representative: **Schiweck, Weinzierl & Koch**
**European Patent Attorneys**
**Landsberger Straße 98**
**80339 München (DE)**

(54) **Improved protein production in fungi or yeasts**

(57) The present invention is in the field of recombinant biotechnology, in particular in the field of protein expression. The invention generally relates to a method of expressing a protein of interest (POI) from a host cell by overexpressing a polynucleotide encoding a protein which is involved in one or more proteinogenic amino acid biosynthesis pathways, thereby increasing the yield of a protein of interest. The invention relates particularly to improving a host cell's capacity to express and/or secrete a protein of interest and use of the host cell for protein expression. The invention also relates to cell culture technology, and more specifically to culturing cells to produce desired molecules for medical purposes or food products.

EP 2 952 585 A1

## Description

### Field of Invention

The present invention is in the field of recombinant biotechnology, in particular in the field of protein expression. The invention generally relates to a method of expressing a protein of interest (POI) from a host cell by overexpressing a polynucleotide encoding a protein which is involved in one or more proteinogenic amino acid biosynthesis pathways, thereby increasing the yield of a protein of interest. The invention relates particularly to improving a host cell's capacity to express and/or secrete a protein of interest and use of the host cell for protein expression. The invention also relates to cell culture technology, and more specifically to culturing cells to produce desired molecules for medical purposes or food products.

### Background of the Invention

Successful production of proteins of interest (POI) has been accomplished both with prokaryotic and eukaryotic hosts. The most prominent examples are bacteria like *Escherichia coli,* yeasts like *Saccharomyces cerevisiae, Pichia pastoris* or *Hansenula polymorpha,* filamentous fungi like *Aspergillus awamori* or *Trichoderma reesei,* or mammalian cells like CHO cells. While the yield of some proteins is readily achieved at high rates, many other proteins are only produced at comparatively low levels.

Generally, heterologous protein synthesis may be limited at different levels. Potential limits are transcription and translation, protein folding and, if applicable, secretion, disulfide bridge formation and glycosylation, as well as aggregation and degradation of the target proteins. Transcription can be enhanced by utilizing strong promoters or increasing the copy number of the heterologous gene. However, these measures clearly reach a plateau, indication that other bottlenecks downstream of transcription limit expression. While issues of protein folding and posttranslational modifications have been studied in great detail both for prokaryotic hosts and eukaryotic hosts, there is still a lack of knowledge on the impact of amino acid synthesis rates on the potential limitation of heterologous protein production capacity.

It has been observed in numerous cases that the expression of heterologous proteins in microorganisms decreases growth and biomass yield, even when the absolute amounts of the produced protein is quantitatively negligible in relation to biomass and cellular protein synthesized (e.g. Heyland et al 2010. Biotechnol Bioeng. 107(2):357-68; Marx et al. 2009. FEMS Yeast Res. 9(8):1260-70.). Especially when producing secreted proteins with yeasts, the total amounts of product are usually very low compared to the produced cellular protein, but still lead to a significant burden on the host cells. In order to understand the metabolic processes behind this phenomenon on a quantitative level, the flux rates of substrate (carbon and energy source) to biomass, energy production and redox equivalents were elucidated using metabolic flux analysis employing isotope labelling, in combination with quantitative analysis of specific metabolites and co-factors (Guerrasio et al. 2014. Anal Bioanal Chem. 406(3):915-22.; Klavins et al. 2013. Anal Bioanal Chem. 405(15):5159-69.; Guerrasio et al. 2013. Anal Bioanal Chem. 405(15):5133-46; Neubauer et al. 2012. J Sep Sci. 35(22):3091-105.)

The limitation of the synthesis capacity of building blocks such as amino acids has been largely neglected in yeast research so far. It can be assumed that this question is of special relevance as production processes with yeasts (but also bacteria) are preferentially performed in mineral media without any external amino acids source. Limitations of amino acids will at the first instance occur when heterologous proteins containing a high fraction of amino acids with low abundance in the biomass are overexpressed. Especially cystein is extremely rare in native *P. pastoris* proteins, and occurs at a much higher frequency in many recombinant proteins.

Moreover, high level of protein yield in host cells may be limited at one or more different steps, like folding, disulfide bond formation, glycosylation, transport within the cell, or release from the cell. Many of the mechanisms involved are still not fully understood and cannot be predicted on the basis of the current knowledge of the state-of-the-art, even when the DNA sequence of the entire genome of a host organism is available. Moreover, the phenotype of cells producing recombinant proteins in high yields can be decreased growth rate, decreased biomass formation and overall decreased cell fitness.

There is thus a constant need for methods to improve a host cell's capacity to produce and/or secrete proteins of interest. One object of the invention is to provide new methods and uses to increase the yield of recombinant proteins in host cells which are simple and efficient and suitable for use in industrial methods. It is another object to provide host cells to achieve this purpose.

It must be noted that as used herein, the singular forms "a", "an" and "the" include plural references and vice versa unless the context clearly indicates otherwise. Thus, for example, a reference to "a host cell" or "a method" includes one or more of such host cells or methods, respectively, and a reference to "the method" includes equivalent steps and methods that could be modified or substituted known to those of ordinary skill in the art. Similarly, for example, a reference to "methods" or "host cells" includes "a host cell" or "a method", respectively.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

The term "and/or" wherever used herein includes the meaning of "and", "or" and "all or any other combination of the elements connected by said term". For example, A, B and/or C means A, B, C, A+B, A+C, B+C and A+B+C.

The term "about" or "approximately" as used herein means within 20%, preferably within 10%, and more preferably within 5% of a given value or range. It includes also the concrete number, e.g., about 20 includes 20.

The term "less than", "more than" or "larger than" includes the concrete number. For example, less than 20 means ≤20 and more than 20 means ≥20.

Throughout this specification and the claims or items, unless the context requires otherwise, the word "comprise" and variations such as "comprises" and "comprising" will be understood to imply the inclusion of a stated integer (or step) or group of integers (or steps). It does not exclude any other integer (or step) or group of integers (or steps). When used herein, the term "comprising" can be substituted with "containing", "composed of", "including", "having" or "carrying." When used herein, "consisting of" excludes any integer or step not specified in the claim/item. When used herein, "consisting essentially of" does not exclude integers or steps that do not materially affect the basic and novel characteristics of the claim/item. In each instance herein any of the terms "comprising", "consisting essentially of" and "consisting of" may be replaced with either of the other two terms.

Further, in describing representative embodiments of the present invention, the specification may have presented the method and/or process of the present invention as a particular sequence of steps. However, to the extent that the method or process does not rely on the particular order of steps set forth herein, the method or process should not be limited to the particular sequence of steps described. As one of ordinary skill in the art would appreciate, other sequences of steps may be possible. Therefore, the particular order of the steps set forth in the specification should not be construed as limitations on the claims. In addition, the claims directed to the method and/or process of the present invention should not be limited to the performance of their steps in the order written, and one skilled in the art can readily appreciate that the sequences may be varied and still remain within the spirit and scope of the present invention.

It should be understood that this invention is not limited to the particular methodology, protocols, material, reagents, and substances, etc., described herein. The terminologies used herein are for the purpose of describing particular embodiments only and are not intended to limit the scope of the present invention, which is defined solely by the claims/items.

All publications and patents cited throughout the text of this specification (including all patents, patent applications, scientific publications, manufacturer's specifications, instructions, etc.), whether supra or infra, are hereby incorporated by reference in their entirety. Nothing herein is to be construed as an admission that the invention is not entitled to antedate such disclosure by virtue of prior invention. To the extent the material incorporated by reference contradicts or is inconsistent with this specification, the specification will supersede any such material.

## Summary

The present invention is based on the surprising findings of polynucleotide sequences ("polynucleotides of the present invention") encoding a protein which is involved in one or more proteinogenic amino acid biosynthesis pathways ("helper protein of the present invention") whose expression, preferably overexpression led to an increase in the yield of protein of interest (POI). This disclosure provides methods and materials useful for improving the yield of POI by engineering host cells such that they are capable of overexpressing one or more proteins which are involved in one or more proteinogenic amino acid biosynthesis pathways. Preferred polynucleotides encode a helper protein (involved in one or more proteinogenic amino acid biosynthesis pathways) having an amino acid sequence as shown in any one of SEQ ID NOs: 1-14, or a functional homologue thereof, wherein the functional homologue has at least 30% sequence identity to an amino acid sequence as shown in any one of SEQ ID NOs: 1-14.

This finding was rather surprising, since the the external supplementation of amino acids rather than improving the growth rate of a yeast producer strain and thus the yield of two different model proteins, decreased its growth rate and thus the yield of the two model proteins. One model protein is the enzyme carboxylesterase (CES) of *Sphingopyxis macrogoltabida* (Genbank Accession No. ACS27056, SEQ ID No. 29), in particular amino acids 48-540 thereof, the second model protein is the antibody Fab fragment of HyHEL (HyHEL-Fab, heavy and light chain shown in SEQ ID Nos. 30 and 31). In fact, a successful approach in external supplementation of amino acids to producer strains is described in Heyland et al. (2011), Biotechnol Bioeng. 108(8):1942-53. Heyland et al. quantified the impact of low to high production of intracellular β-aminopeptidase BapA on cell physiology of *P. pastoris.* BapA producing strains had reduced growth rate, reduced byproduct formation and increased TCA-cycle flux, which was thought to compensate for the additional resources needed for recombinant protein synthesis. Similar results were also obtained for other model proteins later on (e.g. Jorda et al. 2012. Microb Cell Fact. 11:57; Nocon et al. 2014. Metab Eng. doi 10.1016/j.ymben.2014.05.011). Addition of amino acids (all 20 proteinogenic or combinations thereof) to the growth medium of BapA producing strains could partially restore the reduced growth, thus increasing the rate of BapA synthesis up to twofold. However, the present inventors did not observe positive results when following the teaching of Heyland et al.

Thus, it came as a surprise when the present inventors, despite the discouraging experience with external supplementation of amino acids to producer strains with the aim of increasing bio mass and thus product yield, overexpressed polynucleotides encoding a protein which is involved in one or more proteinogenic amino acid biosynthesis pathways and observed that the yield of a protein of interest in a host cell increased. In fact, in view of the unsuccessful attempts to increase the yield of a protein of interest by external supplementation of amino acids, it could not at all have been expected that increasing the "internal" (i.e. intracellular) level/concentration of amino acids will result in increased biomass and thus increased yield of a protein of interest. This is true all the more for fungal or yeast cells, since it could not at all have been expected that a helper protein of the present invention is capable of increasing the yield of a protein of interest, exemplified herein by the model protein CES. In fact, though attempts were made in E. coli host cells to increase the yield of a serine-rich protein by co-expressing cystein synthase A (Han et al. (2003), Appl Env Microbiol 69(10): 5772-5781), it was neither possible nor reasonable to consider such an approach in a fungal or yeast cell. In fact, fungal or yeast cells do not have a gene encoding cysteine synthase A and a conclusion from bacterial to fungal or yeast cells is neither plausible not reasonable when bearing in mind that the yield of a protein of interest should be increased in a fungal or yeast cell. Namely, a skilled artisan could not and would not have thought that what may be possible in E. coli will also be possible in fungal or yeast cells and this all the more since yeast or fungal cells do not haave a cysteine synthase A.

That being said, even though Heyland et al. were successful with external amino acid supplementation of a producer strain, it could not at all have been expected that the overexpression of one or more polynucleotides encoding a protein which is involved in one or more proteinogenic amino acid biosynthesis pathways in fact increases the yield of protein of interest (POI), since external supplementation does not allow one to extrapolate to "internal" supplementation. This is so because it can, for example, not be predicted which effect overexpression of a polynucleotide may have and, all the more, wherther it would contribute to increasing the yield of a protein of interest.

The term "yield" refers to the amount of POI or model protein(s) as described herein, in particular CES (SEQ ID No. 29) which is, for example, harvested from the engineered host cell, and increased yields can be due to increased amounts of production or secretion of the POI by the host cell. Yield may be presented by mg POI/g biomass (measured as dry cell weight or wet cell weight) of a host cell. The term "titer" when used herein refers similarly to the amount of produced POI or model protein, presented as mg POI/L culture supernatant. An increase in yield can be determined when the yield obtained from an engineered host cell is compared to the yield obtained from a host cell prior to engineering, i.e., from a non-engineered host cell. Other model proteins that can be used in the context of the present invention are HyHel-Fab (SEQ ID No. 30 and 31) and/or SDZ-Fab (SEQ ID No. 32 and 33).

Preferably, "yield" when used herein in the context of a model protein as described herein, is determined as described in Example 2d for model proteins HyHEI-Fab and SDZ-Fab and in Example 2e for model protein CES. Accordingly, the "yield" when used herein in the context of the model proteins HyHEL-Fab and SDZ-Fab, respectively, as described herein is also referred to as "Fab yield" or "Fab titer". A Fab titer is given as mg/L and a Fab yield as mg/g biomass (measured as dry cell weight or wet cell weight). The "yield" when used herein in the context of the model protein CES as described herein is also referred to as relative yield or fold change yield ("FC yield" or "FC titer"). A FC titer and a FC yield are dimensionless, as they are given as yield or titer of the co-overexpressing strain devided by yield or titer of the protein overproducing strain prior to engineering.

Briefly, the *P. pastoris* protein overproducing strains CBS7435mut$^s$ pAOX HyHEL-Fab, CBS pPM2d_pGAP HyHEL and CBS7435 pPM2d_pGAP CES were used as host strains for co-overexpression (see Example 4b). For co-overexpression, the gene encoding a helper protein is cloned under control of the *P. pastoris* GAP promoter and transformed into the CES producing strain as described in examples 4a and 4b. Engineered cells are cultivated as described in Example 2b. The amount of secreted CES is analysed with western Blot as described in Example 2c and ELISA as described in Example 2e. In particular, biomass is determined by measuring the weight of the cell pellet derived from 1 mL cell suspension. An increase in the yield may be determined based on a comparison of POI yield before and after the cell is engineered to overexpress the polypeptide. A standard test involving model protein CES as shown in the example may be used to determine the yield difference.

In a first aspect, the present invention relates to a method of increasing the yield of a protein of interest in a host cell, comprising overexpressing a polynucleotide encoding a protein which is involved in one or more proteinogenic amino acid biosynthesis pathways, thereby increasing the yield of a protein of interest. Such a protein which is involved in one or more proteinogenic amino acid biosynthesis pathways is also referred to herein as "helper protein of the present invention".

In a second aspect, the present invention relates to a method of increasing the yield of a protein of interest in a host cell, comprising overexpressing a polynucleotide encoding a protein which is involved in one or more proteinogenic amino acid biosynthesis pathways, preferably a polynucleotide encoding a helper protein having an amino acid sequence as shown in any one of SEQ ID NOs: 1-14, or a functional homologue thereof, wherein the functional homologue has at least 30% sequence identity to an amino acid sequence as shown in any one of SEQ ID NOs: 1-14.

In a third aspect, the present invention relates to a method of increasing the yield of a protein of interest in a host cell, comprising

- engineering the host cell to overexpress a polynucleotide encoding a protein which is involved in one or more proteinogenic amino acid biosynthesis pathways, preferably a polynucleotide encoding a helper protein having an amino acid sequence as shown in any one of SEQ ID NOs: 1-14 or a functional homologue thereof, wherein the functional homologue has at least 30% sequence identity to an amino acid sequence as shown in any one of SEQ ID NOs: 1-14,

- recombining in said host cell a heterologous polynucleotide encoding the protein of interest,

- culturing said host cell under suitable conditions to overexpress the helper protein or functional homologue thereof and the protein of interest, and optionally

- isolating the protein of interest from the cell culture.

In the context of methods for increasing the yield of a protein the order of the "engineering step" and "recombining step" can alternatively be reversed such that the "recombining step" precedes the "engineering step". Notably, as described herein, the yield of a protein of interest is increased when a helper protein is overexpressed.

In a fourth aspect, the present invention relates to a method of manufacturing a protein of interest in a host cell comprising

- providing the host cell engineered to overexpress a polynucleotide encoding a protein which is involved in one or more proteinogenic amino acid biosynthesis pathways, preferably a polynucleotide encoding a helper protein having an amino acid sequence as shown in any one of SEQ ID NOs: 1-14 or a functional homologue thereof, wherein the functional homologue has at least 30% sequence identity to an amino acid sequence as shown in any one of SEQ ID NOs: 1-14, wherein said host cell comprises a heterologous polynucleotide encoding a protein of interest;

- culturing the host cell under suitable conditions to overexpress the helper protein or functional homologue thereof and express the protein of interest, and optionally

- isolating the protein of interest from the cell culture.

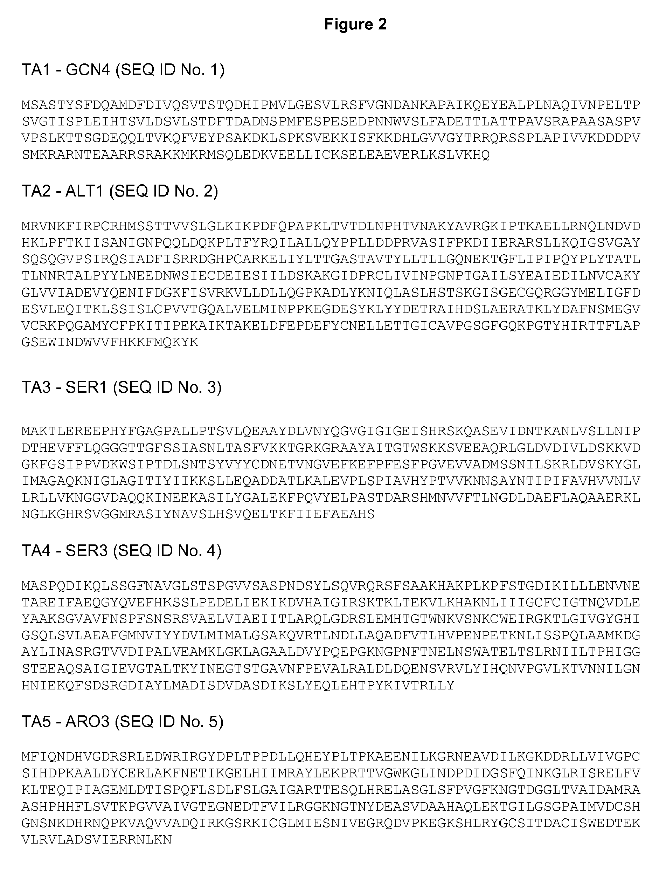

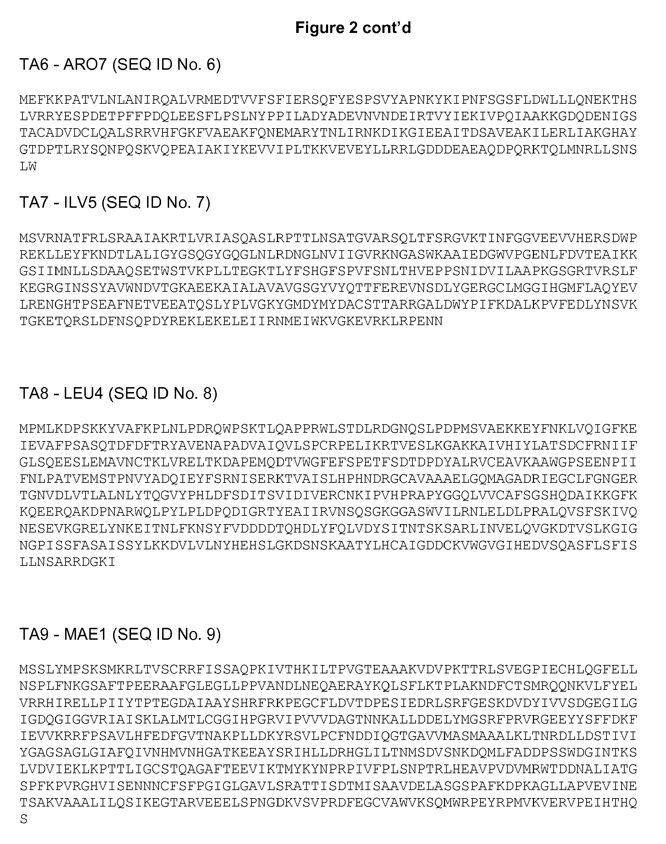

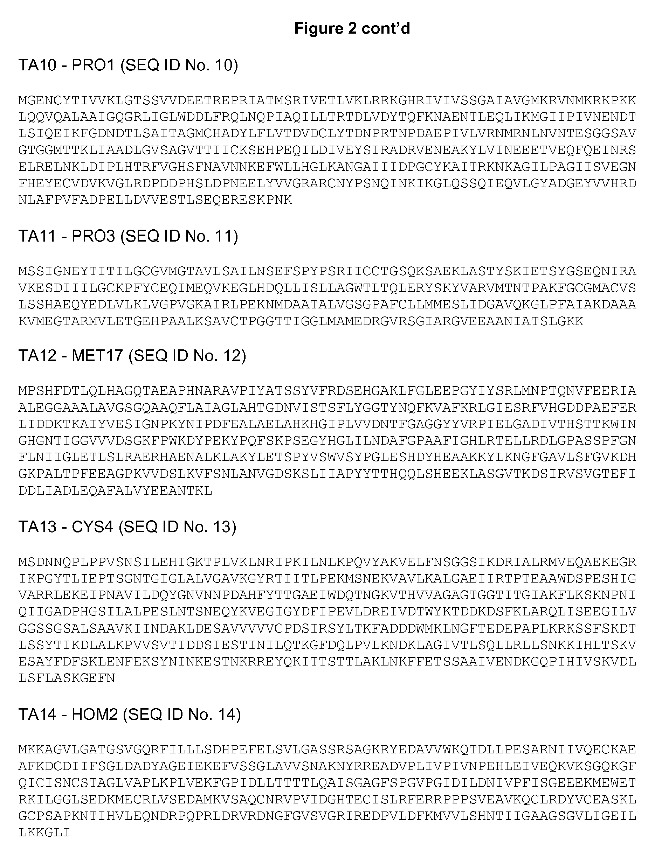

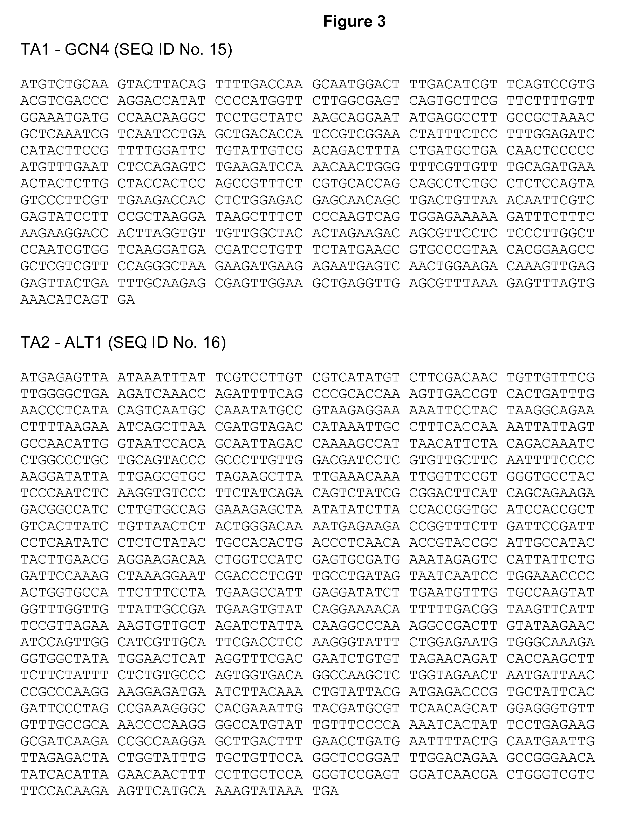

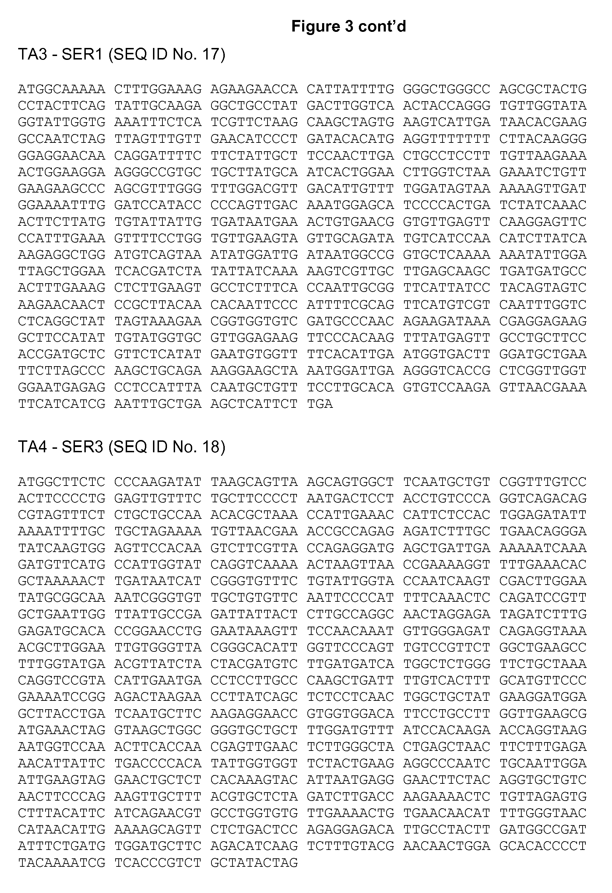

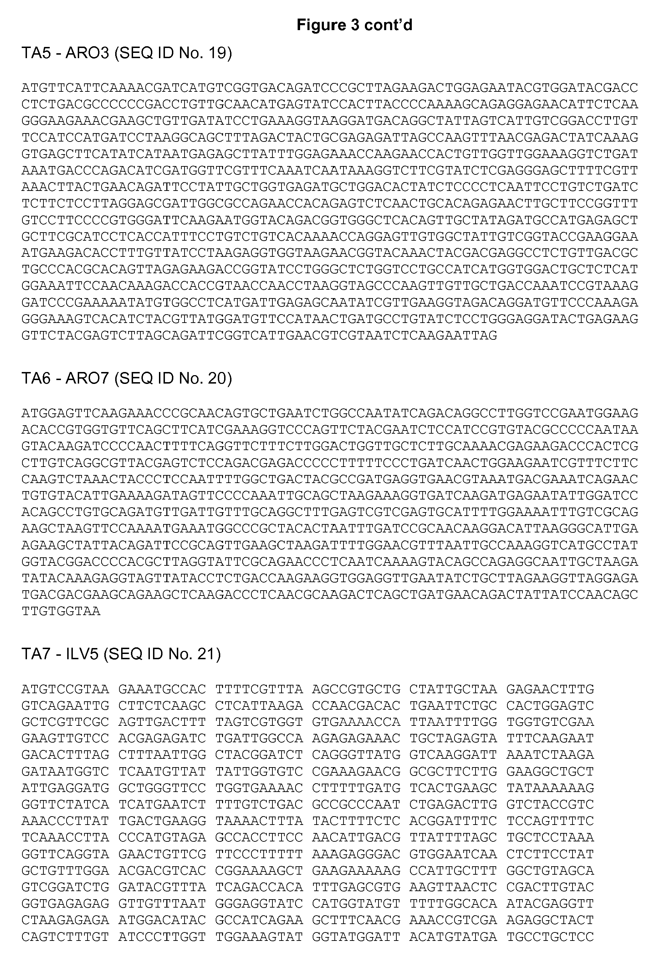

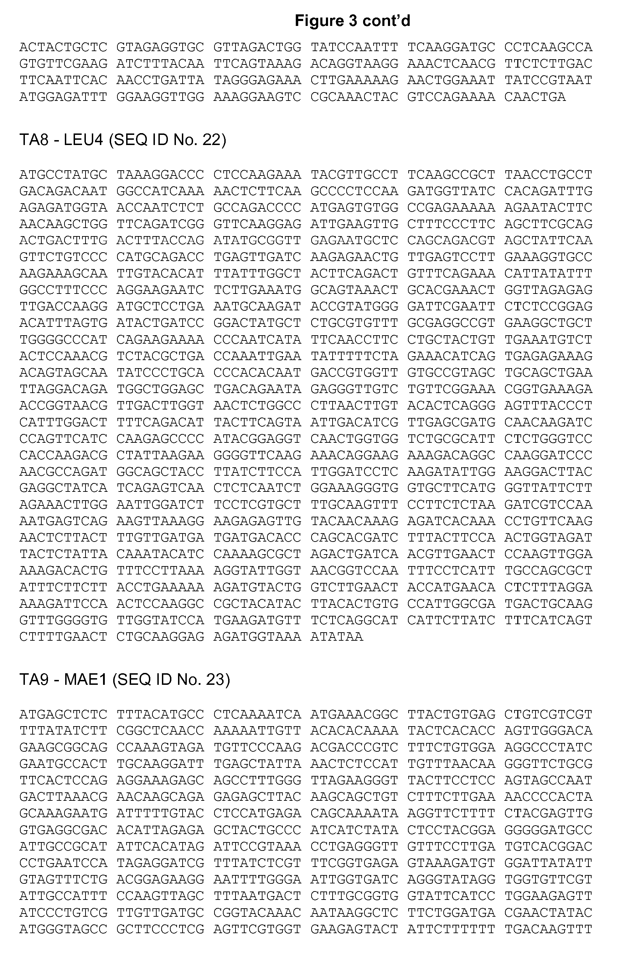

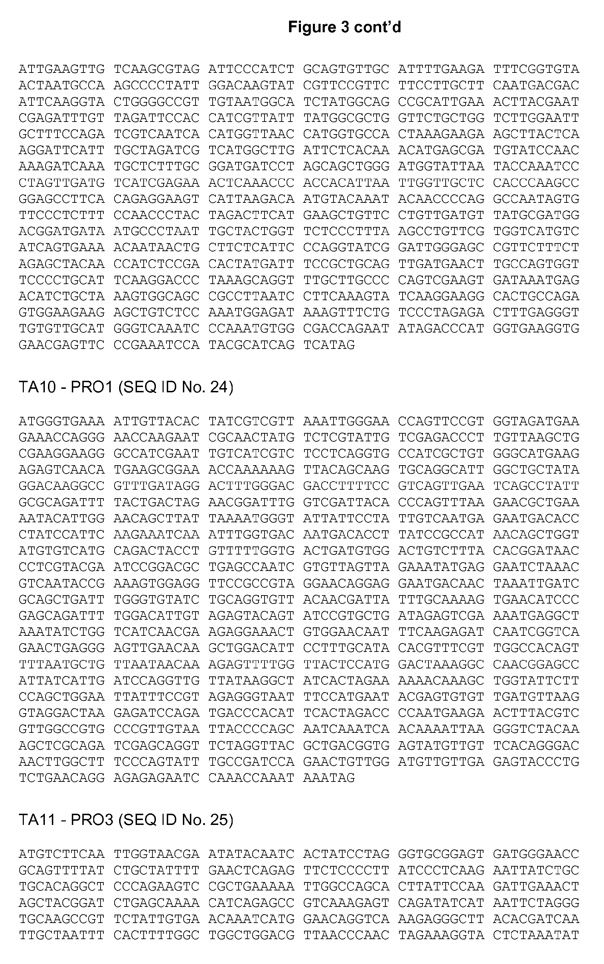

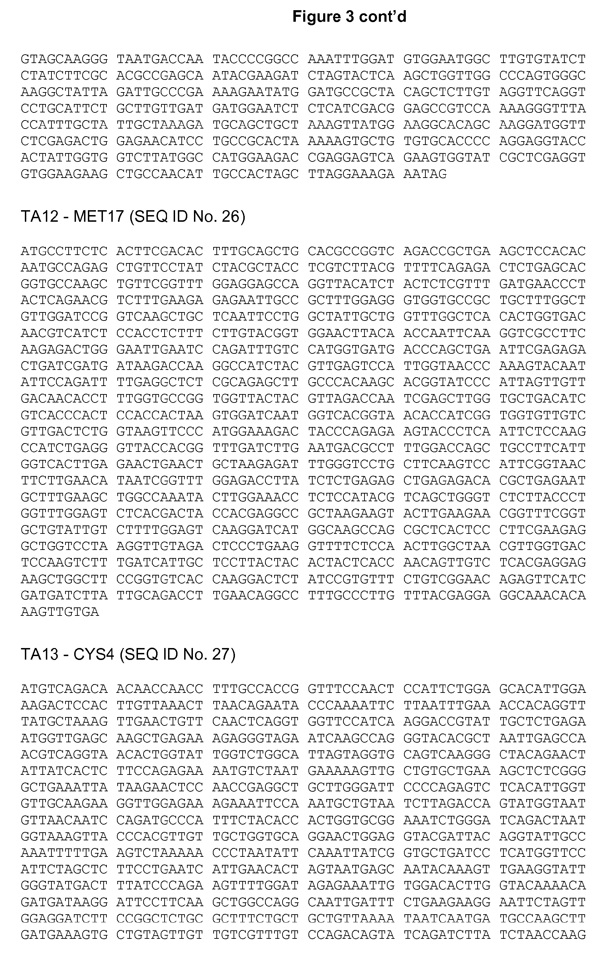

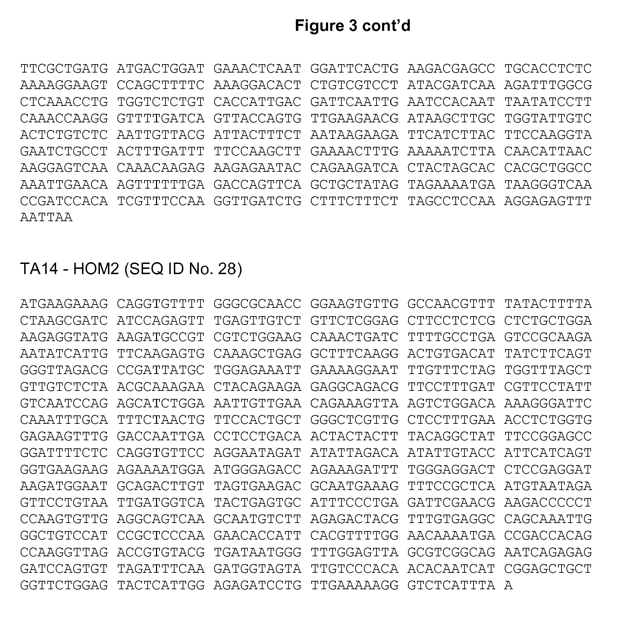

As noted above, the present invention is partly based on newly discovered helper proteins which are involved in one or more proteinogenic amino acid biosynthesis pathways and their use to increase POI yield. The present invention is based on, but not limited to, the helper protein TA1 to TA14 (see Tables 2a and 2b as well as Figures 2 and 3) or functional homologues thereof. The meaning of functional homologue is defined in the latter part of the application. The amino acid sequences of the helper proteins TA1 to TA14 are listed respectively in SEQ ID NO: 1-14. The nucleotide sequence encoding the helper proteins TA1 to TA14 are listed respectively in SEQ ID NO: 15-28. As used herein, such polynucleotides/nucleotide sequences or amino acid sequences/proteins are referred to in the present invention interchangeably in plural or singular forms, which however should be understood as in singular form unless expressly stated otherwise.

In a fifth aspect, the present invention provides a recombinant host cell for manufacturing a protein of interest, wherein the host cell is engineered to overexpress a polynucleotide encoding a protein which is involved in one or more proteinogenic amino acid biosynthesis pathways

In a further aspect, the present inventon provides a recombinant host cell for manufacturing a protein of interest, wherein the host cell is engineered to overexpress a polynucleotide encoding a helper protein (involved in one or more proteinogenic amino acid biosynthesis pathways) having an amino acid sequence as shown in SEQ ID NO: 1, 2, 3, 4, 5, 6, 7, 8, 9, 10, 11, 12, 13, or 14 or a functional homologue thereof, wherein the functional homologue has at least 30%, such as at least 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or 100% sequence identity to an amino acid sequence as shown in SEQ ID NO: 1, 2, 3, 4, 5, 6, 7, 8, 9, 10, 11, 12, 13, or 14.

In a preferred embodiment, the helper protein, preferably when overexpressed, may increase (is capable of increasing) the yield of the model protein CES (SEQ ID No. 29) in the host cell by at least 1%, such as at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 40, 50, 60, 70, 80, 90, 100, 110, 120, 130, 140, 150, 160, 170, 180, 190 or at least 200%, compared to the host cell prior to being engineered to overexpress said helper protein. Alternative model proteins are HyHEL-Fab (SEQ ID NO: 30 for heavy chain and SEQ ID NO: 31 for light chain) or SDZ-Fab (SEQ ID NO: 32 for heavy chain and SEQ ID NO: 33 for light chain. A host cell prior to engineering does not overexpress the helper protein of the present invention and after engineering is able to overexpress the helper protein under suitable culturing conditions. It has been surprisingly found that exemplary recombinant cells described in the Examples were all able to increase (are capable of increasing) the yield of the model protein CES by at least 20% (1.2 fold change). In some instances, the yield increased by more than 100%, as shown in Example 5 (see also Table 3).

Preferably, the present invention provides a recombinant host cell for manufacturing a protein of interest, wherein the host cell is engineered to overexpress a polynucleotide encoding a helper protein having an amino acid sequence as shown in SEQ ID NO: 1, 2, 3, 4, 5, 6, 7, 8, 9, 10, 11, 12, 13, or 14, or a functional homologue thereof, wherein the functional homologue has at least 30% sequence identity to SEQ ID NO: 1, 2, 3, 4, 5, 6, 7, 8, 9, 10, 11, 12, 13, or 14, respectively, wherein the helper protein, preferably when overexpressed, may increase (is capable of increasing) the production of the model protein CES in the host cell by at least 1%, such as at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 40, 50, 60, 70, 80, 90, 100, 110, 120, 130, 140, 150, 160, 170, 180, 190 or at least 200%, compared to the host cell prior to engineering.

In a sixth aspect, the present invention provides the use of the engineered host cells for manufacturing a protein of interest. The host cells can be advantageously used for introducing polypeptides encoding one or more POI(s), and thereafter can be cultured under suitable condition to express the POI.

The invention additionally relates in a seventh aspect to polynucleotides encoding the helper proteins (hereinafter referred to as "polynucleotides of the present invention" or "a polynucleotide of the present invention") and their individual or combined use to increase POI yield. The polynucleotide(s) can be introduced into a host cell or, if already existing in the cell, manipulated in a way such that they are overexpressed. The polynucleotide of the present invention encodes any one of SEQ ID NOs. 1-14 or functional homologues thereof. Examples of the polynucleotide sequences are as set forth in SEQ ID NOs. 15-28.

The present invention provides an isolated polynucleotide sequence comprising, consisting essentially of, or consisting of SEQ ID NO: SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, or SEQ ID NO: 28 and optionally may be operably linked to a promoter which may be heterologous to said polynucleotide sequence. Furthermore, the present invention provides an isolated polynucleotide sequence which encodes a polypeptide sequence comprising any one of SEQ ID NOs. 1-14, or functional homologues thereof. Preferably, the present invention provides an isolated polynucleotide sequence which encodes a polypeptide sequence comprising SEQ ID NO: 1, 2, 3, 4, 5, 6, 7, 8, 9, 10, 11, 12, 13, or 14, or functional homologues thereof.

In some embodiments, the present invention provides an isolated polynucleotide sequence having at least 30%, such as at least 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or 100% sequence identity to a polynucleotide sequence selected from the group consisting of SEQ ID NO: 14, 15, 16, 17, 18, 19, 20, 21, 22, 23, 24, 25, 26, 27, or 28.

The invention provides the use of a polynucleotide sequence according to the invention for integration into a host cell in its chromosome or in a plasmid, mini-plasmid, YAC, BAC, cosmid, or any other vector or the like. The polynucleotide sequence according to the invention may be introduced into a host cell, e.g., via transformation or transfection. Additionally, the invention provides the use of such polynucleotide sequence in the manufacturing of a protein of interest in a host cell.

Furthermore, the present invention provides in an eighth aspect an isolated polypeptide comprising the polypeptide sequence of any one of SEQ ID NO: 1-14, or functional homologues thereof. Preferably, the present invention provides an isolated polypeptide comprising the polypeptide sequence of SEQ ID NO: 1, 2, 3, 4, 5, 6, 7, 8, 9, 10, 11, 12, 13, or 14, or functional homologues thereof. Additionally, the invention provides the use of such polypeptides for manufacturing of a protein of interest.

In a further aspect, the present invention provides an isolated polypeptide with a polypeptide sequence having at least 30%, such as at least 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100% sequence identity to an amino acid sequence selected from the group consisting of SEQ ID NO: 1, 2, 3, 4, 5, 6, 7, 8, 9, 10, 11, 12, 13, or 14.

In a ninth aspect, the present invention provides a composition comprising at least 10%, 20%, 30%, 40%, or 50% of a protein of interest and a polynucleotide as described herein, wherein said polynucleotide is operably linked with a heterologous promoter. Such a composition can be obtained as a direct product of the methods and uses of the present invention. As such, a composition directly obtained by a method or use of the present invention may comprise a polynucleotide as described herein, wherein said polynucleotide is operably linked with a heterologous promoter

Brief Description of the Drawings

FIG. 1 shows the fold changes of Fab yields of pAOX-HyHEL-Fab#8 upon addition of different amino acid combinations.

FIG. 2 shows the amino acid sequences of TA1 to TA14.

FIG. 3 shows the nucleotide sequences of TA1 to TA14.

Items of the Invention

1) A method of increasing the yield of a protein of interest in a host cell, comprising overexpressing a polynucleotide encoding a protein which is involved in one or more proteinogenic amino acid biosynthesis pathways, thereby increasing the yield of a protein of interest.

2) The method of item 1, wherein said polynucleotide encodes a helper protein having an amino acid sequence as shown in any one of SEQ ID NOs: 1-14, or a functional homologue thereof, wherein the functional homologue has at least 30% sequence identity to an amino acid sequence as shown in any one of SEQ ID NOs: 1-14.

3) The method of item 1 or 2 comprising:

- engineering the host cell to overexpress a polynucleotide encoding a protein which is involved in one or more proteinogenic amino acid biosynthesis pathways, preferably a polynucleotide encoding a helper protein having an amino acid sequence as shown in any one of SEQ ID NOs: 1-14 or a functional homologue thereof, wherein the functional homologue has at least 30% sequence identity to an amino acid sequence as shown in any one of SEQ ID NOs: 1-14,
- recombining in said host cell a heterologous polynucleotide encoding the protein of interest,
- culturing said host cell under suitable conditions to overexpress the helper protein or functional homologue thereof and the protein of interest, and optionally
- isolating the protein of interest from the cell culture.

4) The method of item 1 or 2 comprising:

- providing the host cell engineered to overexpress a polynucleotide encoding a protein which is involved in one or more proteinogenic amino acid biosynthesis pathways, preferably a polynucleotide encoding a helper protein having an amino acid sequence as shown in any one of SEQ ID NOs: 1-14 or a functional homologue thereof, wherein the functional homologue has at least 30% sequence identity to an amino acid sequence as shown in any one of SEQ ID NOs: 1-14, wherein said host cell comprises a heterologous polynucleotide encoding a protein of interest;
- culturing the host cell under suitable conditions to overexpress the helper protein or functional homologue thereof and express the protein of interest, and optionally
- isolating the protein of interest from the cell culture.

Preferably, said one or more proteinogenic amino acid biosynthesis pathways is one or more amino acid biosynthesis pathways selected from an aliphatic amino acid biosynthesis pathway leading to amino acids such as glycine, alanine, valine, leucine, isoleucine; an aromatic amino acid biosynthesis pathway leading to amino acids such as phenylalanine, tyrosine, tryptophan; a cyclic amino acid biosynthesis pathway leading to amino acids such as such as proline; a hydroxyl amino acid biosynthesis pathway leading to amino acids such as serine, threonine or tyrosine; a branched chain amino acid biosynthesis pathway leading to amino acids such as valin, leucin or isoleucine; or a sulphur-containing amino acid biosynthesis pathway leading to amino acids such as cysteine or methionine

5) The method of any one of items 1-4, wherein overexpression is achieved by having 1, 2, 3, 4 or more copies of said polynucleotide encoding the helper protein or functional homologue thereof in said host cell.

6) The method of any one of items 1-5, wherein said polynucleotide is integrated in the genome of said host cell.

7) The method of item 6, wherein the integration is ectopically and/or in the natural locus.

8) The method of item 7, wherein the polynucleotide is integrated in AOX1, GAP, ENO1, TEF, HIS4, TYR1, HIS3, LEU2, URA3, LYS2, ADE2, TRP1, GAL1, or ADH1 locus of the host cell genome.

9) The method of any one of items 1-6, wherein the polynucleotide is contained in a vector or plasmid.

10)The method of item 9, wherein the vector is Yip type vector, YEp type vector, YRp type vector, YCp type vector, pGPD-2, pAO815, pGAPZ, pGAPZ$\alpha$, pHIL-D2, pHIL-S1, pPIC3.5K, pPIC9K, pPICZ, pPICZ$\alpha$, pPIC3K, pHWO10, pPUZZLE, or 2 plasmid.

11) The method of any one of items 1-10, wherein the overexpression is achieved by using a recombinant promoter which drives expression of said polynucleotide.

12) The method of any one of items 1-10, wherein overexpression is achieved by modifying a regulatory sequence operably linked to the polynucleotide encoding the helper protein or functional homolog thereof.

13) The method of item 11, wherein the promoter is PAOX1, PTPI, PPGK, PGAPDH, PLAC, PGAL, PPGI, PGAP, PTEF, PENO1, PTPI, PRPS2, PRPS7, PRPS31, PRPL1, PFLD, PICL, PTHI, PSSA1, PHSP90, PKAR2, PGND1, PGPM1, PTKL1, PPIS1, PFET3, PFTR1, PPHO8, PNMT1, PMCM1, PUBI4, PRAD2, PPET9, PFMD, PGAL1, PADH1, PADH2/GAP, PCUP1, or PMAL.

14) The method of any one of items 1-10, wherein the overexpression of the polynucleotide is achieved by using an enhancer to enhance the promoter activity.

15) The method of item 14, wherein the enhancer is the yeast upstream activating sequence UAS/GAL.

16) The method of any one of items 1 to 15, wherein the host cell is *Pichia pastoris, Hansenula polymorpha, Trichoderma reesei, Saccharomyces cerevisiae, Kluyveromyces lactis, Yarrowia lipolytica, Pichia methanolica, Candida boidinii, Komagataella sp., Aspergillus sp. or Schizosaccharomyces pombe.*

17)The method of any one of items 1 to 16, wherein 2, 3, 4, 5, 6, 7, 8 or more of helper proteins selected from any one of SEQ ID NOs: 1-14 or functional homologues thereof are overexpressed.

18)The method of item 17, wherein said host cell overexpresses the following helper proteins

(a) helper proteins having the amino acid sequence as shown in SEQ ID NOs: 3 and 4 or a functional homologue thereof, wherein the functional homologue has at least 30% sequence identity to an amino acid sequence as shown in SEQ ID NOs: 3 and 4, respectively;

(b) helper proteins having the amino acid sequence as shown in SEQ ID NOs: 5 and 6 or a functional homologue thereof, wherein the functional homologue has at least 30% sequence identity to an amino acid sequence as shown in SEQ ID NOs: 5 and 6, respectively;

(c) helper proteins having the amino acid sequence as shown in SEQ ID NOs: 7 and 8 or a functional homologue thereof, wherein the functional homologue has at least 30% sequence identity to an amino acid sequence as shown in SEQ ID NOs: 7 and 8, respectively;

(d) helper proteins having the amino acid sequence as shown in SEQ ID NOs: 10 and 11 or a functional homologue thereof, wherein the functional homologue has at least 30% sequence identity to an amino acid sequence as shown in SEQ ID NOs: 10 and 11, respectively; or

(e) helper proteins having the amino acid sequence as shown in SEQ ID NOs: 12, 13 and 14 or a functional homologue thereof, wherein the functional homologue has at least 30% sequence identity to an amino acid sequence as shown in SEQ ID NOs: 12, 13 and 14, respectively.

19)The method of any one of items 1 to 18, wherein the protein of interest is an enzyme, a therapeutic protein, a food additive or feed additive.

20) The method of item 19, wherein the therapeutic protein comprises an antibody.

21) The method of any one of items 1-20, wherein said helper protein or functional homologue thereof increases the yield of the model protein CES (SEQ ID NO: 29) compared to the host cell prior to engineering.

22)A recombinant host cell for manufacturing a protein of interest, wherein the host cell is engineered to overexpress a polynucleotide encoding a protein which is involved in one or more proteinogenic amino acid biosynthesis pathways.

23) The host cell of item 22, wherein said polynucleotide encodes a helper protein having an amino acid sequence as shown in any one of SEQ ID NOs: 1-14 or a functional homologue thereof, wherein the functional homologue has at least 30% sequence identity to an amino acid sequence as shown in any one of SEQ ID NOs: 1-14.

24) The host cell of item 22 or 23, wherein said helper protein or said functional homologue thereof increases the yield of the model protein CES (SEQ ID NO. 29) compared to the host cell prior to engineering.

25)The host cell of any one of items 22-24, wherein the overexpression is achieved by having 1, 2, 3, 4 or more copies of said polynucleotide encoding the helper protein or functional homologue thereof in said host cell.

26)The host cell of any one items 22-25, wherein said polynucleotide is integrated in the genome of said host cell.

27) The host cell of item 26, wherein the integration is ectopically and/or in the natural locus.

28) The host cell of item 27, wherein at least one of the polynucleotides is integrated in AOX1, GAP, ENO1, TEF, HIS4, TYR1, HIS3, LEU2, URA3, LYS2, ADE2, TRP1, GAL1, or ADH1 locus of the host cell genome.

29)The host cell of any one of items 22-25, wherein the polynucleotide is contained in a vector or plasmid.

30) The host cell of item 29, wherein the vector is YIp type vector, YEp type vector, YRp type vector, YCp type vector, pGPD-2, pAO815, pGAPZ, pGAPZ$\alpha$, pHIL-D2, pHIL-S1, pPIC3.5K, pPIC9K, pPICZ, pPICZ$\alpha$, pPIC3K, pHWO10, pPUZZLE, or 2 $\mu$m plasmid.

31) The host cell of any one of items 22-30, wherein the overexpression is achieved by using a recombinant promoter which drives expression of said polynucleotide.

32)The host cell of item 31, wherein the promoter is PAOX1, PTPI, PPGK, PGAPDH, PLAC, PGAL, PPGI, PGAP, PTEF, PENO1, PTPI, PRPS2, PRPS7, PRPS31, PRPL1, PFLD, PICL, PTHI, PSSA1, PHSP90, PKAR2, PGND1, PGPM1, PTKL1, PPIS1, PFET3, PFTR1, PPHO8, PNMT1, PMCM1, PUBI4, PRAD2, PPET9, PFMD, PGAL1, PADH1, PADH2/GAP, PCUP1, or PMAL.

33) The host cell of item 31, wherein the overexpression of the polynucleotide is achieved by using an enhancer to enhance the promoter activity.

34) The host cell of item 33, wherein the enhancer is the yeast upstream activating sequence UAS/GAL.

35)The host cell of any one of items 22-34, wherein the host cell is *Pichia pastoris, Hansenula polymorpha, Trichoderma reesei, Saccharomyces cerevisiae, Kluyveromyces lactis, Yarrowia lipolytica, Pichia methanolica, Candida boidinii, Komagataella sp., Aspergillus sp.* and *Schizosaccharomyces pombe.*

36) The host cell of any one items 22-35, wherein 2, 3, 4, 5, 6, 7, 8 or more of helper proteins selected from any one of SEQ ID NOs: 1 to 14 or functional homologues thereof are overexpressed.

37) The host cell of item 36, wherein the following helper proteins are overexpressed

(a) helper proteins having the amino acid sequence as shown in SEQ ID NOs: 3 and 4 or a functional homologue thereof, wherein the functional homologue has at least 30% sequence identity to an amino acid sequence as shown in SEQ ID NOs: 3 and 4, respectively;

(b) helper proteins having the amino acid sequence as shown in SEQ ID NOs: 5 and 6 or a functional homologue thereof, wherein the functional homologue has at least 30% sequence identity to an amino acid sequence as shown in SEQ ID NOs: 5 and 6, respectively;

(c) helper proteins having the amino acid sequence as shown in SEQ ID NOs: 7 and 8 or a functional homologue thereof, wherein the functional homologue has at least 30% sequence identity to an amino acid sequence as shown in SEQ ID NOs: 7 and 8, respectively;

(d) helper proteins having the amino acid sequence as shown in SEQ ID NOs: 10 and 11 or a functional homologue thereof, wherein the functional homologue has at least 30% sequence identity to an amino acid sequence as shown in SEQ ID NOs: 10 and 11, respectively; or

(e) helper proteins having the amino acid sequence as shown in SEQ ID NOs: 12, 13 and 14 or a functional homologue thereof, wherein the functional homologue has at least 30% sequence identity to an amino acid sequence as shown in SEQ ID NOs: 12, 13 and 14, respectively.

38)The host cell of any one of items 22-38, comprising a heterologous polynucleotide sequence encoding the protein of interest.

39)The host cell of item 38, wherein the protein of interest is an enzyme, a therapeutic protein, a food additive or feed additive.

40) The host cell of item 39, wherein the therapeutic protein comprises an antibody.

41) The host cell of any one of items 22-40, wherein overexpression is achieved by modifying a regulatory sequence operably linked to the polynucleotide encoding the helper protein or functional homolog thereof.

42) Use of the host cell of any one of items 22-41 for manufacturing a protein of interest.

43)An isolated polynucleotide encoding a helper protein having an amino acid sequence as shown in any one of SEQ ID NOs: 1-14 or a functional homologue thereof, wherein the functional homologue has at least 30% sequence identity to an amino acid sequence as shown in any one of SEQ ID NOs: 1-14.

44)An isolated polynucleotide having the nucleotide sequence as shown in any one of SEQ ID NOs: 15-28.

45) Use of the isolated polynucleotide according to item 43 or 44 for integration in a host cell.

46) Use of a polynucleotide according to item 43 or 44 for manufacturing a protein of interest.

47) Use of a polynucleotide according to item 43 or 44 for manufacturing a host cell.

48)An isolated polypeptide comprising an amino acid sequence having at least 30% identity to an amino acid sequence shown in any one of SEQ ID NOs: 1-14.

49) Use of the isolated polypeptide according to item 48 for manufacturing a protein of interest.

50)A composition comprising at least 10%, 20%, 30%, 40%, or 50% of a protein of interest and a polynucleotide according to item 43 or 44, wherein said polynucleotide is operably linked with a heterologous promoter.

## Detailed Description of the Invention

The present invention is partly based on the surprising finding of the expression of a protein involved in one or more proteinogenic amino acid biosynthesis pathways such as the helper proteins TA1 to TA14, which were found to increase secretion of model protein and/or of a protein of interest. The following Table A provides, inter alia, the name of the preferred helper proteins of the present invention.

| Helper protein(TA) | Microarray probe name | Gene identifier P. pastoris CBS7435 | Designation (in assumed analogy to S. cerevisiae) | Amino acid sequence (SEQ ID No.) | Nucleotide sequence (SEQ ID No.) |
|---|---|---|---|---|---|
| TA1 | pas_chr1-4_0339 | PP7435 Chr1-1124 | GCN4 | 1 | 15 |
| TA2 | pas_chr3_0482 | PP7435_Chr3-0727 | ALT1 | 2 | 16 |
| TA3 | pas_chr3_0566 | PP7435_Chr3-0640 | SER1 | 3 | 17 |
| TA4 | pas_chr2-1_0657 | PP7435_Chr2-0624 | SER3 | 4 | 18 |
| TA5 | pas_chr3_0936 | PP7435_Chr3-0235 | ARO3 | 5 | 19 |
| TA6 | pas_chr4_0050 | PP7435_Chr4-0965 | ARO7 | 6 | 20 |
| TA7 | pas_chr1-1_0432 | PP7435_Chr1-0750 | ILV5 | 7 | 21 |
| TA8 | pas_chr2-1_0415 | PP7435_Chr2-0884 | LEU4 | 8 | 22 |
| TA9 | pas_chr3_0181 | PP7435_Chr3-1050 | MAE1 | 9 | 23 |
| TA10 | pas_chr3_0294 | PP7435_Chr3-0925 | PRO1 | 10 | 24 |
| TA11 | pas_chr4_0398 | PP7435_Chr4-0587 | PRO3 | 11 | 25 |
| TA12 | pas_chr4_0330 | PP7435_Chr4-0665 | MET17 | 12 | 26 |
| TA13 | pas_chr2-2_0137 | PP7435_Chr2-0144 | CYS4 | 13 | 27 |
| TA14 | pas_chr3_0471 | PP7435_Chr3-0736 | HOM2 | 14 | 28 |

A "helper protein" as used in the present invention means a protein which is involved in one or more proteinogenic amino acid biosynthesis pathways and which enhances the yield of a model protein as described herein or of a protein of interest (POI), preferably when being overexpressed in a host cell which also expresses the protein of interest. This term should be understood broadly and should not be limited to, e.g. chaperones or chaperone-like proteins. As will appear evident from the present disclosure, helper proteins of the present invention are varied in their functions. A preferred helper protein of the present invention comprises the amino acid sequences of any one of SEQ ID NOs: 1 to 14 or a functional homologue thereof. A preferred helper protein of the present invention can be encoded by the nucleotide sequences of any one of SEQ ID NOs: 15 to 28 or variants thereof encoding a functional homologue of SEQ ID NO: 1, 2, 3, 4, 5, 6, 7, 8, 9, 10, 11, 12, 13, or 14, respectively. For the purpose of the present invention, the term "helper protein" is also meant to encompass functional homologues of TA1, TA2, TA3, TA4, TA5, TA6, TA7, TA8, TA9, TA10, TA11, TA12, TA13, or TA14, respectively. For example, a helper protein TA1 comprises the amino acid sequences encoding SEQ ID NO: 1 or functional homologues of encoding SEQ ID NO: 1. The invention provides an isolated polynucleotide sequence encoding a helper protein having an amino acid sequence as shown in SEQ ID NO: 1, 2, 3, 4, 5, 6, 7, 8, 9, 10, 11, 12, 13, or 14 or a functional homologue thereof, wherein the functional homologue has at least 30% sequence identity to an amino acid sequence as shown in SEQ ID NO: 1, 2, 3, 4, 5, 6, 7, 8, 9, 10, 11, 12, 13, or 14.

The term "involved in one or more proteinogenic amino acid biosynthesis pathways" means that a helper protein has regulatory or enzymatic function/activity in one or more pathways that result in the synthesis of amino acids within a cell. The term "amino acid biosynthesis pathway" includes those pathways of a cell, such as a host cell, that result in the synthesis of one or more of the known proteinogenic amino acids, with the term "proteonogenic" meaning that such one or more amino acids is incorporated into a polypeptide that is synthesized in a cell via translation of an mRNA. The term "involved in one or more proteinogenic amino acid biosynthesis pathways" also includes pathways that feed in precursors or building blocks used to synthesize one or more amino acids.

Preferably the "proteinogenic amino acid biosynthesis pathway" results in the formation of alanine, proline, sulphur-containing amino acids such as methionen or cysteine, phenylalanine, tyrosine, leucine, valine, isoleucine, serine. Accordingly, in a preferred embodiment the one or more proteinogenic amino acid biosynthesis pathway is one or more selected from an aliphatic amino acid biosynthesis pathway leading to amino acids such as glycine, alanine, valine, leucine, isoleucine; an aromatic amino acid biosynthesis pathway leading to amino acids such as phenylalanine, tyrosine, tryptophan; a cyclic amino acid biosynthesis pathway leading to amino acids such as such as proline; a hydroxyl amino acid biosynthesis pathway leading to amino acids such as serine, threonine or tyrosine; a branched chain amino acid biosynthesis pathway leading to amino acids such as valin, leucin or isoleucine; or a sulphur-containing amino acid biosynthesis pathway leading to amino acids such as cysteine or methionine.

While a preferred helper protein or functional homologue thereof of the present invention increases the yield of a model protein or protein of interest as described herein, preferably when a helper protein is overexpressed in a host cell, a preferred helper protein has additionally or alternatively preferably the function or activity as described in the below

Table. Thus, a helper protein or functional homologue thereof of the present invention can, additionally or alternatively to the function of increasing the yield of a model protein or protein of interest in a host cell, preferably when being overexpressed, also be tested for the function or activity as described in the below Table B. For example, a TA1 helper protein or functional homologue thereof functions as a transcription factor or has the activity of being a transcriptional activator/regulator of amino acid biosynthetic genes, while TA2 helper protein or functional homologue thereof functions as an enzyme.

| Helper protein(TA) | Protein function | Activity |
|---|---|---|
| TA1 | Transcription factor | bZIP transcriptional activator of amino acid biosynthetic genes |
| TA2 | Alanine transaminase | involved in alanine biosynthesis and catabolism |
| TA3 | Phosphoserine aminotransferase | catalyzes the formation of phosphoserine from 3-phosphohydroxypyruvate, required for serine and glycine biosynthesis |
| TA4 | D-3-phosphoglycerate dehydrogenase | catalyzes the first step in serine and glycine biosynthesis |
| TA5 | 3-deoxy-D-arabinoheptulosonate-7-phosphate (DAHP) synthase | catalyzes the first step in aromatic amino acid biosynthesis |
| TA6 | Chorismate mutase | catalyzes the conversion of chorismate to prephenate to initiate the tyrosine/phenylalanine-specific branch of aromatic amino acid biosynthesis |
| TA7 | Ketol-acid reductoisomerase | involved in branched-chain amino acid biosynthesis |
| TA8 | 2-isopropylmalate synthase | responsible for the first step in the leucine biosynthesis pathway |
| TA9 | Malic Enzyme | catalyzes the oxidative decarboxylation of malate to pyruvate |
| TA10 | Glutamate 5-kinase | catalyzes the first step in proline biosynthesis |
| TA11 | Pyrroline-5-carboxylate reductase | catalyzes the last step in proline biosynthesis |
| TA12 | O-acetylhomoserine aminocarboxypropylt ransferase | required for Methionine and cysteine biosynthesis |
| TA13 | Cystathionine beta-synthase | catalyzes synthesis of cystathionine from serine and homocysteine |
| TA14 | Aspartate-semialdehyde dehydrogenase | catalyzes the second step in the common pathway for methionine and threonine biosynthesis |

As used herein, a "homologue" or "functional homologue" of a polypeptide shall mean that a polypeptide has the same or conserved residues at a corresponding position in their primary, secondary or tertiary structure. The term also extends to two or more nucleotide sequences encoding homologous polypeptides. In particular, polypeptides homologous to the present helper proteins have at least about 30% amino acid sequence identity with regard to a full-length native sequence or any fragment thereof. Preferably, a homologous polypeptide will have at least about 35% amino acid sequence identity, more preferably at least about 40% amino acid sequence identity, more preferably at least about 45% amino acid sequence identity, more preferably at least about 50% amino acid sequence identity, more preferably at least about 55% amino acid sequence identity, more preferably at least about 60% amino acid sequence identity, more preferably at least about 65% amino acid sequence identity, more preferably at least about 70% amino acid sequence identity, more preferably at least about 75% amino acid sequence identity, more preferably at least about 80% amino acid sequence identity, more preferably at least about 85% amino acid sequence identity, more preferably at least about 90%, such as 91, 92, 93, 94, 95, 96, 97, 98 or 99% amino acid sequence identity, more preferably at least about 95% amino acid sequence identity to a native compound, or any other specifically defined fragment of a full-length compound. When the function as a helper protein is proven with such a homologue, the homologue is called "functional homologue". A functional homologue performs the same or substantially the same function as the helper protein from which it is derived from, i.e. it increases the yield of the model protein CES as described herein andor has the function or activity as shown in Table 2b. Alternative model proteins are HyHEL-Fab and/or SDZ-Fab. The function of a helper protein as given in Table B or Table 2b, above, can be tested by assay known in the art or preferably with the assay using the model proteins as described in Example 5, particularly as described herein above in the context of "Fab titer", "Fab yield", "FC titer", or "FC yield".

Generally, homologues can be prepared using any mutagenesis procedure known in the art, such as site-directed mutagenesis, synthetic gene construction, semi-synthetic gene construction, random mutagenesis, shuffling, etc. Site-directed mutagenesis is a technique in which one or more (e.g., several) mutations are introduced at one or more defined sites in a polynucleotide encoding the parent. Site-directed mutagenesis can be accomplished in vitro by PCR involving the use of oligonucleotide primers containing the desired mutation. Site-directed mutagenesis can also be performed in vitro by cassette mutagenesis involving the cleavage by a restriction enzyme at a site in the plasmid comprising a polynucleotide encoding the parent and subsequent ligation of an oligonucleotide containing the mutation in the polynucleotide. Usually the restriction enzyme that digests the plasmid and the oligonucleotide is the same, permitting sticky ends of the plasmid and the insert to ligate to one another. See, e.g., Scherer and Davis, 1979, Proc. Natl. Acad. Sci. USA 76: 4949-4955; and Barton et ai, 1990, Nucleic Acids Res. 18: 7349-4966. Site-directed mutagenesis can also be accomplished in vivo by methods known in the art. See, e.g., U.S. Patent Application Publication No. 2004/0171 154; Storici et ai, 2001 , Nature Biotechnol. 19: 773-776; Kren et ai, 1998, Nat. Med. 4: 285-290; and Calissano and Macino, 1996, Fungal Genet. Newslett. 43: 15-16. Synthetic gene construction entails in vitro synthesis of a designed polynucleotide molecule to encode a polypeptide of interest. Gene synthesis can be performed utilizing a number of techniques, such as the multiplex microchip-based technology described by Tian et al. (2004, Nature 432: 1050-1054) and similar technologies wherein oligonucleotides are synthesized and assembled upon photo-programmable microfluidic chips. Single or multiple amino acid substitutions, deletions, and/or insertions can be made and tested using known methods of mutagenesis, recombination, and/or shuffling, followed by a relevant screening procedure, such as those disclosed by Reidhaar-Olson and Sauer, 1988, Science 241:53-57; Bowie and Sauer, 1989, Proc. Natl. Acad. Sci. USA 86: 2152-2156; WO 95/17413; or WO 95/22625. Other methods that can be used include error-prone PCR, phage display (e.g., Lowman et al, 1991, Biochemistry 30: 10832-10837; U.S. Patent No. 5,223,409; WO 92/06204) and region-directed mutagenesis (Derbyshire et al., 1986, Gene 46: 145; Ner et al., 1988, DNA 7:127). Mutagenesis/shuffling methods can be combined with high-throughput, automated screening methods to detect activity of cloned, mutagenized polypeptides expressed by host cells (Ness et a/., 1999, Nature Biotechnology 17: 893-896). Mutagenized DNA molecules that encode active polypeptides can be recovered from the host cells and rapidly sequenced using standard methods known in the art. These methods allow the rapid determination of the importance of individual amino acid residues in a polypeptide. Semi-synthetic gene construction is accomplished by combining aspects of synthetic gene construction, and/or site-directed mutagenesis, and/or random mutagenesis, and/or shuffling. Semisynthetic construction is typified by a process utilizing polynucleotide fragments that are synthesized, in combination with PCR techniques. Defined regions of genes may thus be synthesized de novo, while other regions may be amplified using site-specific mutagenic primers, while yet other regions may be subjected to error-prone PCR or non-error prone PCR amplification. Polynucleotide subsequences may then be shuffled. Alternatively, homologues can be obtained from a natural source such as by screening cDNA libraries of closely or distantly related microorganisms.

The function of a homologue of SEQ I D NO: 1, 2, 3, 4, 5, 6, 7, 8, 9, 10, 11, 12, 13, or 14 can be tested by providing expression cassettes into which the homologue sequences have been inserted, transforming host cells that carry the sequence encoding a test protein such as one of the model proteins used in the Example section or a specific POI, and determining the difference in the yield of the model protein or POI under identical conditions. However, as described herein the function of a homologue can additionally or alternatively also be tested in accordance with the function or activity as shown in Table 2b for TA1 to TA14.

A functional homologue may also be a biologically active fragment of the helper proteins. Generally, biologically active fragment of a protein shall mean a fragment that exerts a biological effect similar or comparable to that of the full length protein. Such fragments or variants can be produced e.g. by amino- and/or carboxy- terminal deletions as well as by internal deletions. A fragment may have a length of 50, 100, 150, 200, 250 or more amino acids in length.

The present invention provides an isolated polynucleotide sequence encoding SEQ ID NO: 1, 2, 3, 4, 5, 6, 7, 8, 9, 10, 11, 12, 13, or 14 or functional homologues of SEQ ID NO: 1, 2, 3, 4, 5, 6, 7, 8, 9, 10, 11, 12, 13, or 14. The isolated polynucleotide sequence may comprise any one of SEQ ID NO: 14, 15, 16, 17, 18, 19, 20, 21, 22, 23, 24, 25, 26, 27, or 28. Preferably, the isolated polynucleotide sequence consists of the nucleotide sequence of any one of SEQ ID NO: 14, 15, 16, 17, 18, 19, 20, 21, 22, 23, 24, 25, 26, 27, or 28:

In a further aspect, the present invention provides an isolated polypeptide comprising a polypeptide sequence having at least 30%, such as at least 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or 100% sequence identity to an amino acid sequence selected from the group consisting of SEQ ID NO: 1, 2, 3, 4, 5, 6, 7, 8, 9, 10, 11, 12, 13, or 14.

The term "isolated" means a substance in a form or environment that does not occur in nature. Non-limiting examples of isolated substances include (1) any non-naturally occurring substance, (2) any substance including, but not limited to, any enzyme, variant, nucleic acid, protein, peptide or cofactor, that is at least partially removed from one or more or all of the naturally occurring constituents with which it is associated in nature; (3) any substance modified by the hand of man relative to that substance found in nature; or (4) any substance modified by increasing the amount of the substance relative to other components with which it is naturally associated (e.g., recombinant production in a host cell; multiple copies of a gene encoding the substance; and use of a stronger promoter than the promoter naturally associated with the gene encoding the substance).

The present invention provides use of any one of the above mentioned isolated polynucleotides for integration in a host cell. Alternatively, if the polynucleotide(s) already exist in the host cell, the host cell can be manipulated in a way such that they are overexpressed, as will be described later. In another aspect, the invention relates to the use of said polynucleotide for increasing a POI yield from a host cell, wherein the nucleotide sequence encoding the POI is co-expressed with said polynucleotides.

"Sequence identity" or "% identity" refers to the percentage of residue matches between at least two polypeptide or polynucleotide sequences aligned using a standardized algorithm. Such an algorithm may insert, in a standardized and reproducible way, gaps in the sequences being compared in order to optimize alignment between two sequences, and therefore achieve a more meaningful comparison of the two sequences. For purposes of the present invention, the sequence identity between two amino acid sequences or nucleotide is determined using the NCBI BLAST program version 2.2.29 (Jan-06-2014) (Altschul et al., Nucleic Acids Res. (1997) 25:3389-3402). Sequence identity of two amino acid sequences can be determined with blastp set at the following parameters: Matrix: BLOSUM62, Word Size: 3; Expect value: 10; Gap cost: Existence = 11, Extension = 1; Filter = low complexity activated; Filter String: L; Compositional adjustments: Conditional compositional score matrix adjustment. For purposes of the present invention, the sequence identity between two nucleotide sequences is determined using the NCBI BLAST program version 2.2.29 (Jan-06-2014) with blastn set at the following exemplary parameters: Word Size: 11; Expect value: 10; Gap costs: Existence = 5 , Extension = 2; Filter = low complexity activated; Match/Mismatch Scores: 2,-3; Filter String: L; m.

In a second aspect, the present invention provides a host cell engineered to overexpress a polynucleotide encoding a helper protein of the present invention. The helper proteins include any one of TA1 to TA14 (SEQ ID NO: 1-14), or functional homologues thereof.

Preferably, the invention provides a recombinant host cell for manufacturing a protein of interest, wherein the host cell is engineered to overexpress a polynucleotide encoding a helper protein having an amino acid having at least 30% sequence identity to an amino acid sequence as shown in SEQ ID NO: 1, 2, 3, 4, 5, 6, 7, 8, 9, 10, 11, 12, 13, or 14.

The term "expressing a polynucleotide" means when a polynucleotide is transcribed to mRNA and the mRNA is translated to a polypeptide. The term "overexpress" generally refers to any amount greater than or equal to an expression level exhibited by a reference standard. The terms "overexpress," "overexpressing," "overexpressed" and "overexpression" in the present invention refer an expression of a gene product or a polypeptide at a level greater than the expression of the same gene product or polypeptide prior to a genetic alteration of the host cell or in a comparable host which has not been genetically altered at defined conditions. If a host cell does not comprise a given gene product, it is possible to introduce the gene product into the host cell for expression; in this case, any detectable expression is encompassed by the term "overexpression."

As used herein, "engineered" host cells are host cells which have been manipulated using genetic engineering, i.e. by human intervention. When a host cell is "engineered to overexpress" a given protein, the host cell is manipulated such that the host cell has the capability to express, preferably overexpress a helper protein or functional homologue thereof, thereby expression of a given protein, e.g. POI or model protein is increased compared to the host cell under the same condition prior to manipulation.

"Prior to engineering" when used in the context of host cells of the present invention means that such host cells are not engineered with a polynucleotide encoding a helper protein or functional homologue thereof. Said term thus also means that host cells do not overexpress a polynucleotide encoding a helper protein or functional homologue thereof or are not engineered to overexpress a polynucleotide encoding a helper protein or functional homologue thereof.

The term "recombining" as used herein means that a host cell of the present invention is equipped with a heterologous polynucleotide encoding a protein of interest, i.e., a host cell of the present invention is engineered to contain a heterologous polynucleotide encoding a protein of interest. This can be achieved, e.g., by transformation or transfection or any other suitable technique known in the art for the introduction of a polynucleotide into a host cell. In the host cell, said polynucleotide can, as described herein, be integrated int o the genome of the host cell or it can replicate in the form of a plasmid, etc. as described herein. For avoidance of doubt, the heterologous polynucleotide can be in the form of an expression cassette or expression vector that contains a promoter, enhancer, or the like and/or a terminator sequence, etc. In any case, it is understood that said heterologous polynucleotide is expressed in said host cell after it was recombined into said host cell.

Overexpression

Overexpression can be achieved in any ways known to a skilled person in the art as will be described later in detail. In general, it can be achieved by increasing transcription/translation of the gene, e.g. by increasing the copy number of the gene or altering or modifying regulatory sequences or sites associated with expression of a gene. For example, overexpression can be achieved by introducing one or more copies of the polynucleotide encoding a helper protein or a functional homologue operably linked to regulatory sequences (e.g. a promoter). For example, the gene can be operably linked to a strong constitutive promoter and/or strong ubiquitous promoter in order to reach high expression levels. Such promoters can be endogenous promoters or recombinant promoters. Alternatively, it is possible to remove regulatory sequences such that expression becomes constitutive. One can substitute the native promoter of a given gene with a heterologous promoter which increases expression of the gene or leads to constitutive expression of the gene. For example, the helper protein maybe overexpressed by more than 10%, 20%, 30%, 40%, 50%, 60%, 70%, 80%, 90%, 100%, 200%, or more than 300% by the host cell compared to the host cell prior to engineering and cultured under the same conditions. Using inducible promoters additionally makes it possible to increase the expression in the course of host cell cultivation. Furthermore, overexpression can also be achieved by, for example, modifying the chromosomal location of a particular gene, altering nucleic acid sequences adjacent to a particular gene such as a ribosome binding site or transcription terminator, modifying proteins (e.g., regulatory proteins, suppressors, enhancers, transcriptional activators and the like) involved in transcription of the gene and/or translation of the gene product, or any other conventional means of deregulating expression of a particular gene routine in the art (including but not limited to use of antisense nucleic acid molecules, for example, to block expression of repressor proteins or deleting or mutating the gene for a transcriptional factor which normally represses expression of the gene desired to be overexpressed. Prolonging the life of the mRNA may also improve the level of expression. For example, certain terminator regions may be used to extend the half-lives of mRNA (Yamanishi et al., Biosci. Biotechnol. Biochem. (2011) 75:2234 and US 2013/0244243). If multiple copies of genes are included, the genes can either be located in plasmids of variable copy number or integrated and amplified in the chromosome. If the host cell does not comprise the gene product encoding the helper protein, it is possible to introduce the gene product into the host cell for expression. In this case, "overexpression" means expressing the gene product using any methods known to a skilled person in the art.

Those skilled in the art will find relevant instructions in Martin et al. (Bio/Technology 5, 137-146 (1987)), Guerrero et al. (Gene 138, 35-41 (1994)), Tsuchiya and Morinaga (Bio/Technology 6, 428-430 (1988)), Eikmanns et al. (Gene 102, 93-98 (1991)), EP 0 472 869, US 4,601,893, Schwarzer and Pühler (Bio/Technology 9, 84-87 (1991)), Reinscheid et al. (Applied and Environmental Microbiology 60, 126-132 (1994)), LaBarre et al. (Journal of Bacteriology 175, 1001-1007 (1993)), WO 96/15246, Malumbres et al. (Gene 134, 15- 24 (1993)), JP-A-10-229891, Jensen and Hammer (Biotechnology and Bioengineering 58, 191-195 (1998)) and Makrides (Microbiological Reviews 60, 512-538 (1996)), inter alia, and in well-known textbooks on genetics and molecular biology.

Helper Proteins

The helper proteins of the present invention were originally isolated from *Pichia pastoris* CBS7435 strain. The methylotrophic yeast *Pichia pastoris* (*Komagataella phaffii*) CBS7435 is the parental strain of commonly used *P. pastoris* recombinant protein production hosts. Its complete genomic sequence is described in Küberl et al. (J Biotechnol. (2011) 154(4):312-20). These genes encoding the helper proteins identified herein have so far not been associated with a beneficial effect on protein yield.

It is envisioned that the helper proteins can be overexpressed over a wide range of host cells because of their presence in other microorganisms. Thus, instead of using the sequences native to the species or the genus, the helper protein sequences may be taken or derived from other prokaryotic or eukaryotic organisms. The foreign DNA sequences encoding the helper proteins may be obtained from a variety of sources, such as from a plant, insect, fungal or mammalian species, preferably from the class of *Saccharomycetes,* preferably from the order of *Saccharomycetales,* preferably from the family of *Saccharomycetaceae,* and preferably from the genus of *Komagataella.*

TA1 to TA14

In particular, the invention refers to a genetically modified host cell which is capable of overexpressing the helper proteins TA1, TA2, TA3, TA4, TA5, TA6, TA7, TA8, TA9, TA10, TA11, TA12, TA13, or TA14 or combinations thereof or functional homologues of TA1, TA2, TA3, TA4, TA5, TA6, TA7, TA8, TA9, TA10, TA11, TA12, TA13, or TA14 or combinations thereof. Host cells being engineered to reflect such a combination are envisaged in a preferred embodiment. These host cells are preferably applied in the methods and uses described herein. A combination includes that 2 or more, such as 2, 3, 4, 5, 6, 7, 8 or more, helper proteins or a functional homologue are chosen from TA1, TA2, TA3, TA4, TA5, TA6, TA7, TA8, TA9, TA10, TA11, TA12, TA13, or TA14. "Reflected" means that the skilled person knows in accordance with the teaching of the present invention that a helper protein is overexpressed, while a KO protein is underexpressed.

However, specific combinations of helper proteins or a functional homologue represent a preferred embodiment of the host cells of the present invention that are preferably applied in the methods and uses of the present invention. These specific combinations are

(a) helper proteins TA3 and TA4 having the amino acid sequence as shown in SEQ ID NOs: 3 and 4 or a functional homologue thereof, wherein the functional homologue has at least 30% sequence identity to an amino acid sequence as shown in SEQ ID NOs: 3 and 4, respectively;

(b) helper proteins TA5 and TA6 having the amino acid sequence as shown in SEQ ID NOs: 5 and 6 or a functional homologue thereof, wherein the functional homologue has at least 30% sequence identity to an amino acid sequence as shown in SEQ ID NOs: 5 and 6, respectively;

(c) helper proteins TA7 and TA8 having the amino acid sequence as shown in SEQ ID NOs: 7 and 8 or a functional homologue thereof, wherein the functional homologue has at least 30% sequence identity to an amino acid sequence as shown in SEQ ID NOs: 7 and 8, respectively;

(d) helper proteins TA10 and TA11 having the amino acid sequence as shown in SEQ ID NOs: 10 and 11 or a functional homologue thereof, wherein the functional homologue has at least 30% sequence identity to an amino acid sequence as shown in SEQ ID NOs: 10 and 11, respectively; or

(e) helper proteins TA12, TA13 and TA14 having the amino acid sequence as shown in SEQ ID NOs: 12, 13 and 14 or a functional homologue thereof, wherein the functional homologue has at least 30% sequence identity to an amino acid sequence as shown in SEQ ID NOs: 12, 13 and 14, respectively.

More preferably, the present invention provides a recombinant host cell for manufacturing a protein of interest, wherein the host cell is engineered to overexpress a polynucleotide encoding a helper protein having an amino acid having at least 60% sequence identity to SEQ ID NO: 1, 50% sequence identity to SEQ ID NO: 2, 50% sequence identity to SEQ ID NO: 3, 50% sequence identity to SEQ ID NO: 4, 50% sequence identity to SEQ ID NO: 5, 50% sequence identity to SEQ ID NO: 6, 50% sequence identity to SEQ ID NO: 7, 50% sequence identity to SEQ ID NO: 8, 50% sequence identity to SEQ ID NO: 9, 50% sequence identity to SEQ ID NO: 10, 50% sequence identity to SEQ ID NO: 11, 50% sequence identity to SEQ ID NO: 12, 50% sequence identity to SEQ ID NO: 13, or 50% sequence identity to SEQ ID NO: 14. Such a host cell is preferably applied in the methods and uses described herein.

## Protein of interest

The term "protein of interest" (POI) as used herein refers to a protein that is produced by means of recombinant technology in a host cell. More specifically, the protein may either be a polypeptide not naturally occurring in the host cell, i.e. a heterologous protein, or else may be native to the host cell, i.e. a homologous protein to the host cell, but is produced, for example, by transformation with a self-replicating vector containing the nucleic acid sequence encoding the POI, or upon integration by recombinant techniques of one or more copies of the nucleic acid sequence encoding the POI into the genome of the host cell, or by recombinant modification of one or more regulatory sequences controlling the expression of the gene encoding the POI, e.g. of the promoter sequence. In general, the proteins of interest referred to herein may be produced by methods of recombinant expression well known to a person skilled in the art.

## Host cell

As used herein, a "host cell" refers to a cell which is capable of protein expression and optionally protein secretion. Such host cell is applied in the methods of the present invention. For that purpose, for the host cell to express a polypeptide, a nucleotide sequence encoding the polypeptide is present or introduced in the cell. Host cells provided by the present invention can be prokaryotes or eukaryotes. As will be appreciated by one of skill in the art, a prokaryotic cell lacks a membrane-bound nucleus, while a eukaryotic cell has a membrane-bound nucleus. Examples of eukaryotic cells include, but are not limited to, vertebrate cells, mammalian cells, human cells, animal cells, invertebrate cells, plant cells, nematodal cells, insect cells, stem cells, fungal cells or yeast cells.

Examples of yeast cells include but are not limited to the *Saccharomyces* genus (e.g. *Saccharomyces cerevisiae, Saccharomyces kluyveri, Saccharomyces uvarum*), the *Komagataella* genus (*Komagataella pastoris, Komagataella pseudopastoris* or *Komagataella phaffii*), *Kluyveromyces* genus (e.g. *Kluyveromyces lactis, Kluyveromyces marxianus*), the *Candida* genus (e.g. *Candida utilis, Candida cacaoi*), the *Geotrichum* genus (e.g. *Geotrichum fermentans*), as well as *Hansenula polymorpha* and *Yarrowia lipolytica,*

The genus *Pichia* is of particular interest. *Pichia* comprises a number of species, including the species *Pichia pastoris, Pichia methanolica, Pichia kluyveri,* and *Pichia angusta.* Most preferred is the species *Pichia pastoris.*

The former species *Pichia pastoris* has been divided and renamed to *Komagataella pastoris* and *Komagataella phaffii.* Therefore *Pichia pastoris* is synonymous for both *Komagataella pastoris* and *Komagataella phaffii.*

Examples for *Pichia pastoris* strains useful in the present invention are X33 and its subtypes GS115, KM71, KM71H; CBS7435 (mut+) and its subtypes CBS7435 mut$^s$, CBS7435 mut$^s\Delta$Arg, CBS7435 mut$^s\Delta$His, CBS7435 mut$^s$-$\Delta$Arg, $\Delta$His, CBS7435 mut$^s$ PDI$^+$, CBS 704 (=NRRL Y-1603 = DSMZ 70382), CBS 2612 (=NRRL Y-7556), CBS 9173-9189 and DSMZ 70877 as well as mutants thereof.

Examples of *E. coli* include those derived from *Escherichia coli* K12 strain, specifically, HMS 174, HMS174 (DE3), NM533, XL1-Blue, C600, DH1, HB101, JM109, as well as those derived from B-strains, specifically BL-21, BL21 (DE3) and the like.

According a further preferred embodiment, the host cell is a *Pichia pastoris, Hansenula polymorpha, Trichoderma reesei, Saccharomyces cerevisiae, Kluyveromyces lactis, Yarrowia lipolytica, Pichia methanolica, Candida boidinii, Komagataella sp. Schizosaccharomyces pombe* or *Apergillus sp., such* as *A. niger.* It may also be a host cell from *Ustilago maydis.*

Preferably, the helper proteins expressed by the host cell is from the same cell or recombined from a cell of the same species, genus or family. As used herein, "recombinant" refers to the alteration of genetic material by human intervention. Typically, recombinant refers to the manipulation of DNA or RNA in a virus, cell, plasmid or vector by molecular biology (recombinant DNA technology) methods, including cloning and recombination. A recombinant cell, polypeptide, or nucleic acid can be typically described with reference to how it differs from a naturally occurring counterpart (the "wild-type"). A "recombinant cell" or "recombinant host cell" refers to a cell or host cell that has been genetically altered to comprise a nucleic acid sequence which was not native to said cell.

The term "manufacture" or "manufacturing" as used presently refers to the process in which the protein of interest is expressed. A "host cell for manufacturing a protein of interest" refers to a host cell in which nucleic acid sequences encoding a protein of interest may be introduced. The recombinant host cell within the present invention does not necessarily contain the nucleic acid sequences encoding a protein of interest. It is appreciated by a skilled person in the art that the host cells can be provided for inserting desired nucleotide sequences into the host cell, for example, in a kit.

The term "nucleotide sequence" or "nucleic acid sequence" used herein refers to either DNA or RNA. "Polynucleotide sequence" or simply "polynucleotide" refers to a single or double-stranded polymer of deoxyribonucleotide or ribonucleotide bases read from the 5' to the 3' end. It includes both self-replicating plasmids, infectious polymers of DNA or RNA, and non-functional DNA or RNA. The aforementioned terms can be used interchangeably herein and can thus substitute each other.

The term "amino acid" refers to naturally occurring and synthetic amino acids, as well as amino acid analogs and amino acid mimetics that function in a manner similar to the naturally occurring amino acids. Naturally occurring amino acids are those encoded by the genetic code, as well as those amino acids that are later modified, e.g., hydroxyproline, $\gamma$-carboxyglutamate, and O-phosphoserine. Amino acid analogs refers to compounds that have the same basic chemical structure as a naturally occurring amino acid, i.e., a carbon that is bound to a hydrogen, a carboxyl group, an amino group, and an R group, e.g., homoserine, norleucine, methionine sulfoxide, methionine methyl sulfonium. Such analogs have modified R groups (e.g., norleucine) or modified peptide backbones, but retain the same basic chemical structure as a naturally occurring amino acid. Amino acid mimetics refers to chemical compounds that have a structure that is different from the general chemical structure of an amino acid, but that function in a manner similar to a naturally occurring amino acid.

The terms "polypeptide" and "protein" are interchangeably used. The term "polypeptide" refers to a protein or peptide that contains two or more amino acids, typically at least 3, preferably at least 20, more preferred at least 30, such as at least 50 amino acids. Accordingly, a polypeptide comprises an amino acid sequence, and, thus, sometimes a polypeptide comprising an amino acid sequence is referred to herein as a "polypeptide comprising a polypeptide sequence". Thus, herein the term "polypeptide sequence" is interchangeably used with the term "amino acid sequence". As mentioned, overexpression can be achieved by insertion of one or more than one extra copy of the selected helper protein. According to a preferred embodiment, the polynucleotide encoding the helper protein can be presented in a single copy or in multiple copies per cell. The copies may be adjacent to or distant from each other. According to another preferred embodiment, the method of the invention employs recombinant nucleotide sequences encoding the helper proteins provided on one or more plasmids suitable for integration into the genome of the host cell, in a single copy or in multiple copies per cell. The copies may be adjacent to or distant from each other. Overexpression can be in one embodiment achieved by expressing one or multiple copies of the polynucleotide, such as 2, 3, 4, 5, 6 or more copies of said polynucleotide per host cell. The polynucleotides are preferably operably linked to transcriptional and translational regulatory sequences that provide for expression of the polypeptide in the host cells. The term "transcriptional regulatory sequences" as used herein refers to nucleotide sequences that are associated with a gene nucleic acid sequence and which regulate the transcription of the gene. The term "translational regulatory sequences" as used herein refers to nucleotide sequences that are associated with a gene nucleic acid sequence and which regulate the translation of the gene. Transcriptional and/or translational regulatory sequences can either be located in plasmids or vectors or integrated in the chromosome of the host cell. Transcriptional and/or translational regulatory sequences are located in the same nucleic acid molecule of the gene which it regulates.

Preferably, the overexpression can be achieved by having 1, 2, 3, 4 or more copies of a polynucleotide encoding TA1, TA2, TA3, TA4, TA5, TA6, TA7, TA8, TA9, TA10, TA11, TA12, TA13, or TA14, or functional homologues thereof per host cell.

The polynucleotide encoding the helper protein and/or the polynucleotide encoding the POI is/are preferably integrated into the genome of the host cell. The term "genome" generally refers to the whole hereditary information of an organism that is encoded in the DNA (or RNA for certain viral species). It may be present in the chromosome, on a plasmid or vector, or both. Preferably, the polynucleotide encoding the helper protein is integrated into the chromosome of said cell.

The polynucleotide encoding the helper protein or functional homologue thereof may be integrated in its natural locus. "Natural locus" means the location on a specific chromosome, where the polynucleotide encoding the helper protein is located, for example at the natural locus of TA1 to TA14 as shown in Table 2a. However, in another embodiment, the polynucleotide encoding the helper protein is present in the genome of the host cell not at their natural locus, but integrated ectopically. The term "ectopic integration" means the insertion of a nucleic acid into the genome of a microorganism at a site other than its usual chromosomal locus, i.e., predetermined or random integration. In the alternative, the polynucleotide encoding the helper protein or functional homologue thereof may be integrated in its natural locus and ectopically.

For yeast cells, the polynucleotide encoding the helper protein and/or the polynucleotide encoding the POI may be inserted into a desired locus, such as AOX1, GAP, ENO1, TEF, HIS4 (Zamir et al., Proc. NatL Acad. Sci. USA (1981) 78(6):3496-3500), HO (Voth et al. Nucleic Acids Res. 2001 June 15; 29(12): e59), TYR1 (Mirisola et al., Yeast 2007; 24: 761-766), His3, Leu2, Ura3 (Taxis et al., BioTechniques (2006) 40:73-78), Lys2, ADE2, TRP1, GAL1, ADH1 or on the integration of 5S ribosomal RNA gene.

In other embodiments, the polynucleotide encoding the helper protein and/or the polynucleotide encoding the POI can be integrated in a plasmid or vector. The terms "plasmid" and "vector" include autonomously replicating nucleotide sequences as well as genome integrating nucleotide sequences. A skilled person is able to employ suitable plasmids or vectors depending on the host cell used.

Preferably, the plasmid is a eukaryotic expression vector, preferably a yeast expression vector.

Plasmids can be used to for the transcription of cloned recombinant nucleotide sequences, i.e. of recombinant genes and the translation of their mRNA in a suitable host organism. Plasmids can also be used to integrate a target polynuclotide into the host cell genome by methods known in the art, such as described by J. Sambrook et al., Molecular Cloning: A Laboratory Manual (3rd edition), Cold Spring Harbor Laboratory, Cold Spring Harbor Laboratory Press, New York (2001). A "plasmid" usually comprise an origin for autonomous replication in the host cells, selectable markers, a number of restriction enzyme cleavage sites, a suitable promoter sequence and a transcription terminator, which components are operably linked together. The polypeptide coding sequence of interest is operably linked to transcriptional and translational regulatory sequences that provide for expression of the polypeptide in the host cells.

A nucleic acid is "operably linked" when it is placed into a functional relationship with another nucleic acid sequence on the same nucleic acid molecule. For example, a promoter is operably linked with a coding sequence of a recombinant gene when it is capable of effecting the expression of that coding sequence.

Most plasmids exist in only one copy per cell. Some plasmids, however, exist in higher copy numbers. For example, the plasmid ColE1 typically exists in 10 to 20 plasmid copies per chromosome in *E. coli.* If the nucleotide sequences of the present invention are contained in a plasmid, the plasmid may have a copy number of 20-30, 30-100 or more per host cell. With a high copy number of plasmids, it is possible to overexpress helper proteins by the cell.

Large numbers of suitable plasmids or vectors are known to those of skill in the art and many are commercially available. Examples of suitable vectors are provided in Sambrook et al, eds., Molecular Cloning: A Laboratory Manual (2nd Ed.), Vols. 1-3, Cold Spring Harbor Laboratory (1989), and Ausubel et al, eds., Current Protocols in Molecular Biology, John Wiley & Sons, Inc., New York (1997).

A vector or plasmid of the present invention encompass yeast artificial chromosome, which refers to a DNA construct that can be genetically modified to contain a heterologous DNA sequence (e.g., a DNA sequence as large as 3000 kb), that contains telomeric, centromeric, and origin of replication (replication origin) sequences.

A vector or plasmid of the present invention also encompasses bacterial artificial chromosome (BAC), which refers to a DNA construct that can be genetically modified to contain a heterologous DNA sequence (e.g., a DNA sequence as large as 300 kb), that contains an origin of replication sequence (Ori), and may contain one or more helicases (e.g., parA, parB, and parC).

Examples of plasmids using yeast as a host include YIp type vector, YEp type vector, YRp type vector, YCp type vector, pGPD-2, pAO815, pGAPZ, pGAPZα, pHIL-D2, pHIL-S1, pPIC3.5K, pPIC9K, pPICZ, pPICZα, pPIC3K, pHWO10, pPUZZLE and 2 μm plasmids. Such vectors are known and are for example described in Cregg et al., Mol Biotechnol. (2000) 16(1):23-52.

Examples of plasmids using Escherichia coli as their host include pBR322, pUC18, pUC19, pUC118, pVC119, pSP64, pSP65, pTZ-18R/-18U, pTZ-19R/-19U, pGEM-3, pGEM-4, pGEM-3Z, pGEM-4Z, pGEM-5Zf(-), and pBluescript KSTM (Stratagene). Examples of plasmids suitable for expression in Escherichia coli include pAS, pKK223 (Pharmacia), pMC1403, pMC931, and pKC30.

## Promoter

Overexpression of the endogenous polypeptide in the recombinant cell can be achieved by modifying transcriptional and translational regulatory sequences, including, for example, promoters, enhancers, polyadenylation signals, transcription terminators, internal ribosome entry sites (IRES), and the like, that provide for the expression of the polynucleotide sequence in a host cell. Such sequences interact specifically with cellular proteins involved in transcription (Maniatis et al., Science, 236: 1237-1245 (1987)). Exemplary sequences are described in, for example, Goeddel, Gene Expression Technology: Methods in Enzymology, Vol. 185, Academic Press, San Diego, Calif. (1990).

For example, overexpression of the endogenous helper protein in the recombinant cell can be achieved by modifying the promoters, for example, by replacing the endogenous promoter which is operably linked to the helper protein with another stronger promoter in order to reach high expression levels. Such promoter may be inductive or constitutive. Modification of endogenous promoter may be performed by mutation or homologous recombination using methods known in the art.

The overexpression of the polynucleotide encoding the helper proteins, can be achieved by other methods known in the art, for example by genetically modifying their endogenous regulatory regions, as described by Marx et al., 2008 (Marx, H., Mattanovich, D. and Sauer, M. Microb Cell Fact 7 (2008): 23), and Pan et al., 2011 (Pan et al., FEMS Yeast Res. (2011) May; (3):292-8.), such methods include, for example, integration of a recombinant promoter that increases expression of the helper proteins. Transformation is described in Cregg et al. (1985) Mol. Cell. Biol. 5:3376-3385. A "recombinant" promoter is referred to with respect to the sequence whose expression it drives. As used herein, a recombinant promoter means when the promoter is not a native promoter to the given sequence, i.e., when the promoter is different from a naturally occurring promoter (the "native promoter"). Such a promoter is sometimes also referred to herein as heterologous promoter.

The term "promoter" as used herein refers to a region that facilitates the transcription of a particular gene. A promoter typically increases the amount of recombinant product expressed from a nucleotide sequence as compared to the amount of the expressed recombinant product when no promoter exists. A promoter from one organism can be utilized to enhance recombinant product expression from a sequence that originates from another organism. The promoter can be integrated into a host cell chromosome by homologous recombination using methods known in the art (e.g. Datsenko et al, Proc. Natl. Acad. Sci. U.S.A., 97(12): 6640-6645 (2000)). In addition, one promoter element can increase the amount of products expressed for multiple sequences attached in tandem. Hence, one promoter element can enhance the expression of one or more recombinant products.

Promoter activity may be assessed by its transcriptional efficiency. This may be determined directly by measurement of the amount of mRNA transcription from the promoter, e.g. by Northern Blotting, quantitative PCR or indirectly by measurement of the amount of gene product expressed from the promoter.

The promoter could be an "inducible promoter" or "constitutive promoter." "Inducible promoter" refers to a promoter which can be induced by the presence or absence of certain factors, and "constitutive promoter" refers to an unregulated promoter that allows for continuous transcription of its associated gene or genes.

In a preferred embodiment, both the nucleotide sequences encoding the helper protein and the POI are driven by an inducible promoter. In another preferred embodiment, both the nucleotide sequences encoding the helper protein and POI is driven by a constitutive promoter. In yet another preferred embodiment, the nucleotide sequences encoding the helper protein is driven by a constitutive promoter and the POI is driven by an inducible promoter. In yet another preferred embodiment, the nucleotide sequences encoding the helper protein is driven by an inducible promoter and the POI is driven by a constitutive promoter. As an example, the HP may be driven by a constitutive GAP promoter and the POI may be driven by an inducible AOX1 promoter. In one embodiment, the nucleotide sequences encoding the helper protein and POI is driven by the same promoter or similar promoters in terms of promoter activity and/or expression behaviour.

Many inducible promoters are known in the art. Many are described in a review by Gatz, Curr. Op. Biotech., 7: 168 (1996) (see also Gatz, Ann. Rev. Plant. Physiol. Plant Mol. Biol., 48:89 (1997)). Examples include tetracycline repressor system, Lac repressor system, copper-inducible systems, salicylate-inducible systems (such as the PR1 a system), glucocorticoid-inducible (Aoyama et al., 1997), alcohol-inducible systems, e.g., AOX promoters, and ecdysome-inducible systems. Also included are the benzene sulphonamide-inducible (U.S. Patent No. 5,364,780) and alcohol-inducible (WO 97/06269 and WO 97/06268) inducible systems and glutathione S-transferase promoters.

Suitable promoter sequences for use with yeast host cells are described in Mattanovich et al., Methods Mol. Biol. (2012) 824:329-58 and include promoters of glycolytic enzymes like triosephosphate isomerase (TPI), phosphoglycerate kinase (PGK), glyceraldehyde-3-phosphate dehydrogenase (GAPDH or GAP) and variants thereof, lactase (LAC) and galactosidase (GAL), *P. pastoris* glucose-6-phosphate isomerase promoter (PPGI), the 3-phosphoglycerate kinase promoter (PPGK), the glycerol aldehyde phosphate dehydrogenase promoter (PGAP), translation elongation factor promoter (PTEF), and the promoters of *P. pastoris* enolase 1 (PENO1), triose phosphate isomerase (PTPI), ribosomal subunit proteins (PRPS2, PRPS7, PRPS31, PRPL1), alcohol oxidase promoter (PAOX) or variants thereof with modified characteristics, the formaldehyde dehydrogenase promoter (PFLD), isocitrate lyase promoter (PICL), alpha-ketoisocaproate decarboxylase promoter (PTHI), the promoters of heat shock protein family members (PSSA1, PHSP90, PKAR2), 6-Phosphogluconate dehydrogenase (PGND1), phosphoglycerate mutase (PGPM1), transketolase (PTKL1), phosphatidylinositol synthase (PPIS1), ferro-O2-oxidoreductase (PFET3), high affinity iron permease (PFTR1), repressible alkaline phosphatase (PPHO8), N-myristoyl transferase (PNMT1), pheromone response transcription factor (PMCM1), ubiquitin (PUBI4), single-stranded DNA endonuclease (PRAD2), the promoter of the major ADP/ATP carrier of the mitochondrial inner membrane (PPET9) (WO2008/128701) and the formate dehydrogenase (FMD) promoter. The GAP promoter, AOX promoter or a promoter derived from GAP or AOX promoter is particularly preferred. AOX promoters can be induced by methanol and are repressed by e.g. glucose.

Further examples of suitable promoters include Saccharomyces cerevisiae enolase (ENO-1), Saccharomyces cerevisiae galactokinase (GAL1), Saccharomyces cerevisiae alcohol dehydrogenase/glyceraldehyde-3-phosphate dehydrogenase (ADH1, ADH2/GAP), Saccharomyces cerevisiae triose phosphate isomerase (TPI), Saccharomyces cerevisiae metallothionein (CUP1), and Saccharomyces cerevisiae 3-phosphoglycerate kinase (PGK), and the maltase gene promoter (MAL).

Other useful promoters for yeast host cells are described by Romanos et al, 1992, Yeast 8:423-488.

Suitable promoter sequences for use with *E. coli* include T7 promoter, T5 promoter, tryptophan (trp) promoter, lactose (lac) promoter, tryptophan/lactose (tac) promoter, lipoprotein (Ipp) promoter, and λ phage PL promoter in plasmids.

The promoter which drives the expression of the polynucleotide encoding the helper protein is preferably not the endogenous promoter of the helper gene. Preferably, a recombinant promoter is used instead of the endogenous promoter of the helper protein gene.

Enhancer

In a preferred embodiment, the overexpression is achieved by using an enhancer to enhance the promoter activity which drives the expression of the helper protein. Transcriptional enhancers are relatively orientation and position independent, having been found 5' and 3' to the transcription unit, within an intron, as well as within the coding sequence itself. The enhancer may be spliced into the expression vector at a position 5' or 3' to the coding sequence, but is preferably located at a site 5' from the promoter. Most yeast genes contain only one UAS, which generally lies within a few hundred base pairs of the cap site and most yeast enhancers (UASs) cannot function when located 3' of the promoter, but enhancers in higher eukaryotes can function both 5' and 3' of the promoter.

Many enhancer sequences are now known from mammalian genes (globin, RSV, SV40, EMC, elastase, albumin, a-fetoprotein and insulin). One may also use an enhancer from a eukaryotic cell virus, such as the SV40 late enhancer, the cytomegalovirus early promoter enhancer, the polyoma enhancer on the late side of the replication origin, and adenovirus enhancers. Yeast enhancers, also called upstream activating sequences (UASs), such as the UAS/Gal system from *Saccharomyces cerevisiae,* can be advantageously used with yeast promoters (described in European Patent No. 0317254 and Rudoni et al., The International Journal of Biochemistry and Cell Biology, (2000), 32(2):215-224).

In a preferred embodiment, 2, 3, 4, 5, 6, 7, 8, 9 or more types of helper proteins disclosed by present invention are overexpressed. For example, the host cell can be engineered to overexpress 2, 3, 4, 5, 6, 7, 8, 9 or more of helper proteins selected from SEQ ID NO: 1, 2, 3, 4, 5, 6, 7, 8, 9, 10, 11, 12, 13, or 14 or functional homologues thereof, where a functional homologue thereof has an amino acid having at least 30% sequence identity to SEQ ID NO: 1, 2, 3, 4, 5, 6, 7, 8, 9, 10, 11, 12, 13, or 14.

Protein of interest

It is envisioned that when the host cell may be cultured under a suitable condition for the coexpression of the helper protein and the protein of interest, the host cell would express the protein of interest and overexpresses the polynucleotide encoding a helper protein having an amino acid sequence as shown in SEQ ID NO: 1, 2, 3, 4, 5, 6, 7, 8, 9, 10, 11, 12, 13, or 14 or a functional homologue thereof, wherein the functional homologue has at least 30% sequence identity to an amino acid sequence as shown in any one of SEQ ID NO: 1, 2, 3, 4, 5, 6, 7, 8, 9, 10, 11, 12, 13, or 14.

The term "protein of interest" (POI) as used herein refers to a protein that is produced by means of recombinant technology in a host cell. More specifically, the protein may either be a polypeptide not naturally occurring in the host cell, i.e. a heterologous protein, or else may be native to the host cell, i.e. a homologous protein to the host cell, but is produced, for example, by transformation with a self-replicating vector containing the nucleic acid sequence encoding the POI, or upon integration by recombinant techniques of one or more copies of the nucleic acid sequence encoding the POI into the genome of the host cell, or by recombinant modification of one or more regulatory sequences controlling the expression of the gene encoding the POI, e.g. of the promoter sequence. In general, the proteins of interest referred to herein may be produced by methods of recombinant expression well known to a person skilled in the art.

There is no limitation with respect to the protein of interest (POI). The POI is usually a eukaryotic or prokaryotic polypeptide, variant or derivative thereof. The POI can be any eukaryotic or prokaryotic protein. Examples of POIs are described in Schmidt, Appl. Microbiol. Biotechnol. (2004), 65: 363-372 or in Kirk et al., Curr. Opin. Biotechnol. (2002), 13: 345-351. Any of the proteins mentioned in Tables 1 and 2 of Schmidt and in Table 1 of Kirk et al. is encompassed by the term "POI" as used herein. The protein can be a naturally secreted protein or an intracellular protein, i.e. a protein which is not naturally secreted. The present invention also includes biologically active fragments of proteins. In another embodiment, a POI may be an amino acid chain or present in a complex, such as a dimer, trimer, hetero-dimer, multimer or oligomer.

The protein of interest may be a protein used as nutritional, dietary, digestive, supplements, such as in food products, feed products, or cosmetic products. The food products may be, for example, bouillon, desserts, cereal bars, confectionery, sports drinks, dietary products or other nutrition products. Preferably, the protein of interest is a food additive.

In another embodiment, the protein of interest may be used in animal feeds. The POI may be a detoxifying enzyme such as a mycotoxin degrading enzyme. A detoxifying enzyme means an enzyme which breaks down a toxin such to reduce its toxicity. Mycotoxins are toxic secondary metabolites produced by fungi that readily colonize crops and are often characterized by the ability to harm crops and cause health problems for people and animals. Mycotoxin degrading enzymes include aflatoxin detoxizyme, zearalenone esterases, zearalenone lactonases, zearalenone hydrolase, fcarboxylesterases, aminotransferases, aminopolyol amine oxidases, deoxynivalenol expoxide hydrolases. The POI may also be an enzyme which degrades ochratoxin derivatives or ergot alkaloid. Ochratoxins are a group of mycotoxins produced as secondary metabolites by several fungi of the *Aspergillus* or *Penicillium* families and are weak organic acids consisting of a derivative of an isocoumarin. There are three generally recognized ochratoxins, designated A, B and C. Ochratoxin A is the most abundant member of the ochratoxin family and hence the most commonly detected, but is also the most toxic. Ochratoxin A (ochratoxin A) is a nephrotoxic, teratogenic, hepatotoxic, and carcinogenic mycotoxin present in cereals and other starch rich foods. Ergot alkaloids are compounds containing amide bonds and include, for example, ergocornine, ergocorninine, ergocristine, ergocristinine, ergocryptine, ergocryptinine, ergometrine, ergosine, ergotamine and ergotaminine. These compounds are toxic to living organisms including humans and farm animals. Examples of such enzymes include ochratoxin amidase, ergotamine hydrolase, ergotamine amylase. Mycotoxin degrading enzymes in animal feed is useful in controlling mycotoxin contamination of feed.

Further examples of POI include anti-microbial proteins, such as lactoferrin, lysozyme, lactoferricin, lactohedrin, kappa-casein, haptocorrin, lactoperoxidase, a milk protein, acute-phase proteins, e.g., proteins that are produced normally in production animals in response to infection, and small anti-microbial proteins such as lysozyme and lactoferrin. Other examples include bactericidal protein, antiviral proteins, acute phase proteins (induced in production animals in response to infection), probiotic proteins, bacteriostatic protein, and cationic antimicrobial proteins.

"Feed" means any natural or artificial diet, meal or the like or components of such meals intended or suitable for being eaten, taken in, digested, by a non-human animal. A "feed additive" generally refers to substances added in a feed. It typically includes one or more compounds such as vitamins, minerals, enzymes and suitable carriers and/or excipient. For the present invention, a food additive may be an enzyme or other proteins. Examples of enzymes which can be used as feed additive include phytase, xylanase and β-glucanase.A "food" means any natural or artificial diet meal or the like or components of such meals intended or suitable for being eaten, taken in, digested, by a human being.

A "food additive" generally refers to substances added in a food. It typically includes one or more compounds such as vitamins, minerals, enzymes and suitable carriers and/or excipient. For the present invention, a food additive may be an enzyme or other proteins. Examples of enzymes which can be used as food additive include protease, lipase, lactase, pectin methyl esterase, pectinase, transglutaminase, amylase, β-glucanase, acetolactate decarboxylase and laccase.

In some embodiments, the food additive is an anti-microbial protein, which includes, for example, (i) antimicrobial milk proteins (either human or non-human) lactoferrin, lysozyme, lactoferricin, lactohedrin, kappa-casein, haptocorrin, lactoperoxidase, alpha- 1-antitrypsin, and immunoglobulins, e.g., IgA, (ii) acute-phase proteins, such as C-reactive protein (CRP); lactoferrin; lysozyme; serum amyloid A (SAA); ferritin; haptoglobin (Hp); complements 2-9, in particular complement-3; seromucoid; ceruloplasmin (Cp); 15-keto-13,14-dihydro- prostaglandin F2 alpha (PGFM); fibrinogen (Fb); alpha(1)-acid glycoprotein (AGP); alpha(1)-antitrypsin; mannose binding protein; lipoplysaccharide binding protein; alpha-2 macroglobulin and various defensins, (iii) antimicrobial peptides, such as cecropin, magainin, defensins, tachyplesin, parasin I.buforin I, PMAP-23, moronecidin, anoplin, gambicin, and SAMP-29, and (iv) other antimicrobial protein(s), including CAP37, granulysin, secretory leukocyte protease inhibitor, CAP18, ubiquicidin, bovine antimicrobial protein-1, Ace- AMP1, tachyplesin, big defensin, Ac-AMP2, Ah-AMP1 , and CAP18.

Enzyme

A POI may be an enzyme. Preferred enzymes are those which can be used for industrial application, such as in the manufacturing of a detergent, starch, fuel, textile, pulp and paper, oil, personal care products, or such as for baking, organic synthesis, and the like. Examples of such enzymes include protease, amylase, lipase, mannanase and cellulose for stain removal and cleaning; pullulanase amylase and amyloglucosidase for starch liquefaction and saccharification; glucose isomerase for glucose to fructose conversion; cyclodextrin-glycosyltransferase for cyclodextrin production; xylanase for xiscosity reduction in fuel and starch; amylase, xylanase, lipase, phospholipase, glucose, oxidase, lipoxygenase, transglutaminase for dough stability and conditioning in baking; cellulase in textile manufacturing for denim finishing and cotton softening; amylase for de-sizing of texile; pectate lyase for scouring; catalase for bleach termination; laccase for bleaching; peroxidase for excess dye removal; lipase, protease, amylase, xylanase, cellulose, in pulp and paper production; lipase for transesterification and phospholipase for de-gumming in fat processing fats and oils; lipase for resolution of chiral alcohols and amides in organic synthesis; acylase for synthesis of semisynthetic penicillin, nitrilase for the synthesis of enantiopure carboxylic acids; protease and lipase for leather production; amyloglucosidase, glucose oxidase, and peroxidase for the making personal care products (see Kirk et al., Current Opinion in Biotechnology (2002) 13:345-351)

Therapeutic protein

A POI may be a therapeutic protein. A POI may be but is not limited to a protein suitable as a biopharmaceutical substance like an antibody or antibody fragment, growth factor, hormone, enzyme, vaccine, etc. as described in more detail herein.

The POI may be a naturally secreted protein or an intracellular protein, i.e. a protein which is not naturally secreted. The present invention also provides for the recombinant production of functional homologues, functional equivalent variants, derivatives and biologically active fragments of naturally secreted or not naturally secreted proteins. Functional homologues are preferably identical with or correspond to and have the functional characteristics of a sequence.

The POI may be structurally similar to the native protein and may be derived from the native protein by addition of one or more amino acids to either or both the C- and N-terminal end or the side-chain of the native protein, substitution of one or more amino acids at one or a number of different sites in the native amino acid sequence, deletion of one or more amino acids at either or both ends of the native protein or at one or several sites in the amino acid sequence, or insertion of one or more amino acids at one or more sites in the native amino acid sequence. Such modifications are well known for several of the proteins mentioned above.

Preferably, the protein of interest is a mammalian polypeptide or even more preferably a human polypeptide. Especially preferred therapeutic proteins, which refer to any polypeptide, protein, protein variant, fusion protein and/or fragment thereof which may be administered to a mammal. It is envisioned but not required that therapeutic protein according to the present invention is heterologous to the cell. Examples of proteins that can be produced by the cell of the present invention are, without limitation, enzymes, regulatory proteins, receptors, peptide hormones, growth factors, cytokines, scaffold binding proteins (e.g. anticalins), structural proteins, lymphokines, adhesion molecules, receptors, membrane or transport proteins, and any other polypeptides that can serve as agonists or antagonists and/or have therapeutic or diagnostic use. Moreover, the proteins of interest may be antigens as used for vaccination, vaccines, antigen-binding proteins, immune stimulatory proteins. It may also be an antigen-binding fragment of an antibody, which can include any suitable antigen-binding antibody fragment known in the art. The term antibody when used herein includes fragments and variants as described in more detail hereinbelow. For example, an antibody fragment may include but is not not limited to Fv (a molecule comprising the VL and VH), single-chain Fv (scFV) (a molecule comprising the VL and VH connected with by peptide linker), Fab, Fab', $F(ab')_2$, single domain antibody (sdAb) (molecules comprising a single variable domain and 3 CDR), and multivalent presentations thereof. The antibody or fragments thereof may be murine, human, humanized or chimeric antibody or fragments thereof. Examples of therapeutic proteins include an antibody, polyclonal antibody, monoclonal antibody, recombinant antibody, antibody fragments, such as Fab', F(ab')2, Fv, scFv, di-scFvs, bi-scFvs, tandem scFvs, bispecific tandem scFvs, sdAb, nanobodies, $V_H$, and $V_L$, or human antibody, humanized antibody, chimeric antibody, IgA antibody, IgD antibody, IgE antibody, IgG antibody, IgM antibody, intrabody, minibody or monobody.

Such therapeutic proteins include, but are not limited to, insulin, insulin-like growth factor, hGH, tPA, cytokines, e.g. interleukines such as IL-1, IL-2, IL-3, IL-4, IL-5, IL-6, IL-7, IL-8, IL-9, IL-10, IL-11, IL-12, IL-13, IL-14, IL-15, IL-16, IL-17, IL-18, interferon (IFN) alpha, IFN beta, IFN gamma, IFN omega or IFN tau, tumor necrosisfactor (TNF) TNF alpha and TNF beta, TRAIL; G-CSF, GM-CSF, M-CSF, MCP-1 and VEGF.

In a preferred embodiment, the protein is an antibody. The term "antibody" is intended to include any polypeptide chain-containing molecular structure with a specific shape that fits to and recognizes an epitope, where one or more non-covalent binding interactions stabilize the complex between the molecular structure and the epitope. The archetypal antibody molecule is the immunoglobulin, and all types of immunoglobulins, IgG, IgM, IgA, IgE, IgD, etc., from all sources, e.g. human, rodent, rabbit, cow, sheep, pig, dog, other mammals, chicken, other avians, etc., are considered to be "antibodies." Numerous antibody coding sequences have been described; and others may be raised by methods well-known in the art.

For example, antibodies or antigen binding fragments may be produced by methods known in the art. Generally, antibody-producing cells are sensitized to the desired antigen or immunogen. The messenger RNA isolated from antibody producing cells is used as a template to make cDNA using PCR amplification. A library of vectors, each containing one heavy chain gene and one light chain gene retaining the initial antigen specificity, is produced by insertion of appropriate sections of the amplified immunoglobulin cDNA into the expression vectors. A combinatorial library is constructed by combining the heavy chain gene library with the light chain gene library. This results in a library of clones which co-express a heavy and light chain (resembling the Fab fragment or antigen binding fragment of an antibody molecule). The vectors that carry these genes are co-transfected into a host cell. When antibody gene synthesis is induced in the transfected host, the heavy and light chain proteins self-assemble to produce active antibodies that can be detected by screening with the antigen or immunogen.

Antibody coding sequences of interest include those encoded by native sequences, as well as nucleic acids that, by virtue of the degeneracy of the genetic code, are not identical in sequence to the disclosed nucleic acids, and variants thereof. Variant polypeptides can include amino acid (aa) substitutions, additions or deletions. The amino acid substitutions can be conservative amino acid substitutions or substitutions to eliminate non-essential amino acids, such as to alter a glycosylation site, or to minimize misfolding by substitution or deletion of one or more cysteine residues that are not necessary for function. Variants can be designed so as to retain or have enhanced biological activity of a particular region of the protein (e.g., a functional domain, catalytic amino acid residues, etc). Variants also include fragments of the polypeptides disclosed herein, particularly biologically active fragments and/or fragments corresponding to functional domains. Techniques for in vitro mutagenesis of cloned genes are known. Also included in the subject invention are polypeptides that have been modified using ordinary molecular biological techniques so as to improve their resistance to proteolytic degradation or to optimize solubility properties or to render them more suitable as a therapeutic agent.

Chimeric antibodies may be made by recombinant means by combining the variable light and heavy chain regions (VK and VH), obtained from antibody producing cells of one species with the constant light and heavy chain regions from another. Typically, chimeric antibodies utilize rodent or rabbit variable regions and human constant regions, in order to produce an antibody with predominantly human domains. The production of such chimeric antibodies is well known in the art, and may be achieved by standard means (as described, e.g., in U.S. Patent No. 5,624,659).

Humanized antibodies are engineered to contain even more human-like immunoglobulin domains, and incorporate only the complementarity-determining regions of the animal-derived antibody. This is accomplished by carefully examining the sequence of the hyper-variable loops of the variable regions of the monoclonal antibody, and fitting them to the structure of the human antibody chains. Although facially complex, the process is straightforward in practice. See, e.g., U.S. Patent No. 6,187,287.

In addition to entire immunoglobulins (or their recombinant counterparts), immunoglobulin fragments comprising the epitope binding site (e.g., Fab', F(ab')2, or other fragments) may be synthesized. "Fragment" or minimal immunoglobulins may be designed utilizing recombinant immunoglobulin techniques. For instance "Fv" immunoglobulins for use in the present invention may be produced by synthesizing a variable light chain region and a variable heavy chain region. Combinations of antibodies are also of interest, e.g. diabodies, which comprise two distinct Fv specificities.

Immunoglobulins may be modified post-translationally, e.g. to add chemical linkers, detectable moieties, such as fluorescent dyes, enzymes, substrates, chemiluminescent moieties and the like, or specific binding moieties, such as streptavidin, avidin, or biotin, and the like may be utilized in the methods and compositions of the present invention.

Further examples of therapeutic proteins include blood coagulation factors (VII, VIII, IX), alkaline protease from Fusarium, calcitonin, CD4 receptor darbepoetin, DNase (cystic fibrosis), erythropoetin, eutropin (human growth hormone derivative), follicle stimulating hormone (follitropin), gelatin, glucagon, glucocerebrosidase (Gaucher disease), glucosamylase from A. niger, glucose oxidase from A. niger, gonadotropin, growth factors (GCSF, GMCSF), growth hormones (somatotropines), hepatitis B vaccine, hirudin, human antibody fragment, human apolipoprotein AI, human calcitonin precursor, human collagenase IV, human epidermal growth factor, human insulin-like growth factor, human interleukin 6, human laminin, human proapolipoprotein AI, human serum albumininsulin, insulin and muteins, insulin, interferon alpha and muteins, interferon beta, interferon gamma (mutein), interleukin 2, luteinization hormone, monoclonal antibody 5T4, mouse collagen, OP-1 (osteogenic, neuroprotective factor), oprelvekin (interleukin 11-agonist), organophosphohydrolase, PDGF-agonist, phytase,platelet derived growth factor (PDGF), recombinant plasminogen-activator G, staphylokinase, stem cell factor, tetanus toxin fragment C, tissue plasminogen-activator, and tumor necrosis factor (see Schmidt, Appl Microbiol Biotechnol (2004) 65:363-372).

Leader sequence

The protein of interest may be linked with a leader sequence which causes secretion of the POI from the host cell. The presence of such a secretion leader sequence in the expression vector is required when the POI intended for recombinant expression and secretion is a protein which is not naturally secreted and therefore lacks a natural secretion leader sequence, or its nucleotide sequence has been cloned without its natural secretion leader sequence. In general, any secretion leader sequence effective to cause secretion of the POI from the host cell may be used in the present invention. The secretion leader sequence may originate from yeast source, e.g. from yeast α-factor such as MFa of *Saccharomyces cerevisiae,* or yeast phosphatase, from mammalian or plant source, or others. The selection of the appropriate secretion leader sequence is apparent to a skilled person. Alternatively, the secretion leader sequence can be fused to the nucleotide sequence encoding a POI intended for recombinant expression by conventional cloning techniques known to a skilled person prior to cloning of the nucleotide sequence in the expression vector or the nucleotide sequence encoding a POI comprising a natural secretion leader sequence is cloned in the expression vector. In these cases the presence of a secretion leader sequence in the expression vector is not required.

The recombinant nucleotide sequence encoding the POI(s), as well as those encoding the helper proteins, may also be provided on one or more autonomously replicating plasmids in a single copy or in multiple copies per cell.

Alternatively, the recombinant nucleotide sequence encoding the POI and the recombinant nucleotide sequence encoding a helper protein are present on the same plasmid in single copy or multiple copies per cell.

In a further aspect, the present invention provides the use of the engineered host cells for manufacturing a protein of interest. The host cells can be advantageously used for introducing polypeptides encoding one or more POI(s), and thereafter can be cultured under suitable conditions to express the POI. Details of such use are described in the later section concerning methods of the present invention.

Polynucleotides encoding the helper proteins and the POI may be recombined in to the host cell by ligating the relevant genes each into one vector. It is possible to construct single vectors carrying the genes, or two separate vectors, one to carry the helper protein genes and the other one the POI genes. These genes can be integrated into the host cell genome by transforming the host cell using such vector or vectors. In some embodiments, the genes encoding the POI is integrated in the genome and the gene encoding the helper protein is integrated in a plasmid or vector. In some embodiments, the genes encoding the helper protein is integrated in the genome and the gene encoding the POI is integrated in a plasmid or vector. In some embodiments, the genes encoding the POI and the helper protein are integrated in the genome. In some embodiments, the gene encoding the POI and the helper protein is integrated in a plasmid or vector. If multiple genes encoding the POI are used, some genes encoding the POI are integrated in the genome while others are integrated in the same or different plasmids or vectors. If multiple genes encoding the helper proteins are used, some of the genes encoding the helper protein are integrated in the genome while others are integrated in the same or different plasmids or vectors. More teaching ca be found in the following sections of the application.

Generally, proteins of interest can be produced using the recombinant host cell by culturing the host cell in an appropriate medium, isolating the expressed POI from the culture, and purifying it by a method appropriate for the expressed product, in particular to separate the POI from the cell.

In further aspect, the present invention relates to a method of increasing the yield of a protein of interest in a host cell, comprising overexpressing a polynucleotide of the present invention. The polynucleotide encodes a helper protein having an amino acid sequence having at least 30% sequence identity to an amino acid sequence as shown in any one of SEQ ID NO: 1, 2, 3, 4, 5, 6, 7, 8, 9, 10, 11, 12, 13, or 14.

As used herein, the term "increasing the yield of a protein of interest in a host cell" means that the yield of the protein of interest is increased when compared to the same cell expressing the same POI under the same culturing conditions, however, without the polynucleotide encoding the helper protein being overexpressed.

As will be appreciated by a skilled person in the art, the overexpression of the helper proteins of the present invention have been shown to increase product yield of POI. Therefore, for a given host cell which expressed a POI with a level that should be increased, it is possible to apply the present invention by expressing any one or several of the helper proteins in the host cell, if helper protein is not present in the host cell, or further increasing the level of expression the helper proteins in the cell, if genes encoding the helper protein is already present in the host cell.

In yet a further aspect, the present invention provides a method of increasing the yield of a protein of interest in a host cell. The method comprises (i) engineering the host cell to express or overexpress a helper protein, (ii) recombining in said host cell a heterologous polynucleotide encoding a protein of interest, and (iii) culturing said host cell under suitable conditions to express the helper protein and the protein of interest. It should be noted that the steps recited in (i) and (ii) do not have to be performed in the recited sequence. It is possible to first perform the step recited in (ii) and then (i). In step (i), the host cell can be engineered to overexpress a polynucleotide encoding a helper protein having an amino acid having at least 30% sequence identity to an amino acid sequence as shown in any one of SEQ ID NO: 1, 2, 3, 4, 5, 6, 7, 8, 9, 10, 11, 12, 13, or 14.

Procedures used to manipulate polynucleotide sequences, e.g. coding for the helper proteins and/or the POI, the promoters, enhancers, leaders, etc., are well known to persons skilled in the art, e.g. described by J. Sambrook et al., Molecular Cloning: A Laboratory Manual (3rd edition), Cold Spring Harbor Laboratory, Cold Spring Harbor Laboratory Press, New York (2001).

A foreign or target polynucleotide such as the polynucleotdies encoding the helper protein or POI can be inserted into the chromosome by various means, e.g., by homologous recombination or by using a hybrid recombinase that specifically targets sequences at the integration sites. The foreign or target polynucleotide described above is typically present in a vector ("inserting vector"). These vectors are typically circular and linearized before used for homologous recombination. As an alternative, the foreign or target polynucleotides may be DNA fragments joined by fusion PCR or synthetically constructed DNA fragments which are then recombined into the host cell. In addition to the homology arms, the vectors may also contain markers suitable for selection or screening, an origin of replication, and other elements. It is also possible to use heterologous recombination which results in random or non-targeted integration. Heterologous recombination refers to recombination between DNA molecules with significantly different sequences. Methods of recombinations are known in the art and for example described in Boer et al., Appl Microbiol Biotechnol (2007) 77:513-523. One may also refer to Principles of Gene Manipulation and Genomics by Primrose and Twyman (7th edition, Blackwell Publishing 2006) for genetic manipulation of yeast cells.

Polynucleotides encoding the helper protein and/or POI may also be present on an expression vector. Such vectors are known in the art and already described above. In expression vectors, a promoter is placed upstream of the gene encoding the heterologous protein and regulates the expression of the gene. Multi-cloning vectors are especially useful due to their multi-cloning site. For expression, a promoter is generally placed upstream of the multi-cloning site. A vector for integration of the polynucleotide encoding a helper protein and/or the POI may be constructed either by first preparing a DNA construct containing the entire DNA sequence coding for the helper protein and/or the POI and subsequently inserting this construct into a suitable expression vector, or by sequentially inserting DNA fragments containing genetic information for the individual elements, such as the leader sequence, the target DNA sequence, followed by ligation. As an alternative to restriction and ligation of fragments, recombination methods based on attachment sites (att) and recombination enzymes may be used to insert DNA sequences into a vector. Such methods are described, for example, by Landy (1989) Ann. Rev. Biochem. 58:913-949; and are known to those of skill in the art.

Host cells according to the present invention can be obtained by introducing a vector or plasmid comprising the target polynucleotide sequences into the cells. Techniques for transfecting or transforming eukaryotic cells or transforming prokaryotic cells are well known in the art. These can include lipid vesicle mediated uptake, heat shock mediated uptake, calcium phosphate mediated transfection (calcium phosphate/DNA co-precipitation), viral infection, particularly using modified viruses such as, for example, modified adenoviruses, microinjection and electroporation. For prokaryotic transformation, techniques can include heat shock mediated uptake, bacterial protoplast fusion with intact cells, microinjection and electroporation. Techniques for plant transformation include *Agrobacterium* mediated transfer, such as by *A. tumefaciens,* rapidly propelled tungsten or gold microprojectiles, electroporation, microinjection and polyethylyne glycol mediated uptake. The DNA can be single or double stranded, linear or circular, relaxed or supercoiled DNA. For various techniques for transfecting mammalian cells, see, for example, Keown et al. (1990) Processes in Enzymology 185:527-537.

In a further aspect, the present invention provides a method of manufacturing a protein of interest in a host cell comprising (i) providing the host cell engineered to overexpress a polynucleotide encoding a helper protein having an amino acid having at least 30% sequence identity to an amino acid sequence as shown in SEQ ID NO: 1, 2, 3, 4, 5, 6, 7, 8, 9, 10, 11, 12, 13, or 14, wherein said host cell comprises a heterologous polynucleotide encoding a protein of interest; and (ii) culturing the host cell under suitable conditions to overexpress the helper protein and express protein of interest.

It is understood that the methods disclosed herein may further include cultivating said recombinant host cells under conditions permitting the expression of the POI and helper protein. A recombinantly produced POI can then be isolated from the cell or the cell culture medium, depending on the nature of the expression system and the expressed protein, e.g. whether the protein is fused to a signal peptide and whether the protein is soluble or membrane-bound. As will be understood by the skilled artisan, cultivation conditions will vary according to factors that include the type of host cell, in particular the expression vector employed. Signal peptides generally contain a positively charged N-terminus followed by a hydrophobic core, followed by a recognition site for an enzyme known as signal peptidase. This enzyme cleaves the signal peptide from the protein during translocation. The protein is transported from the endoplasmic reticulum to the Golgi apparatus, and then follows one of a number of routes in the secretory pathway, depending on the nature of the protein. The protein may be secreted into the culture medium or may be retained on the cell surface, for example. Certain receptors that comprise extracellular, transmembrane, and cytoplasmic domains are examples of proteins that may be retained on the cell membrane, with only the extracellular domain located outside the cell. The leader sequences of certain secreted proteins comprise peptides that are located C-terminal to the signal peptide and are processed from the mature protein of interest subsequent to cleavage of the signal peptide. Such leaders often are referred to as prepro peptides, wherein the pre region is the signal sequence and the pro region designates the remainder of the leader.

One example is the yeast $\alpha$-factor leader, which contains a signal peptide (including a C-terminal signal peptidase recognition site AlaLeuAla) followed by a pro region containing a basic amino acid pair LysArg that constitutes a KEX2 protease processing site, immediately followed by a peptide GluAlaGluAla at the C-terminus of the pro region. Processing of this leader involves removal of the signal peptide by signal peptidase, followed by cleavage between the Lys and Arg residues by KEX2 protease. The GluAlaGluAla residues are subsequently removed by a peptidase that is the product of the STE13 gene (Julius et al., Cell (1983) 32:839). The yeast $\alpha$-factor leader is described in U.S. Patent 4,546,082. Signal peptides derived from proteins naturally secreted by yeast cells have been employed in recombinant expression systems for production of heterologous proteins in yeast. The use of mammalian signal peptides in yeast expression systems also has been reported, although certain of the mammalian signal peptides were not effective in promoting secretion of heterologous proteins in yeast.

The phrase "culturing under suitable condition such that a desired polypeptide is expressed" refers to maintaining and/or growing microorganisms under conditions (e.g., temperature, pressure, pH, duration, etc.) appropriate or sufficient to obtain production of the desired compound or to obtain desired polypeptide.

A host cell according to the invention obtained by transformation with the helper protein gene(s) and/or the POI genes may preferably first be cultivated at conditions to grow efficiently to a large cell number without the burden of expressing a heterologous protein. When the cells are prepared for POI expression, suitable cultivation conditions are selected and optimized to produce the POI.

By way of example, using different promoters and/or copies and/or integration sites for the helper gene(s) and the POI(s), the expression of the helper genes can be controlled with respect to time point and strength of induction in relation to the expression of the POI(s). For example, prior to induction of POI expression, the helper protein(s) may be first expressed. This has the advantage that the helper proteins is/are already present at the beginning of POI translation. Alternatively, the helper protein(s) and POI(s) can be induced at the same time.

An inducible promoter may be used that becomes activated as soon as an inductive stimulus is applied, to direct transcription of the gene under its control. Under growth conditions with an inductive stimulus, the cells usually grow more slowly than under normal conditions, but since the culture has already grown to a high cell number in the previous stage, the culture system as a whole produces a large amount of the heterologous protein. An inductive stimulus is preferably the addition of an appropriate agents (e.g. methanol for the AOX-promoter) or the depletion of an appropriate nutrient (e.g., methionine for the MET3-promoter). Also, the addition of ethanol, methylamine, cadmium or copper as well as heat or an osmotic pressure increasing agent can induce the expression.

It is preferred to cultivate the hosts according to the invention in a bioreactor under optimized growth conditions to obtain a cell density of at least 1 g/L, preferably at least 10 g/L cell dry weight, more preferably at least 50 g/L cell dry weight. It is advantageous to achieve such yields of biomolecule production not only on a laboratory scale, but also on a pilot or industrial scale.

According to the present invention, due to co-expression of the helper proteins, the POI is obtainable in high yields, even when the biomass is kept low. Thus, a high specific yield, which is measured in mg POI/g dry biomass, may be in the range of 0.1 to 100, 0.1 to 200, such as 0.2, 0.3, 0.4, or 0.5 to 100, such as 0.2, 0.3, 0.4, or 0.5 to 200, such as 1-100 or 1-200, in the laboratory, pilot and industrial scale is feasible. The specific yield of a production host according to the invention preferably provides for an increase of at least 1.1 fold, more preferably at least 1.2 fold, at least 1.3 or at least 1.4 fold, in some cases an increase of more than 2 fold can be shown, when compared to the expression of the product without the overexpression of helper proteins.

The host cell according to the invention may be tested for its expression/secretion capacity or yield by standard tests, e.g. ELISA, activity assays, HPLC, Surface Plasmon Resonance (Biacore), Western Blot, capillary electrophoresis (Caliper) or SDS-Page.

Preferably, the cells are cultivated in a minimal medium with a suitable carbon source, thereby further simplifying the isolation process significantly. By way of example, the minimal medium contains an utilizable carbon source (e.g. glucose, glycerol, ethanol or methanol), salts containing the macro elements (potassium, magnesium, calcium, ammonium, chloride, sulphate, phosphate) and trace elements (copper, iodide, manganese, molybdate, cobalt, zinc, and iron salts, and boric acid).

In the case of yeast cells, the cells may be transformed with one or more of the above-described expression vector(s), mated to form diploid strains, and cultured in conventional nutrient media modified as appropriate for inducing promoters, selecting transformants or amplifying the genes encoding the desired sequences. A number of minimal media suitable for the growth of yeast are known in the art. Any of these media may be supplemented as necessary with salts (such as sodium chloride, calcium, magnesium, and phosphate), buffers (such as HEPES, citric acid and phosphate buffer), nucleosides (such as adenosine and thymidine), antibiotics, trace elements, vitamins, and glucose or an equivalent energy source. Any other necessary supplements may also be included at appropriate concentrations that would be known to those skilled in the art. The culture conditions, such as temperature, pH and the like, are those previously used with the host cell selected for expression and are known to the ordinarily skilled artisan. Cell culture conditions for other type of host cells are also known and can be readily determined by the artisan. Descriptions of culture media for various microorganisms are for example contained in the handbook "Manual of Methods for General Bacteriology" of the American Society for Bacteriology (Washington D.C, USA, 1981).

Cells can be cultured (e.g., maintained and/or grown) in liquid media and preferably are cultured, either continuously or intermittently, by conventional culturing methods such as standing culture, test tube culture, shaking culture (e.g., rotary shaking culture, shake flask culture, etc.), aeration spinner culture, or fermentation. In some embodiments, cells are cultured in shake flasks or deep well plates. In yet other embodiments, cells are cultured in a bioreactor (e.g., in a bioreactor cultivation process). Cultivation processes include, but are not limited to, batch, fed-batch and continuous methods of cultivation. The terms "batch process" and "batch cultivation" refer to a closed system in which the composition of media, nutrients, supplemental additives and the like is set at the beginning of the cultivation and not subject to alteration during the cultivation; however, attempts may be made to control such factors as pH and oxygen concentration to prevent excess media acidification and/or cell death. The terms "fed-batch process" and "fed-batch cultivation" refer to a batch cultivation with the exception that one or more substrates or supplements are added (e.g., added in increments or continuously) as the cultivation progresses. The terms "continuous process" and "continuous cultivation" refer to a system in which a defined cultivation media is added continuously to a bioreactor and an equal amount of used or "conditioned" media is simultaneously removed, for example, for recovery of the desired product. A variety of such processes has been developed and is well-known in the art.

In some embodiments, cells are cultured for about 12 to 24 hours, in other embodiments, cells are cultured for about 24 to 36 hours, about 36 to 48 hours, about 48 to 72 hours, about 72 to 96 hours, about 96 to 120 hours, about 120 to 144 hours, or for a duration greater than 144 hours. In yet other embodiments, culturing is continued for a time sufficient to reach desirable production yields of POI.

The above mentioned methods may further comprise a step of isolating the expressed POI. If the POI is secreted from the cells, it can be isolated and purified from the culture medium using state of the art techniques. Secretion of the POI from the cells is generally preferred, since the products are recovered from the culture supernatant rather than from the complex mixture of proteins that results when cells are disrupted to release intracellular proteins. A protease inhibitor, such as phenyl methyl sulfonyl fluoride (PMSF) may be useful to inhibit proteolytic degradation during purification, and antibiotics may be included to prevent the growth of adventitious contaminants. The composition may be concentrated, filtered, dialyzed, etc., using methods known in the art. Alternatively, cultured host cells may also be ruptured sonically or mechanically, enzymatically or chemically to obtain a cell extract containing the desired POI, from which the POI may be isolated and purified.

As isolation and purification methods for obtaining the POI may be based on methods utilizing difference in solubility, such as salting out and solvent precipitation, methods utilizing difference in molecular weight, such as ultrafiltration and gel electrophoresis, methods utilizing difference in electric charge, such as ion-exchange chromatography, methods utilizing specific affinity, such as affinity chromatography, methods utilizing difference in hydrophobicity, such as reverse phase high performance liquid chromatography, and methods utilizing difference in isoelectric point, such as isoelectric focusing may be used. Specific purification steps are preferably employed to remove any helper protein that is also expressed and would contaminate the POI preparation.

The isolated and purified POI can be identified by conventional methods such as Western Blotting or specific assays for its activity. The structure of the purified POI can be defined by amino acid analysis, amino-terminal analysis, primary structure analysis, and the like. It is preferred that the POI is obtainable in large amounts and in a high purity level, thus meeting the necessary requirements for being used as an active ingredient in pharmaceutical compositions or as feed or food additive.

It is to be understood that this invention is not limited to the particular methodology, protocols, cell lines, animal species or genera, constructs, and reagents described, as such may, of course, vary. It is also to be understood that the terminology used herein is for the purpose of describing particular embodiments only, and is not intended to limit the scope of the present invention, which will be limited only by the appended items.

Unless defined otherwise, all technical and scientific terms used herein have the same meaning as commonly understood to one of ordinary skill in the art to which this invention belongs. Although any methods, devices and materials similar or equivalent to those described herein can be used in the practice or testing of the invention, the preferred methods, devices and materials are now described.

All publications mentioned herein are for the purpose of describing and disclosing, for example, the cell lines, constructs, and methodologies that are described in the publications, which might be used in connection with the presently described invention. The publications discussed above and throughout the text are provided solely for their disclosure prior to the filing date of the present application. Nothing herein is to be construed as an admission that the inventors are not entitled to antedate such disclosure by virtue of prior invention.

The following examples are put forth so as to provide those of ordinary skill in the art with a complete disclosure and description of how to make and use the subject invention, and are not intended to limit the scope of what is regarded as the invention and defined in the items. Efforts have been made to ensure accuracy with respect to the numbers used (e.g. amounts, temperature, concentrations, etc.) but some experimental errors and deviations should be allowed for. Unless otherwise indicated, parts are parts by weight, molecular weight is average molecular weight, temperature is in degrees centigrade; and pressure is at or near atmospheric.

## Examples

The below examples will demonstrate that the newly identified target(s) increases the titer (product per volume in mg/L) and the yield (product per biomass in mg/g biomass measured as dry cell weight or wet cell weight), respectively, of recombinant proteins upon its/their overexpression. As an example, the yield of recombinant antibody Fab fragments and recombinant enzymes in the yeast *Pichia pastoris* are increased.

**Example 1:** Generation of *P. pastoris* production strains

a) Construction of *P. pastoris* strains secreting antibody Fab fragment HyHEL

*P. pastoris* CBS7435 (CBS, genome sequenced by Küberl et al. 2011) mut$^S$ variant was used as host strain. The pPM2d_pGAP and pPM2d_pAOX expression vectors are derivatives of the pPuzzle_ZeoR vector backbone described in WO2008/128701A2, consisting of the pUC19 bacterial origin of replication and the Zeocin antibiotic resistance cassette. Expression of the heterologous gene is mediated by the *P. pastoris* glyceraldehyde-3-phosphate dehydrogenase (GAP) promoter or alcohol oxidase 1 (AOX1) promoter, respectively, and the S. *cerevisiae* CYC1 transcription terminator. The genes encoding heavy chain (HC) and the light chain (LC) of the antibody Fab fragment HyHEL (SEQ ID NOs. 30 and 31, respectively) were codon optimized for *P. pastoris* and synthesized by GeneArt. The obtained vectors carrying the gene of interests with N-terminal S. *cerevisiae* alpha mating factor signal leader sequence were digested with SbfI and SfiI and the genes were each ligated into the vector pPM2d_pGAP and pPM2d_pAOX digested with *Sbf*I and *Sfi*I. The LC fragments were ligated into variants of pPM2d_pGAP and pPM2d_pAOX, where one restriction enzyme site in the promoter region was exchanged for another to allow subsequent linearization (*Nde*I instead of *Avr*II in pPM2d_pGAP, *Bsu36*I instead of *Bpu*1102I in pPM2d_pAOX), the HC fragments were ligated into the unmodified versions of the vectors. After sequence verification of LC and HC, the expression cassettes for both chains were combined onto one vector by using the compatible restriction enzymes *Mre*I and *Age*I.

Plasmids were linearized using *Nde*I restriction enzyme (for pPM2d_pGAP) or *Bsu36*I restriction enzyme (for pPM2d_pAOX), respectively, prior to electroporation (using a standard transformation protocol described in Gasser et al. 2013. Future Microbiol. 8(2):191-208) into *P. pastoris.* Selection of positive transformants was performed on YPD plates (per liter: 10 g yeast extract, 20 g peptone, 20 g glucose, 20 g agar-agar) containing 50 μg/mL of Zeocin. Colony PCR was used to ensure the presence of the transformed plasmid. Therefore, genomic DNA was obtained by cooking and freezing of *P. pastoris* colonies for 5 minutes each and directly applied for PCR with the appropriate primers.

b) Construction of a *P. pastoris* strain secreting antibody Fab fragment SDZ

The light chain (LC) and the heavy chain (HC) of the antibody Fab fragment SDZ (Fig. 2) were amplified from vector DNA template (carrying the genes of interest with N-terminal alpha mating factor signal leader sequence) using the primers for SDZ-HC and SDZ-LC in Table 3, and each ligated into pPM2d_pAOX or the variant of pPM2d_pAOX with the *Bsu36*I restriction site, respectively, each digested with *Sbf*I and *Sfi*I. After sequence verification of LC and HC, the expression cassettes for both chains were combined onto one vector by using the compatible restriction enzymes *Mre*I and *Age*I.

Plasmids were linearized using *Bsu36*I restriction enzyme prior to electroporation (using a standard transformation protocol described in Gasser et al. 2013. Future Microbiol. 8(2):191-208) into *P. pastoris.* Selection of positive transformants was performed on YPD plates (per liter: 10 g yeast extract, 20 g peptone, 20 g glucose, 20 g agar-agar) containing 50 μg/mL of Zeocin. Colony PCR was used to ensure the presence of the transformed plasmid. Therefore, genomic DNA was obtained by cooking and freezing of *P. pastoris* colonies for 5 minutes each and directly applied for PCR with the appropriate primers.

c) Construction of a *P. pastoris* strain secreting carboxylesterase from *Sphingopyxis macrogoltabida*

*P. pastoris* CBS7435 (Centraalbureau voor Schimmelcultures, NL, genome sequenced by Küberl et al. 2011) variant was used as host strain. Plasmid pPM2dZ30-PGAPα, a derivative pPuzzle_ZeoR vector backbone described in WO2008/128701A2, was used for the expression of a carboxylesterase from *Sphingopyxis macrogoltabida* (aa residues 48 to 540 of the amino acid sequence as deposited with GenBank: ACS27056 or shown in SEQ ID No. 29) under control of the *P. pastoris GAP* promoter, with the *S. cerevisiae* α-MF leader sequence for secretion and a Zeocin resistance marker cassette. The expression vector was linearized within the *GAP* promoter using the restriction enzyme *Bln*I for homologous integration into the native *GAP* promoter locus of the *P. pastoris* genome prior to electroporation (using a standard transformation protocol described in Gasser et al. 2013. Future Microbiol. 8(2):191-208) into *P. pastoris.* Selection of positive transformants was performed on YPD plates (per liter: 10 g yeast extract, 20 g peptone, 20 g glucose, 20 g agar-agar) containing 50 μg/mL of Zeocin. Colony PCR was used to ensure the presence of the transformed plasmid. Therefore, genomic DNA was obtained by cooking and freezing of *P. pastoris* colonies for 5 minutes each and directly applied for PCR with the appropriate primers.

**Example 2:** Screening for production of the recombinant proteins of interest

Example 2) a) Small scale cultivation of *Pichia pastoris* Fab production strains

2 mL YP-medium (10 g/L yeast extract, 20 g/L peptone) containing 10 g/L glycerol and 50 μg/mL Zeocin were inoculated with a single colony of *P. pastoris* strains and grown overnight at 25 °C. For clones expressing under control of pGAP, aliquots of these cultures (corresponding to a final $OD_{600}$ of 2.0) were transferred to 2 mL of Synthetic screening medium M2 (media composition is given below) containing a glucose feed tablet (Kuhner, Switzerland; CAT# SMFB63319) and incubated for 48 h at 25 °C at 170 rpm in 24 deep well plates. For clones expressing under control of pAOX, aliquots of these cultures (corresponding to a final $OD_{600}$ of 2.0) were transferred to 2 mL of Synthetic screening medium M2 (media composition is given below) supplemented with 20 g/L glucose and a glucose feed tablet (Kuhner, Switzerland; CAT# SMFB63319) and incubated for 25 h at 25 °C at 170 rpm in 24 deep well plates. The cultures were washed once by centrifugation, then the pellets were resuspended in Synthetic screening medium M2 and aliquots (corresponding to a final $OD_{600}$ of 4.0) were transferred into 2 mL of Synthetic screening medium M2 in fresh 24 deep well plates. Methanol (5 g/L) was added repeatedly every 12 h for 48 hours, before cells were harvested by centrifugation at 2500xg for 10 min at room temperature and prepared for analysis. Biomass was determined by measuring the cell weight of 1 mL cell suspension, while determination of the recombinant secreted protein in the supernatant is described in the following Examples 2c-2e.

*Synthetic screening medium M2 contained per litre:* 22.0 g Citric acid monohydrate 3.15 g $(NH_4)_2PO_4$, 0.49 g $MgSO_4{*}7H_2O$, 0.80 g KCl, 0.0268 g $CaCl_2{*}2H_2O$, 1.47 mL PTM1 trace metals, 4 mg Biotin; pH was set to 5 with KOH (solid)

Example 2) b) Small scale cultivation of *Pichia pastoris* CES production strains

<u>Pre-culture:</u>

10 mL YPD containing Zeocin 50 μg/mL (and G418 if appropriate) were inoculated with a single colony from a fresh selective YPD plate. The preculture was cultivated at 25°C and 180 rpm for 22 h.

After 22h of pre culture the cells were harvested by centrifugation (3000 g, 5 min) and washed once with 10 mL of sterile water. For inoculation of the main culture the cells were resuspended in 3 mL of sterile water. After resuspending of the cell pellet the $OD_{600}$ of the washed pre culture was measured.

<u>Main Culture:</u>

For the main culture 10 mL of freshly prepared M2-Medium (pH 5.0, containing 20g/L glucose as carbon source) was filled into a 100 mL shake flask. The main culture was inoculated with an OD of 0.1 with the washed pre culture. After inoculation the main culture was cultivated for 52 h at 25° and 180 rpm on a shaker. Cells were fed with 0.5 % Glucose (100 μL of a 50% glucose stock solution) after 22h, 28h, 42h and 48h.

After 52h samples were taken. Therefore 2 mL of culture were transferred into a 2 mL microcentrifuge tube in duplicates and centrifuged at 13,000 rpm for 5 min. The supernatant was transferred into a fresh microcentrifuge tube and used for determination of Carboxylesterase levels. Detection of the Carboxylesterase was done by Western blotting and ELISA as described in Example 2)c) and 2)e).

For the determination of the cell dry weight the pellets are washed once with water and centrifuged (13,000 rpm, 5 min). After the washing step the cell pellets are transferred into a preweight glass tube and dried for 2 days at 110°C. After drying the glass tubes are weighted again and the cell dry mass was calculated with following formula:

$$CDW\ [g/L] = [Glass\ tube(full) - Glass\ tube(empty)]*500$$

For each cultivation CDW determination was done in duplicates.

Example 2) c) SDS-PAGE & Western Blot analysis

For protein gel analysis the NuPAGE® Novex® Bis-Tris system was used, using 12 % Bis-Tris or 4-12 % Bis-Tris gels and MOPS running buffer (all from Invitrogen). After electrophoresis, the proteins were either visualized by silver staining or transferred to a nitrocellulose membrane for Western blot analysis. Therefore, the proteins were electroblotted onto a nitrocellulose membrane using the XCell II™ *Blot* Module for wet *(tank)* transfer (Invitrogen) according to the manufacturer's instructions. After blocking, the Western Blots were probed with the following antibodies: For Fab light chain: anti-human kappa light chains (bound and free) - alkaline phosphatase (AP) conjugated antibody, Sigma A3813 (1:5,000); For Fab heavy chain: Mouse Anti-Human IgG antibody (Ab7497, Abcam) diluted 1:1,000 and Anti-Mouse IgG (Fc specific)-Alkaline Phosphatase antibody produced in goat (A1418, Sigma) as secondary antibody diluted 1:5,000. For CES: rabbit anti-CES (rabbit 6287, produced in house) diluted 1:1000,and Anti-Rabbit IgG (whole molecule)-Peroxidase conjugated produced in goat as secondary antibody diluted 1:20000

Detection was performed with the colorimetric AP detection kit (BioRad) based on the NBT/BCIP system for AP-conjugates, or the chemoluminescent Super Signal West Chemiluminescent Substrate (Thermo Scientific) for HRP-conjugates.

Example 2) d) Quantification of Fab by ELISA

Quantification of intact Fab by ELISA was done using anti-human IgG antibody (ab7497, Abcam) as coating antibody and a goat anti-human IgG (Fab specific) - alkaline phosphatase conjugated antibody (Sigma A8542) as detection antibody. Human Fab/Kappa, IgG fragment (Bethyl P80-115) was used as standard with a starting concentration of 100 ng/mL, supernatant samples are diluted accordingly. Detection was done with pNPP (Sigma S0942). Coating-, Dilution- and Washing buffer were based on PBS (2 mM $KH_2PO_4$, 10 mM $Na_2HPO_4$.2 $H_2O$, 2.7 mM g KCl, 8 mM NaCl, pH 7.4) and completed with BSA (1% (w/v)) and/or Tween20 (0.1% (v/v)) accordingly.

Example 2) e) Quantification of CES by ELISA

Quantification of CES by ELISA was done using rabbit-Anti-CES antiserum 6287 (produced in house) as coating antibody, guinea pig-anti-CES antiserum 12206 (produced in house) and goat-anti-guinea pig-IgG-alkaline phosphatase conjugate (Sigma, A-5062) as detection antibody. Purified CES (produced in house) was used as standard with a starting concentration of 100 ng/mL, supernatant samples are diluted accordingly. Detection was done with pNPP (Sigma S0942). Coating-, Dilution- and Washing buffer were based on PBS (2 mM $KH_2PO_4$, 10 mM $Na_2HPO_4$.2 $H_2O$, 2.7 mM g KCl, 8 mM NaCl, pH 7.4) and completed with BSA (1% (w/v)) and/or Tween20 (0.1% (v/v)) accordingly.

**Example 3:** Screenings addition of Amino Acids

In our study, the impact of secreted proteins was evaluated, using the readily expressed enzyme Carboxylesterase ACS27056 von *Sphingopyxis macrogoltabida* (CES) or the more difficult antibody Fab fragment of HyHEL (HyHEL-Fab) as model proteins. While expression of both secretory proteins decreased the maximum specific growth rate, in particular in the HyHEL Fab secreting strain also significantly increased TCA cycle flux was observed (independent of carbon source or promoter system pAOX or pGAP - data not shown). Thus, we tested if addition of amino acids as described by Heyland et al. 2011 would also increase biomass yield and thus product titers for the HyHEL Fab secreting *P. pastoris.* No improvement of growth by addition of amino acids was observed. On the contrary, addition of amino acids to the Fab-secreting strains even decreased biomass concentration down to 60% of the non-treated cultures, depending on the combinations of amino acids added.

Amino Acid Stock preparation:

50 mL of the respective amino acid stock was prepared. The calculated amount of amino acids was weighted in and dissolved in 50 mL of water. For the preparation of the tyrosine 1 mL of 1 M NaOH was added. Stock concentration of the amino acids was always 10 g/L, except for serine with 40 g/L.

a) Screening pGAP-HyHEL-Fab#3

Pre-Culture:

50 mL of YPD containing 50 μg/mL Zeocin was inoculated with a single colony of a fresh selective YPD agar plate. The pre culture was cultivated at 25°C, 180 rpm for 22h. After 22 h of preculture the cells were harvested by centrifugation ( 3000 g, 5 min) and washed once with 10 mL of sterile water. For inoculation of the main culture the cells were resuspended in 10 mL of sterile water. After resuspending of the cell pellet the OD600 of the cell suspension was measured.

Main Culture:

For the preparation of the main culture media with the different amino acids compositions a 2 times concentrated M2 media without glucose was prepared. Amino acids stocks were added to reach the desired concentration. In a next step the pH of the media was set to a pH of 5.0 with 4 M KOH and diluted with water up to 50 mL end volume.

Following main culture media were prepared:

1. Screening Media
2. Screening Media + Proline (100 mg/L)
3. Screening Media + Glutamate (100 mg/L)
4. Screening Media + Methionine (100 mg/L)
5. Screening Media + Cysteine (100 mg/L)
6. Screening Media + Pro+Glu+Met (100 mg/L)
7. Screening Media + all amino acids except aspartate

All amino acids, except serine, valine and threonine, had a final concentration of 100 mg/L in the different media. The final concentration of serine was 400 mg/L, for valine 150 mg/L and for threonine 200 mg/L.

The screening was done in 24 deep well plates. The cultivation volume was 2 mL per well. Therefore 2 mL of the media containing the different amino acids mixtures were transferred into the wells. Each well was inoculated with an OD 2.0 using the washed pre culture cells. After inoculation a glucose feed bead (Kuhner Shaker) was added to each well. For cultivation on the shaker the 24 deep well plates are closed with a sterile oxygen permeable membrane. The cells were cultivated at 25°C and 320 rpm for 48 h.

After 48h samples were taken. Therefore 1 mL of culture from each well was transferred into a 2 mL microcentrifuge tube. The microcentrifuge tubes were centrifuged at 13.000 rpm for 5 min. The supernatant was transferred into a fresh microcentrifuge tube and used for determination of the HyHEL-Fab by ELISA. Cell dry weight (CDW) was determined as described in Example 2.

b) Screening pAOX-HyHEL-Fab#8

Pre-Culture:

50 mL of YPD containing 50 μg/mL Zeocin was inoculated with a single colony of a fresh selective YPD agar plate. The pre culture was cultivated at 25°C, 180 rpm for 22h. After 22h of preculture the cells are harvested by centrifugation (3000 g, 5 min) and washed once with 10 ml of sterile water. For inoculation of the main culture the cells were resuspended in 3 ml of sterile water. After resuspending of the cell pellet the OD600 of the cell suspension was measured.

Main Culture:

For preparation of the media 25 mL of 2 times concentrated M2 Medium was taken and amino acids stocks were added to reach the desired concentration. In a next step the pH of the media was set to a pH of 5.0 with 4 M KOH and diluted with water up to 50 mL end volume.

Following main culture media were prepared:

1. Screening Media
2. Screening Media + Proline (100 mg/L)
3. Screening Media + all amino acids except aspartate

All amino acids, except serine, valine and threonine, had a final concentration of 100 mg/L in the different media. The final concentration of serine was 400 mg/L, for valine 150 mg/L and for threonine 200 mg/L.

The screening was done in 24 deep well plates. Therefore 2 mL of the media containing the different amino acids mixtures were transferred into the wells. Each well was inoculated with an OD 4.0 using the washed pre culture cells. For methanol induction of the cells 0.5 % methanol (10 μl of a 100 % Methanol) was directly added after inoculation. For cultivation on the shaker the 24 deep well plates were closed with a sterile oxygen permeable membrane. The cells were cultivated at 25°C, 320 rpm for 48 h. After 6h, 22h and 30h of cultivations cells were fed with 1% methanol (20 μL of 100% methanol).

After 48h samples were taken. Therefore 1 mL of culture was transferred into a 2 mL microcentrifuge tube. The microcentrifuge tube was centrifuged at 13,000 rpm for 5 min. The supernatant was transferred into a new microcentrifuge tube and used for determination of Fab by ELISA as described in Example 2d.

The determination of the cell dry weight was performed as in Example 3a.

**Results**

<u>Screening of pAOX-SDZ-Fab#9 and pAOX-HyHEL-Fab#8 with addition of different amino acid combinations</u>

Addition of all amino acid to the cultivation media had a negative effect on Fab production. A drop in Fab level in the supernatant was observed. Therefore screenings with different amino acids mixtures were performed to identify the amino acid or amino acids mixture, which led to this lower Fab production. Screenings with pAOX-SDZ-Fab#9 (SDZ-Fab heavy chain is shown in SEQ ID No. 32 and SDZ-Fab light chain is shown in SEQ ID No. 33) showed that addition of serine was responsible for the lower Fab production.

To shorten the list of possible candidates causing this effect a screening with amino acid mixtures excluding specific groups of amino acids was performed.

The following media were used for this first screening round:

1. SCM w/o amino acids (control)
2. SCM+AA lacking ARG,PRO,ALA,LYS (SCM-ARG,PRO,ALA,LYS)
3. SCM+AA lacking HIS,MET,CYS (SCM-HIS,MET,CYS)
4. SCM+AA lacking ILE,LEU,VAL (SCM-ILE,LEU,VAL)
5. SCM+AA lacking GLU,GLN,ASN (SCM-GLU,GLN,ASN)
6. SCM+AA lacking SER,GLY,THR (SCM-SER,GLY,THR)
7. SCM+AA lacking PHE,TYR,TRP (SCM-PHE,TYR,TRP)
8. SCM + all AA (SCM+ALL AS)

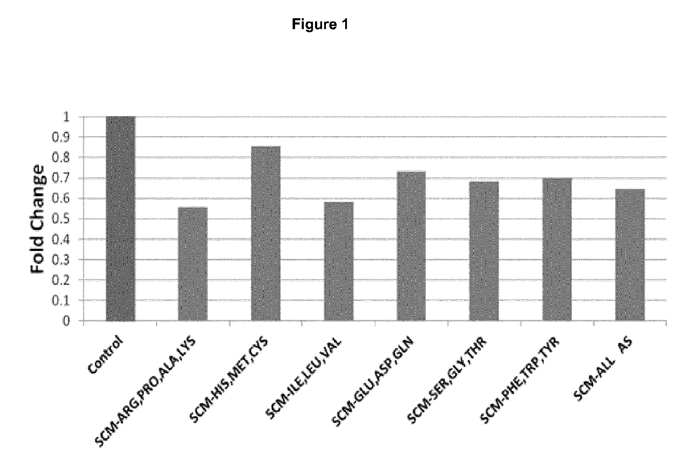

The results for the first screening round showed that for pAOX-HyHEL-Fab#8 **(Fig.1)** none of the prepared amino acids combination was identified as being responsible for the above mentioned effect (see Figure 1).

Also for another fab expressing strain (pAOX-SDZ-Fab#9), addition of amino acids to the growth medium had a negative effect on the Fab level (not shown). This shows that the approach suggested by Heyland et al. 2011 (Biotechnol Bioeng. 108(8):1942-53) is not generally applicable to reduce metabolic burden caused by recombinant protein production.

**Example 4:** Generation of strains overexpressing identified genes

For the investigation of positive effects on Fab secretion, the identified genes were overexpressed in different protein producing strains: CBS7435mut$^S$ pPM2d_pAOX HyHEL, CBS pPM2d_pGAP HyHEL and CBS7435 pPM2d_pGAP CES (strain generation see Example 1).

a) Amplification and cloning of the identified target genes into pPM2aK21 expression vectors

The target genes were amplified by PCR (Phusion Polymerase, New England Biolabs) from start to stop codon using the primers shown in Table 1. The sequences were cloned into the MCS of the pPM2aK21 expression vector with the two restriction enzymes *Sbf*I and *Sfi*I. pPMKaK21 is a derivative of pPM2d (described in Example 1a), consisting of an AOX1 terminator sequence (for integration into the native AOX1 terminator locus), an origin of replication for *E. coli* (pUC19), an antibiotic resistance cassette (kanMX conferring resistance to Kanamycin and G418) for selection in *E. coli* and yeast, an expression cassette for the gene of interest (GOI) consisting of a GAP promoter, a multiple cloning site (MCS) and the *S. cerevisiae CYC1* transcription terminator. Gene sequences were verified by Sanger sequencing.

Table 1

| Name | Forward primer (*Sbf*I attached) | Backward primer (*Sfi*I attached) |
|---|---|---|
| GCN4 | GATCCCTGCAGGATGTCTGCAAGT ACTTACAGTTTTG (SEQ ID NO. 34) | GATCGGCCGAGGCGGCCTCACTGATGT TTCACTAAACTCTTT (SEQ ID NO. 35) |
| ALT1 | GATCCCTGCAGGATGAGAGTTAATA AATTTATTCGTCC (SEQ ID NO. 36) | GATCGGCCGAGGCGGCCTCATTTATAC TTTTGCATGAACTTC (SEQ ID NO. 37) |
| SER1 | GATCCCTGCAGGATGGCAAAAACT TTGGAAAG (SEQ ID NO. 38) | GATCGGCCGAGGCGGCCTCAAGAATGA GCTTCAGCAA (SEQ ID NO. 39) |
| SER3 | GATCCCTGCAGGATGGCTTCTCCC CAAGATAT (SEQ ID NO. 40) | GATCGGCCGAGGCGGCCCTAGTATAGC AGACGGGTGAC (SEQ ID NO. 41) |
| ARO3 | GATCCCTGCAGGATGTTCATTCAAA ACGATCATGT (SEQ ID NO. 42) | GATCGGCCGAGGCGGCCCTAATTCTTG AGATTACGACGTTC (SEQ ID NO. 43) |
| ARO7 | GATCCCTGCAGGATGGAGTTCAAG AAACCCG (SEQ ID NO. 44) | GATCGGCCGAGGCGGCCTTACCACAAG CTGTTGGATAATA (SEQ ID NO. 45) |
| ILV5 | GATCCCTGCAGGATGTCCGTAAGA AATGCCAC (SEQ ID NO. 46) | GATCGGCCGAGGCGGCCTCAGTTGTTT TCTGGACGTAG (SEQ ID NO. 47) |
| LEU4 | GATCCCTGCAGGATGCCTATGCTA AAGGACCCCT (SEQ ID NO. 48) | GATCGGCCGAGGCGGCCTTATATTTTA CCATCTCTCCTTGCA (SEQ ID NO. 49) |
| MAE1 | GATCCCTGCAGGATGAGCTCTCTTT ACATGCCCT (SEQ ID NO. 50) | GATCGGCCGAGGCGGCCCTATGACTGA TGCGTATGGATTT (SEQ ID NO. 51) |
| PRO1 | GATCCCTGCAGGATGGGTGAAAAT TGTTACACTATC (SEQ ID NO. 52) | GATCGGCCGAGGCGGCCCTATTTATTT GGTTTGGATTCTCT (SEQ ID NO. 53) |
| PRO3 | GATCCCTGCAGGATGTCTTCAATTG GTAACGAATATA (SEQ ID NO. 54) | GATCGGCCGAGGCGGCCCTATTTCTTT CCTAAGCTAGTGGCA (SEQ ID NO. 55) |
| MET17 | GATCCCTGCAGGATGCCTTCTCACT TCGACAC (SEQ ID NO. 56) | GATCGGCCGAGGCGGCCTCACAACTTT GTGTTTGCCTC (SEQ ID NO. 57) |
| CYS4 | GATCCCTGCAGGATGTCAGACAAC AACCAACCT (SEQ ID NO. 58) | GATCGGCCGAGGCGGCCTTAATTAAAC TCTCCTTTGGAGG (SEQ ID NO. 59) |
| HOM2 | GATCCCTGCAGGATGAAGAAAGCA GGTGTTTTG (SEQ ID NO. 60) | GATCGGCCGAGGCGGCCTTAAATGAGA CCCTTTTTCAACA (SEQ ID NO. 61) |

b) Co-overexpression of identified genes in *P. pastoris* protein producing strains

The *P. pastoris* protein overproducing strains CBS7435mut$^S$ pAOX HyHEL-Fab, CBS pPM2d_pGAP HyHEL and CBS7435 pPM2d_pGAP CES were used as host strains for co-overexpression of the genes identified in Example 3 and cloned in Example 4a. Before transformation into the producing strains, the pPM2aK21 vectors containing the target genes identified in Example 3 are linearized in the AOX terminator sequence with the restriction enzyme *Asc*I. Positive transformants were selected on YPD agar plates containing G418.

**Example 5:** Screening for heterologous protein expression in amino acid metabolism engineered strains

In small-scale screenings, (5 clones for GCN4, 8 clones per construct for ALT1, MAE1, SER1/SER3, ARO3/ARO7, PRO1/PRO3 and MET17/CYS4/HOM2) transformants of each target construct were tested in comparison to the non-engineered parental host and ranked based on their impact on cell growth, product titer and product yield.

Small scale cultivation for Fab expressing strains was performed as described in Example 2) a). The amount of secreted Fab was analysed with ELISA as described in Example 2) d). Small scale cultivation for CES expressing strains was performed as described in Example 2) b). The amount of secreted CES was analysed with Western Blots as described in Example 2) c) and ELISA as described in Example 2) e).

In **Example 6,** all of these clones are then cultivated in bioreactors for verification of strain improvement in controlled production processes.

Results

Table 2a lists the genes whose overexpression was shown to increase CES secretion in *P. pastoris* small scale cultivation in comparison to the not engineered CES producing control strains.

| Helper protein(TA) | Microarray probe name | Gene identifier P. pastoris CBS7435 | Designation (in assumed analogy to S. cerevisiae) | Amino acid sequence (SEQ ID No.) | Nucleotide sequence (SEQ ID No.) |
|---|---|---|---|---|---|
| TA1 | pas_chr1-4_0339 | PP7435_Chr1-1124 | GCN4 | 1 | 15 |
| TA2 | pas_chr3_0482 | PP7435_Chr3-0727 | ALT1 | 2 | 16 |
| TA3 | pas_chr3_0566 | PP7435_Chr3-0640 | SER1 | 3 | 17 |
| TA4 | pas_chr2-1_0657 | PP7435_Chr2-0624 | SER3 | 4 | 18 |
| TA5 | pas_chr3_0936 | PP7435_Chr3-0235 | ARO3 | 5 | 19 |
| TA6 | pas_chr4_0050 | PP7435_Chr4-0965 | ARO7 | 6 | 20 |
| TA7 | pas_chr1-1_0432 | PP7435_Chr1-0750 | ILV5 | 7 | 21 |
| TA8 | pas_chr2-1_0415 | PP7435_Chr2-0884 | LEU4 | 8 | 22 |
| TA9 | pas_chr3_0181 | PP7435_Chr3-1050 | MAE1 | 9 | 23 |
| TA10 | pas_chr3_0294 | PP7435_Chr3-0925 | PRO1 | 10 | 24 |
| TA11 | pas_chr4_0398 | PP7435_Chr4-0587 | PRO3 | 11 | 25 |
| TA12 | pas_chr4_0330 | PP7435_Chr4-0665 | MET17 | 12 | 26 |
| TA13 | pas_chr2-2_0137 | PP7435_Chr2-0144 | CYS4 | 13 | 27 |
| TA14 | pas_chr3_0471 | PP7435_Chr3-0736 | HOM2 | 14 | 28 |

Table 2b lists the function and provides the enzyme name of each of the genes whose overexpression was shown to increase CES secretion in *P. pastoris* small scale cultivation in comparison to the not engineered CES producing control strains

| Helper protein(TA) | Protein function | Activity |
|---|---|---|
| TA1 | Transcription factor | bZIP transcriptional activator of amino acid biosynthetic genes |
| TA2 | Alanine transaminase | involved in alanine biosynthesis and catabolism |

(continued)

| Helper protein(TA) | Protein function | Activity |
|---|---|---|
| TA3 | Phosphoserine aminotransferase | catalyzes the formation of phosphoserine from 3-phosphohydroxypyruvate, required for serine and glycine biosynthesis |
| TA4 | D-3-phosphoglycerate dehydrogenase | catalyzes the first step in serine and glycine biosynthesis |
| TA5 | 3-deoxy-D-arabino-heptulosonate-7-phosphate (DAHP) synthase | catalyzes the first step in aromatic amino acid biosynthesis |
| TA6 | Chorismate mutase | catalyzes the conversion of chorismate to prephenate to initiate the tyrosine/phenylalanine-specific branch of aromatic amino acid biosynthesis |
| TA7 | Ketol-acid reductoisomerase | involved in branched-chain amino acid biosynthesis |
| TA8 | 2-isopropylmalate synthase | responsible for the first step in the leucine biosynthesis pathway |
| TA9 | Malic Enzyme | catalyzes the oxidative decarboxylation of malate to pyruvate |
| TA10 | Glutamate 5-kinase | catalyzes the first step in proline biosynthesis |
| TA11 | Pyrroline-5-carboxylate reductase | catalyzes the last step in proline biosynthesis |
| TA12 | O-acetylhomoserine aminocarboxypropyltransferase | required for Methionine and cysteine biosynthesis |
| TA13 | Cystathionine beta-synthase | catalyzes synthesis of cystathionine from serine and homocysteine |
| TA14 | Aspartate-semialdehyde dehydrogenase | catalyzes the second step in the common pathway for methionine and threonine biosynthesis |

The CES product titer was quantified by ELISA (Example 2e). Biomass was determined as wet cell weight or dry cell weight. Changes in product titers and yields are represented as fold change values relative to the respective control strain (Table 3). Fold change values show the improvement in titers and product yields in screening relative to the GAP-CES parental host which was grown and sampled in parallel for direct comparison.

Table 3

| | **First Screening round** | | **Second Screening round** | | | |
|---|---|---|---|---|---|---|
| | **Western blot** | | **Western blot** | | **ELISA** | |
| | | **FC** | | **FC** | **FC Titer** | **FC Yield** |
| **GCN4** | 5 clones tested | - | 5 clones tested | 1.70 | 1.92 | 2.16 |
| **ALT1** | 8 clones tested | 1.89 | 8 clones tested | 1.47 | 1.81 | 1.86 |
| **MAE1** | 8 clones tested | 1.63 | 8 clones tested | 1.70 | 1.12 | 1.38 |
| **ARO3/ARO7** | 8 clones tested | 1.20 | 8 clones tested | 1.36 | 1.48 | 1.54 |
| **SER1/SER3** | 8 clones tested | 1.43 | 8 clones tested | 1.45 | 1.80 | 1.93 |
| **ILV5/LEU4** | 8 clones tested | 2.00 | 8 clones tested | 1.83 | - | - |
| **'FC'** means "fold change" | | | | | | |

As shown, the tested constructs succeeded in increasing the yield (mg/biomass) of the model protein CES by at least 20 % (fold change >1.2) upon over-expression.

**Example 7:** Metabolomics analysis of intracellular free amino acid levels

The engineering of the amino acid metabolism aimed at the increase of intracellular amino acid pools to improve recombinant protein production. To verify if the aim of engineering is achieved the intracellular amino acid levels for samples from screenings done in Example 5 were analysed. Therefore cells were quenched in cold methanol (40 % v/v, -27°C) and extracted with boiling ethanol according to Neubauer et al. (2012. J Sep Sci. 35(22):3091-105. doi: 10.1002/jssc.201200447.). The analysis of intracellular amino acid levels in the cell extracts was done with orthogonal reversed phase (RP LC) and hydrophilic interaction chromatography (HILIC) in combination with tandem mass spectrometry (Klavins et al. 2013. Anal Bioanal Chem. 405(15):5159-69. doi: 10.1007/s00216-013-6964-4.; Guerrasio et al. 2014. Anal Bioanal Chem. 406(3):915-22. doi: 10.1007/s00216-013-7456-2.).

For the comparison of intracellular amino acid levels of engineered strains with the non-engineered control (EVC) log2FC and p-values were calculated.

Criteria for differential abundance:

Metabolites indicated in red have significantly increased levels in the engineered strain (Criteria: log2FC >0.39 and p-value < 0.05).

Metabolites indicated in blue have significantly decreased levels in the engineered strain (Criteria: log2FC < -0.39 and p-value < 0.05).

The colour intensity corresponds to the degree of regulation.

For some samples the intracellular amino acid levels were below the limit of quantification for either the non-engineered control strain or the amino acid engineered strain. Therefore the calculation of log2FC and p-value was not possible. Nevertheless, the direction of regulation was indicated (up or down) by the above mentioned colour code in the following tables (fields marked light red or light blue, without values for log2FC and p-values). #NV in the column log2FC indicates that intracellular amino acids were below the limit of quantification in both strains.

Results

Strains engineered for increased amino acid supply showed higher CES production in Example 5. These strains and the respective empty vector controls (EVC) were analysed for their abundance of free intracellular amino acids. Per construct one to two clones were selected. Changes in intracellular amino acid levels are represented as log2 fold change values relative to the respective EVC (Table 4). In order to assess the significance of the difference to the control strain p-values were calculated using Student's t-test (Table 4, PV). All strains with improved CES production (GCN4, SER1/SER3, ARO3/AR07, ALT1, ILV5/LEU4#2) showed significantly higher intracellular amino acids levels (Table 4 a-e).

The overexpression of the in genes listed in Table 2 succeeded in increasing intracellular amino acid pools. The improvement in CES production is connected to the increase in intracellular amino acid levels.

Thereby the metabolomics data affirm that metabolic engineering of the amino acid biosynthetic pathways indeed increases intracellular amino acid pools, and that this correlates with the positive effect on recombinant protein production. This confirms that the intended engineering strategy (enhancement of amino acid biosynthesis and thus increased levels of free amino acids available for recombinant protein synthesis) could be achieved.

**Table 4 Calculated Log2FC and p-values for amino acid engineered strains compared to the respective control (*fcy fold change in CES yield observed in Example 5)**

a)

| | GCN4#2 /EVC | | GCN4#5 /EVC | |
|---|---|---|---|---|
| | Screening: 2.95 fcy* | | Screening: 2.0 fcy* | |
| | LOG2FC | PV | LOG2FC | PV |
| Ala | | #NV | | #NV |
| Arg | | #NV | | #NV |
| Asn | | #NV | | #NV |
| Asp | | #NV | | #NV |
| Glu | 2.68 | 0.00 | 2.96 | 0.00 |
| Gln | | #NV | | #NV |
| Gly | #NV | #NV | #NV | #NV |
| His | | #NV | | #NV |
| Ile | 3.07 | 0.00 | 3.28 | 0.00 |
| Leu | 4.26 | 0.00 | 3.15 | 0.00 |
| Lys | 2.37 | 0.00 | 2.32 | 0.00 |
| Met | #NV | #NV | #NV | #NV |
| Phe | 4.50 | 0.00 | 3.25 | 0.00 |
| Pro | | #NV | | #NV |
| Ser | 2.16 | 0.00 | 1.81 | 0.00 |
| Thr | 2.22 | 0.00 | 1.44 | 0.01 |
| Trp | 3.95 | 0.00 | 3.53 | 0.00 |
| Tyr | 3.55 | 0.00 | 3.62 | 0.00 |
| Val | 3.05 | 0.00 | 2.58 | 0.00 |

b)

| | ILV5/LEU4#2 /EVC | |
|---|---|---|
| | Screening: | |
| | LOG2FC | PV |
| Ala | | #NV |
| Arg | 1.51 | 0.00 |
| Asn | 1.88 | 0.00 |
| Asp | | #NV |
| Glu | 1.37 | 0.00 |
| Gln | #NV | #NV |
| Gly | #NV | #NV |
| His | 0.21 | 0.01 |
| Ile | 1.63 | 0.00 |
| Leu | 2.16 | 0.00 |
| Lys | | #NV |
| Met | #NV | #NV |
| Phe | 1.24 | 0.00 |
| Pro | | #NV |
| Ser | 0.50 | 0.02 |
| Thr | 1.15 | 0.00 |
| Trp | 1.92 | 0.00 |
| Tyr | 1.82 | 0.00 |
| Val | 1.01 | 0.00 |

c)

| | ALT1#6 /EVC | |
|---|---|---|
| | Screening: 5.13 fcy* | |
| | LOG2FC | PV |
| Ala | | #NV |
| Arg | 1.82 | 0.00 |
| Asn | 1.77 | 0.00 |
| Asp | | #NV |
| Glu | 1.47 | 0.00 |
| Gln | 2.35 | 0.00 |
| Gly | #NV | #NV |
| His | | #NV |
| Ile | 2.12 | 0.00 |
| Leu | 2.31 | 0.00 |
| Lys | 0.44 | 0.00 |
| Met | | #NV |
| Phe | 3.45 | 0.00 |
| Pro | 3.40 | 0.00 |
| Ser | | #NV |
| Thr | 1.15 | 0.00 |
| Trp | 2.62 | 0.00 |
| Tyr | 1.81 | 0.00 |
| Val | 2.48 | 0.00 |

d)

| | ARO3/ARO7#8 / EVC | |
|---|---|---|
| | Screening: 5.39 fcy* | |
| | LOG2FC | PV |
| Ala | #NV | #NV |
| Arg | | #NV |
| Asn | #NV | #NV |
| Asp | #NV | #NV |
| Glu | 0.34 | 0.28 |
| Gln | #NV | #NV |
| Gly | #NV | #NV |
| His | #NV | #NV |
| Ile | 2.08 | 0.00 |
| Leu | 1.58 | 0.00 |
| Lys | | #NV |
| Met | | #NV |
| Phe | 3.23 | 0.00 |
| Pro | 0.81 | 0.00 |
| Ser | #NV | #NV |
| Thr | 1.48 | 0.01 |
| Trp | 2.43 | 0.00 |
| Tyr | 3.08 | 0.00 |
| Val | 1.72 | 0.00 |

e)

| | SER1/SER3#5 / EVC | |
|---|---|---|
| | Screening: 3.24 fcy* | |
| | LOG2FC | PV |
| Ala | | #NV |
| Arg | #NV | #NV |
| Asn | #NV | #NV |
| Asp | #NV | #NV |
| Glu | 1.48 | 0.00 |
| Gln | #NV | #NV |
| Gly | #NV | #NV |
| His | #NV | #NV |
| Ile | 1.87 | 0.00 |
| Leu | 1.82 | 0.00 |
| Lys | | #NV |
| Met | 1.54 | 0.00 |
| Phe | 2.22 | 0.00 |
| Pro | 1.93 | 0.00 |
| Ser | #NV | #NV |
| Thr | 1.29 | 0.00 |
| Trp | 2.15 | 0.00 |
| Tyr | 1.20 | 0.00 |
| Val | 2.18 | 0.00 |

## Claims

1. A method of increasing the yield of a protein of interest in a fungal or yeast host cell engineered to comprise a heterologous polynucleotide encoding said protein of interest, comprising overexpressing a polynucleotide encoding a helper protein or functional homologue thereof which is involved in one or more proteinogenic amino acid biosynthesis pathways, thereby increasing the yield of said protein of interest,
said helper protein having an amino acid sequence as shown in any one of SEQ ID NOs: 1-14, and
said functional homologue having at least 30% sequence identity to an amino acid sequence as shown in any one of SEQ ID NOs: 1-14,
wherein said helper protein or functional homologue thereof is capable of increasing the yield of the model protein CES shown in SEQ ID No. 29 in said host cell by at least 20% compared to the host cell prior to engineering.

2. The method of claim 1, wherein said one or more proteinogenic amino acid biosynthesis pathways is one or more amino acid biosynthesis pathways selected from an aliphatic amino acid biosynthesis pathway; an aromatic amino acid biosynthesis pathway, a cyclic amino acid biosynthesis pathway, a hydroxyl amino acid biosynthesis pathway, a branched chain amino acid biosynthesis pathway or a sulphur-containing amino acid biosynthesis pathway.

3. The method of claim 1 or 2 comprising:

    - engineering the host cell to overexpress said polynucleotide,
    - recombining in said host cell a heterologous polynucleotide encoding the protein of interest,
    - culturing said host cell under suitable conditions to overexpress the helper protein or functional homologue thereof and the protein of interest, and optionally
    - isolating the protein of interest from the cell culture.

4. The method of claim 1 or 2 comprising:

    - providing the host cell engineered to overexpress said polynucleotide, wherein said host cell comprises a heterologous polynucleotide encoding a protein of interest;
    - culturing the host cell under suitable conditions to overexpress the helper protein or functional homologue thereof and express the protein of interest, and optionally
    - isolating the protein of interest from the cell culture.

5. The method of any one of claims 1-4, wherein overexpression is achieved by having 1, 2, 3, 4 or more copies of said polynucleotide encoding the helper protein or functional homologue thereof in said host cell.

6. The method of any one of claims 1-5, wherein said polynucleotide is integrated in the genome of said host cell.

7. The method of any one of claims 1-6, wherein the overexpression is achieved (i) by using a recombinant promoter which drives expression of said polynucleotide, (ii) by modifying a regulatory sequence operably linked to the polynucleotide encoding the helper protein or functional homolog thereof, or (iii) by using an enhancer to enhance the promoter activity.

8. The method of any one of claims 1-7, wherein the host cell is *Pichia pastoris, Hansenula polymorpha, Trichoderma reesei, Saccharomyces cerevisiae, Kluyveromyces lactis, Yarrowia lipolytica, Pichia methanolica, Candida boidinii, Komagataella sp., Aspergillus* sp., or *Schizosaccharomyces pombe.*

9. The method of any one of claims 1-8, wherein 2, 3, 4, 5, 6, 7, 8 or more of helper proteins selected from any one of SEQ ID NOs: 1-14 or functional homologues thereof are overexpressed.

10. The method of claim 9, wherein said host cell overexpresses the following helper proteins

    (a) helper proteins having the amino acid sequence as shown in SEQ ID NOs: 3 and 4 or a functional homologue thereof, wherein the functional homologue has at least 30% sequence identity to an amino acid sequence as shown in SEQ ID NOs: 3 and 4, respectively;
    (b) helper proteins having the amino acid sequence as shown in SEQ ID NOs: 5 and 6 or a functional homologue thereof, wherein the functional homologue has at least 30% sequence identity to an amino acid sequence as shown in SEQ ID NOs: 5 and 6, respectively;

(c) helper proteins having the amino acid sequence as shown in SEQ ID NOs: 7 and 8 or a functional homologue thereof, wherein the functional homologue has at least 30% sequence identity to an amino acid sequence as shown in SEQ ID NOs: 7 and 8, respectively;
(d) helper proteins having the amino acid sequence as shown in SEQ ID NOs: 10 and 11 or a functional homologue thereof, wherein the functional homologue has at least 30% sequence identity to an amino acid sequence as shown in SEQ ID NOs: 10 and 11, respectively; or
(e) helper proteins having the amino acid sequence as shown in SEQ ID NOs: 12, 13 and 14 or a functional homologue thereof, wherein the functional homologue has at least 30% sequence identity to an amino acid sequence as shown in SEQ ID NOs: 12, 13 and 14, respectively.

**11.** The method of any one of claims 1-10, wherein the protein of interest is an enzyme, a therapeutic protein, an antibody or antibody fragment, a food additive or feed additive.

**12.** A recombinant fungal or yeast host cell for manufacturing a protein of interest, wherein the host cell is engineered to comprise a heterologous polynucleotide encoding said protein of interest and to overexpress a polynucleotide encoding a helper protein or functional homologue thereof which is involved in one or more proteinogenic amino acid biosynthesis pathways,
said helper protein having an amino acid sequence as shown in any one of SEQ ID NOs: 1-14, and
said functional homologue having at least 30% sequence identity to an amino acid sequence as shown in any one of SEQ ID NOs: 1-14,
wherein said helper protein or functional homologue thereof is capable of increasing the yield of the model protein CES shown in SEQ ID No. 29 in said host cell by at least 20% compared to the host cell prior to engineering.

**13.** The host cell of claim 12, wherein the following helper proteins are overexpressed

(a) helper proteins having the amino acid sequence as shown in SEQ ID NOs: 3 and 4 or a functional homologue thereof, wherein the functional homologue has at least 30% sequence identity to an amino acid sequence as shown in SEQ ID NOs: 3 and 4, respectively;
(b) helper proteins having the amino acid sequence as shown in SEQ ID NOs: 5 and 6 or a functional homologue thereof, wherein the functional homologue has at least 30% sequence identity to an amino acid sequence as shown in SEQ ID NOs: 5 and 6, respectively;
(c) helper proteins having the amino acid sequence as shown in SEQ ID NOs: 7 and 8 or a functional homologue thereof, wherein the functional homologue has at least 30% sequence identity to an amino acid sequence as shown in SEQ ID NOs: 7 and 8, respectively;
(d) helper proteins having the amino acid sequence as shown in SEQ ID NOs: 10 and 11 or a functional homologue thereof, wherein the functional homologue has at least 30% sequence identity to an amino acid sequence as shown in SEQ ID NOs: 10 and 11, respectively; or
(e) helper proteins having the amino acid sequence as shown in SEQ ID NOs: 12, 13 and 14 or a functional homologue thereof, wherein the functional homologue has at least 30% sequence identity to an amino acid sequence as shown in SEQ ID NOs: 12, 13 and 14, respectively.

**14.** The host cell of claim 12 or 13, wherein the protein of interest is an enzyme, a therapeutic protein, an antibody or antibody fragment, a food additive or feed additive.

**15.** Use of the host cell of any one of claims 12 to 14 for manufacturing a protein of interest.

Figure 1

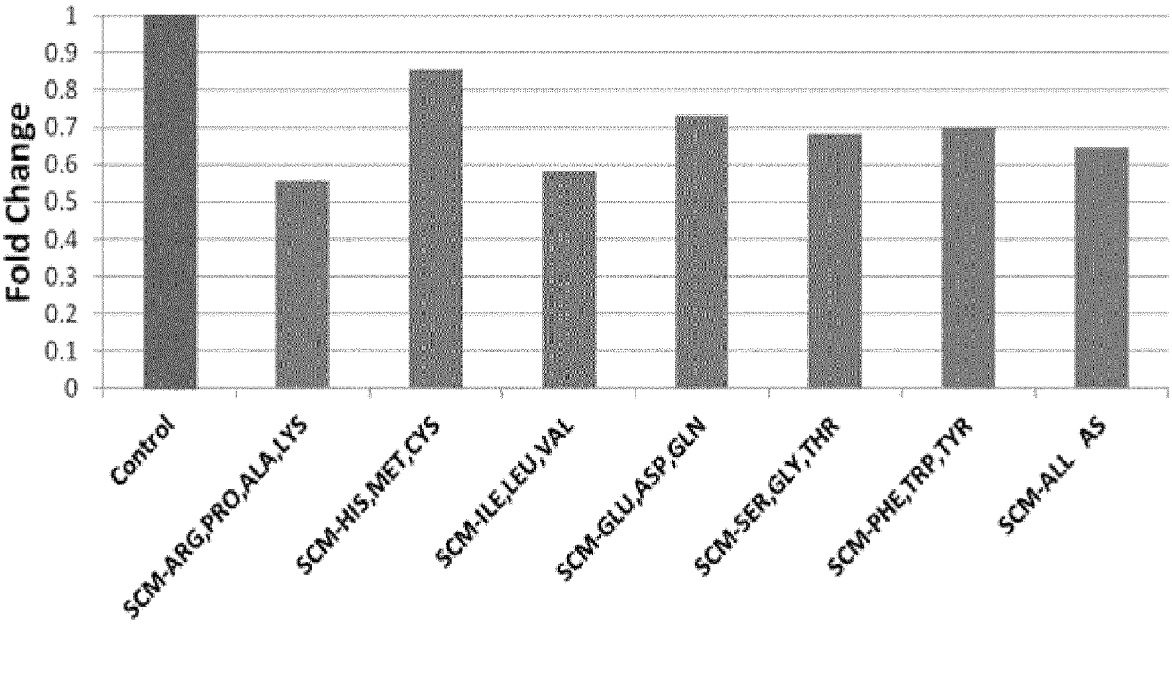
Fold Change
1
0.9
0.8
0.7
0.6
0.5
0.4
0.3
0.2
0.1
0
Control
SCM-ARG,PRO,ALA,LYS
SCM-HIS,MET,CYS
SCM-ILE,LEU,VAL
SCM-GLU,ASP,GLN
SCM-SER,GLY,THR
SCM-PHE,TRP,TYR
SCM-ALL AS

## Figure 2

TA1 - GCN4 (SEQ ID No. 1)

MSASTYSFDQAMDFDIVQSVTSTQDHIPMVLGESVLRSFVGNDANKAPAIKQEYEALPLNAQIVNPELTP
SVGTISPLEIHTSVLDSVLSTDFTDADNSPMFESPESEDPNNWVSLFADETTLATTPAVSRAPAASASPV
VPSLKTTSGDEQQLTVKQFVEYPSAKDKLSPKSVEKKISFKKDHLGVVGYTRRQRSSPLAPIVVKDDDPV
SMKRARNTEAARRSRAKKMKRMSQLEDKVEELLICKSELEAEVERLKSLVKHQ

TA2 - ALT1 (SEQ ID No. 2)

MRVNKFIRPCRHMSSTTVVSLGLKIKPDFQPAPKLTVTDLNPHTVNAKYAVRGKIPTKAELLRNQLNDVD
HKLPFTKIISANIGNPQQLDQKPLTFYRQILALLQYPPLLDDPRVASIFPKDIIERARSLLKQIGSVGAY
SQSQGVPSIRQSIADFISRRDGHPCARKELIYLTTGASTAVTYLLTLLGQNEKTGFLIPIPQYPLYTATL
TLNNRTALPYYLNEEDNWSIECDEIESIILDSKAKGIDPRCLIVINPGNPTGAILSYEAIEDILNVCAKY
GLVVIADEVYQENIFDGKFISVRKVLLDLLQGPKADLYKNIQLASLHSTSKGISGECGQRGGYMELIGFD
ESVLEQITKLSSISLCPVVTGQALVELMINPPKEGDESYKLYYDETRAIHDSLAERATKLYDAFNSMEGV
VCRKPQGAMYCFPKITIPEKAIKTAKELDFEPDEFYCNELLETTGICAVPGSGFGQKPGTYHIRTTFLAP
GSEWINDWVVFHKKFMQKYK

TA3 - SER1 (SEQ ID No. 3)

MAKTLEREEPHYFGAGPALLPTSVLQEAAYDLVNYQGVGIGIGEISHRSKQASEVIDNTKANLVSLLNIP
DTHEVFFLQGGGTTGFSSIASNLTASFVKKTGRKGRAAYAITGTWSKKSVEEAQRLGLDVDIVLDSKKVD
GKFGSIPPVDKWSIPTDLSNTSYVYYCDNETVNGVEFKEFPFESFPGVEVVADMSSNILSKRLDVSKYGL
IMAGAQKNIGLAGITIYIIKKSLLEQADDATLKALEVPLSPIAVHYPTVVKNNSAYNTIPIFAVHVVNLV
LRLLVKNGGVDAQQKINEEKASILYGALEKFPQVYELPASTDARSHMNVVFTLNGDLDAEFLAQAAERKL
NGLKGHRSVGGMRASIYNAVSLHSVQELTKFIIEFAEAHS

TA4 - SER3 (SEQ ID No. 4)

MASPQDIKQLSSGFNAVGLSTSPGVVSASPNDSYLSQVRQRSFSAAKHAKPLKPFSTGDIKILLLENVNE
TAREIFAEQGYQVEFHKSSLPEDELIEKIKDVHAIGIRSKTKLTEKVLKHAKNLIIIGCFCIGTNQVDLE
YAAKSGVAVFNSPFSNSRSVAELVIAEIITLARQLGDRSLEMHTGTWNKVSNKCWEIRGKTLGIVGYGHI
GSQLSVLAEAFGMNVIYYDVLMIMALGSAKQVRTLNDLLAQADFVTLHVPENPETKNLISSPQLAAMKDG
AYLINASRGTVVDIPALVEAMKLGKLAGAALDVYPQEPGKNGPNFTNELNSWATELTSLRNIILTPHIGG
STEEAQSAIGIEVGTALTKYINEGTSTGAVNFPEVALRALDLDQENSVRVLYIHQNVPGVLKTVNNILGN
HNIEKQFSDSRGDIAYLMADISDVDASDIKSLYEQLEHTPYKIVTRLLY

TA5 - ARO3 (SEQ ID No. 5)

MFIQNDHVGDRSRLEDWRIRGYDPLTPPDLLQHEYPLTPKAEENILKGRNEAVDILKGKDDRLLVIVGPC
SIHDPKAALDYCERLAKFNETIKGELHIIMRAYLEKPRTTVGWKGLINDPDIDGSFQINKGLRISRELFV
KLTEQIPIAGEMLDTISPQFLSDLFSLGAIGARTTESQLHRELASGLSFPVGFKNGTDGGLTVAIDAMRA
ASHPHHFLSVTKPGVVAIVGTEGNEDTFVILRGGKNGTNYDEASVDAAHAQLEKTGILGSGPAIMVDCSH
GNSNKDHRNQPKVAQVVADQIRKGSRKICGLMIESNIVEGRQDVPKEGKSHLRYGCSITDACISWEDTEK
VLRVLADSVIERRNLKN

## Figure 2 cont'd

### TA6 - ARO7 (SEQ ID No. 6)

MEFKKPATVLNLANIRQALVRMEDTVVFSFIERSQFYESPSVYAPNKYKIPNFSGSFLDWLLLQNEKTHS
LVRRYESPDETPFFPDQLEESFLPSLNYPPILADYADEVNVNDEIRTVYIEKIVPQIAAKKGDQDENIGS
TACADVDCLQALSRRVHFGKFVAEAKFQNEMARYTNLIRNKDIKGIEEAITDSAVEAKILERLIAKGHAY
GTDPTLRYSQNPQSKVQPEAIAKIYKEVVIPLTKKVEVEYLLRRLGDDDEAEAQDPQRKTQLMNRLLSNS
LW

### TA7 - ILV5 (SEQ ID No. 7)

MSVRNATFRLSRAAIAKRTLVRIASQASLRPTTLNSATGVARSQLTFSRGVKTINFGGVEEVVHERSDWP
REKLLEYFKNDTLALIGYGSQGYGQGLNLRDNGLNVIIGVRKNGASWKAAIEDGWVPGENLFDVTEAIKK
GSIIMNLLSDAAQSETWSTVKPLLTEGKTLYFSHGFSPVFSNLTHVEPPSNIDVILAAPKGSGRTVRSLF
KEGRGINSSYAVWNDVTGKAEEKAIALAVAVGSGYVYQTTFEREVNSDLYGERGCLMGGIHGMFLAQYEV
LRENGHTPSEAFNETVEEATQSLYPLVGKYGMDYMYDACSTTARRGALDWYPIFKDALKPVFEDLYNSVK
TGKETQRSLDFNSQPDYREKLEKELEIIRNMEIWKVGKEVRKLRPENN

### TA8 - LEU4 (SEQ ID No. 8)

MPMLKDPSKKYVAFKPLNLPDRQWPSKTLQAPPRWLSTDLRDGNQSLPDPMSVAEKKEYFNKLVQIGFKE
IEVAFPSASQTDFDFTRYAVENAPADVAIQVLSPCRPELIKRTVESLKGAKKAIVHIYLATSDCFRNIIF
GLSQEESLEMAVNCTKLVRELTKDAPEMQDTVWGFEFSPETFSDTDPDYALRVCEAVKAAWGPSEENPII
FNLPATVEMSTPNVYADQIEYFSRNISERKTVAISLHPHNDRGCAVAAAELGQMAGADRIEGCLFGNGER
TGNVDLVTLALNLYTQGVYPHLDFSDITSVIDIVERCNKIPVHPRAPYGGQLVVCAFSGSHQDAIKKGFK
KQEERQAKDPNARWQLPYLPLDPQDIGRTYEAIIRVNSQSGKGGASWVILRNLELDLPRALQVSFSKIVQ
NESEVKGRELYNKEITNLFKNSYFVDDDDTQHDLYFQLVDYSITNTSKSARLINVELQVGKDTVSLKGIG
NGPISSFASAISSYLKKDVLVLNYHEHSLGKDSNSKAATYLHCAIGDDCKVWGVGIHEDVSQASFLSFIS
LLNSARRDGKI

### TA9 - MAE1 (SEQ ID No. 9)

MSSLYMPSKSMKRLTVSCRRFISSAQPKIVTHKILTPVGTEAAAKVDVPKTTRLSVEGPIECHLQGFELL
NSPLFNKGSAFTPEERAAFGLEGLLPPVANDLNEQAERAYKQLSFLKTPLAKNDFCTSMRQQNKVLFYEL
VRRHIRELLPIIYTPTEGDAIAAYSHRFRKPEGCFLDVTDPESIEDRLSRFGESKDVDYIVVSDGEGILG
IGDQGIGGVRIAISKLALMTLCGGIHPGRVIPVVVDAGTNNKALLDDELYMGSRFPRVRGEEYYSFFDKF
IEVVKRRFPSAVLHFEDFGVTNAKPLLDKYRSVLPCFNDDIQGTGAVVMASMAAALKLTNRDLLDSTIVI
YGAGSAGLGIAFQIVNHMVNHGATKEEAYSRIHLLDRHGLILTNMSDVSNKDQMLFADDPSSWDGINTKS
LVDVIEKLKPTTLIGCSTQAGAFTEEVIKTMYKYNPRPIVFPLSNPTRLHEAVPVDVMRWTDDNALIATG
SPFKPVRGHVISENNNCFSFPGIGLGAVLSRATTISDTMISAAVDELASGSPAFKDPKAGLLAPVEVINE
TSAKVAAALILQSIKEGTARVEEELSPNGDKVSVPRDFEGCVAWVKSQMWRPEYRPMVKVERVPEIHTHQ
S

## Figure 2 cont'd

TA10 - PRO1 (SEQ ID No. 10)

MGENCYTIVVKLGTSSVVDEETREPRIATMSRIVETLVKLRRKGHRIVIVSSGAIAVGMKRVNMKRKPKK
LQQVQALAAIGQGRLIGLWDDLFRQLNQPIAQILLTRTDLVDYTQFKNAENTLEQLIKMGIIPIVNENDT
LSIQEIKFGDNDTLSAITAGMCHADYLFLVTDVDCLYTDNPRTNPDAEPIVLVRNMRNLNVNTESGGSAV
GTGGMTTKLIAADLGVSAGVTTIICKSEHPEQILDIVEYSIRADRVENEAKYLVINEEETVEQFQEINRS
ELRELNKLDIPLHTRFVGHSFNAVNNKEFWLLHGLKANGAIIIDPGCYKAITRKNKAGILPAGIISVEGN
FHEYECVDVKVGLRDPDDPHSLDPNEELYVVGRARCNYPSNQINKIKGLQSSQIEQVLGYADGEYVVHRD
NLAFPVFADPELLDVVESTLSEQERESKPNK

TA11 - PRO3 (SEQ ID No. 11)

MSSIGNEYTITILGCGVMGTAVLSAILNSEFSPYPSRIICCTGSQKSAEKLASTYSKIETSYGSEQNIRA
VKESDIIILGCKPFYCEQIMEQVKEGLHDQLLISLLAGWTLTQLERYSKYVARVMTNTPAKFGCGMACVS
LSSHAEQYEDLVLKLVGPVGKAIRLPEKNMDAATALVGSGPAFCLLMMESLIDGAVQKGLPFAIAKDAAA
KVMEGTARMVLETGEHPAALKSAVCTPGGTTIGGLMAMEDRGVRSGIARGVEEAANIATSLGKK

TA12 - MET17 (SEQ ID No. 12)

MPSHFDTLQLHAGQTAEAPHNARAVPIYATSSYVFRDSEHGAKLFGLEEPGYIYSRLMNPTQNVFEERIA
ALEGGAAALAVGSGQAAQFLAIAGLAHTGDNVISTSFLYGGTYNQFKVAFKRLGIESRFVHGDDPAEFER
LIDDKTKAIYVESIGNPKYNIPDFEALAELAHKHGIPLVVDNTFGAGGYYVRPIELGADIVTHSTTKWIN
GHGNTIGGVVVDSGKFPWKDYPEKYPQFSKPSEGYHGLILNDAFGPAAFIGHLRTELLRDLGPASSPFGN
FLNIIGLETLSLRAERHAENALKLAKYLETSPYVSWVSYPGLESHDYHEAAKKYLKNGFGAVLSFGVKDH
GKPALTPFEEAGPKVVDSLKVFSNLANVGDSKSLIIAPYYTTHQQLSHEEKLASGVTKDSIRVSVGTEFI
DDLIADLEQAFALVYEEANTKL

TA13 - CYS4 (SEQ ID No. 13)

MSDNNQPLPPVSNSILEHIGKTPLVKLNRIPKILNLKPQVYAKVELFNSGGSIKDRIALRMVEQAEKEGR
IKPGYTLIEPTSGNTGIGLALVGAVKGYRTIITLPEKMSNEKVAVLKALGAEIIRTPTEAAWDSPESHIG
VARRLEKEIPNAVILDQYGNVNNPDAHFYTTGAEIWDQTNGKVTHVVAGAGTGGTITGIAKFLKSKNPNI
QIIGADPHGSILALPESLNTSNEQYKVEGIGYDFIPEVLDREIVDTWYKTDDKDSFKLARQLISEEGILV
GGSSGSALSAAVKIINDAKLDESAVVVVVCPDSIRSYLTKFADDDWMKLNGFTEDEPAPLKRKSSFSKDT
LSSYTIKDLALKPVVSVTIDDSIESTINILQTKGFDQLPVLKNDKLAGIVTLSQLLRLLSNKKIHLTSKV
ESAYFDFSKLENFEKSYNINKESTNKRREYQKITTSTTLAKLNKFFETSSAAIVENDKGQPIHIVSKVDL
LSFLASKGEFN

TA14 - HOM2 (SEQ ID No. 14)

MKKAGVLGATGSVGQRFILLLSDHPEFELSVLGASSRSAGKRYEDAVVWKQTDLLPESARNIIVQECKAE
AFKDCDIIFSGLDADYAGEIEKEFVSSGLAVVSNAKNYRREADVPLIVPIVNPEHLEIVEQKVKSGQKGF
QICISNCSTAGLVAPLKPLVEKFGPIDLLTTTTLQAISGAGFSPGVPGIDILDNIVPFISGEEEKMEWET
RKILGGLSEDKMECRLVSEDAMKVSAQCNRVPVIDGHTECISLRFERRPPPSVEAVKQCLRDYVCEASKL
GCPSAPKNTIHVLEQNDRPQPRLDRVRDNGFGVSVGRIREDPVLDFKMVVLSHNTIIGAAGSGVLIGEIL
LKKGLI

## Figure 3

TA1 - GCN4 (SEQ ID No. 15)

ATGTCTGCAA GTACTTACAG TTTTGACCAA GCAATGGACT TTGACATCGT TCAGTCCGTG
ACGTCGACCC AGGACCATAT CCCCATGGTT CTTGGCGAGT CAGTGCTTCG TTCTTTTGTT
GGAAATGATG CCAACAAGGC TCCTGCTATC AAGCAGGAAT ATGAGGCCTT GCCGCTAAAC
GCTCAAATCG TCAATCCTGA GCTGACACCA TCCGTCGGAA CTATTTCTCC TTTGGAGATC
CATACTTCCG TTTTGGATTC TGTATTGTCG ACAGACTTTA CTGATGCTGA CAACTCCCCC
ATGTTTGAAT CTCCAGAGTC TGAAGATCCA AACAACTGGG TTTCGTTGTT TGCAGATGAA
ACTACTCTTG CTACCACTCC AGCCGTTTCT CGTGCACCAG CAGCCTCTGC CTCTCCAGTA
GTCCCTTCGT TGAAGACCAC CTCTGGAGAC GAGCAACAGC TGACTGTTAA ACAATTCGTC
GAGTATCCTT CCGCTAAGGA TAAGCTTTCT CCCAAGTCAG TGGAGAAAAA GATTTCTTTC
AAGAAGGACC ACTTAGGTGT TGTTGGCTAC ACTAGAAGAC AGCGTTCCTC TCCCTTGGCT
CCAATCGTGG TCAAGGATGA CGATCCTGTT TCTATGAAGC GTGCCCGTAA CACGGAAGCC
GCTCGTCGTT CCAGGGCTAA GAAGATGAAG AGAATGAGTC AACTGGAAGA CAAAGTTGAG
GAGTTACTGA TTTGCAAGAG CGAGTTGGAA GCTGAGGTTG AGCGTTTAAA GAGTTTAGTG
AAACATCAGT GA

TA2 - ALT1 (SEQ ID No. 16)

ATGAGAGTTA ATAAATTTAT TCGTCCTTGT CGTCATATGT CTTCGACAAC TGTTGTTTCG
TTGGGGCTGA AGATCAAACC AGATTTTCAG CCCGCACCAA AGTTGACCGT CACTGATTTG
AACCCTCATA CAGTCAATGC CAAATATGCC GTAAGAGGAA AAATTCCTAC TAAGGCAGAA
CTTTTAAGAA ATCAGCTTAA CGATGTAGAC CATAAATTGC CTTTCACCAA AATTATTAGT
GCCAACATTG GTAATCCACA GCAATTAGAC CAAAAGCCAT TAACATTCTA CAGACAAATC
CTGGCCCTGC TGCAGTACCC GCCCTTGTTG GACGATCCTC GTGTTGCTTC AATTTTCCCC
AAGGATATTA TTGAGCGTGC TAGAAGCTTA TTGAAACAAA TTGGTTCCGT GGGTGCCTAC
TCCCAATCTC AAGGTGTCCC TTCTATCAGA CAGTCTATCG CGGACTTCAT CAGCAGAAGA
GACGGCCATC CTTGTGCCAG GAAAGAGCTA ATATATCTTA CCACCGGTGC ATCCACCGCT
GTCACTTATC TGTTAACTCT ACTGGGACAA AATGAGAAGA CCGGTTTCTT GATTCCGATT
CCTCAATATC CTCTCTATAC TGCCACACTG ACCCTCAACA ACCGTACCGC ATTGCCATAC
TACTTGAACG AGGAAGACAA CTGGTCCATC GAGTGCGATG AAATAGAGTC CATTATTCTG
GATTCCAAAG CTAAAGGAAT CGACCCTCGT TGCCTGATAG TAATCAATCC TGGAAACCCC
ACTGGTGCCA TTCTTTCCTA TGAAGCCATT GAGGATATCT TGAATGTTTG TGCCAAGTAT
GGTTTGGTTG TTATTGCCGA TGAAGTGTAT CAGGAAAACA TTTTTGACGG TAAGTTCATT
TCCGTTAGAA AAGTGTTGCT AGATCTATTA CAAGGCCCAA AGGCCGACTT GTATAAGAAC
ATCCAGTTGG CATCGTTGCA TTCGACCTCC AAGGGTATTT CTGGAGAATG TGGGCAAAGA
GGTGGCTATA TGGAACTCAT AGGTTTCGAC GAATCTGTGT TAGAACAGAT CACCAAGCTT
TCTTCTATTT CTCTGTGCCC AGTGGTGACA GGCCAAGCTC TGGTAGAACT AATGATTAAC
CCGCCCAAGG AAGGAGATGA ATCTTACAAA CTGTATTACG ATGAGACCCG TGCTATTCAC
GATTCCCTAG CCGAAAGGGC CACGAAATTG TACGATGCGT TCAACAGCAT GGAGGGTGTT
GTTTGCCGCA AACCCCAAGG GGCCATGTAT TGTTTCCCCA AAATCACTAT TCCTGAGAAG
GCGATCAAGA CCGCCAAGGA GCTTGACTTT GAACCTGATG AATTTTACTG CAATGAATTG
TTAGAGACTA CTGGTATTTG TGCTGTTCCA GGCTCCGGAT TTGGACAGAA GCCGGGAACA
TATCACATTA GAACAACTTT CCTTGCTCCA GGGTCCGAGT GGATCAACGA CTGGGTCGTC
TTCCACAAGA AGTTCATGCA AAAGTATAAA TGA

## Figure 3 cont'd

TA3 - SER1 (SEQ ID No. 17)

ATGGCAAAAA CTTTGGAAAG AGAAGAACCA CATTATTTTG GGGCTGGGCC AGCGCTACTG
CCTACTTCAG TATTGCAAGA GGCTGCCTAT GACTTGGTCA ACTACCAGGG TGTTGGTATA
GGTATTGGTG AAATTTCTCA TCGTTCTAAG CAAGCTAGTG AAGTCATTGA TAACACGAAG
GCCAATCTAG TTAGTTTGTT GAACATCCCT GATACACATG AGGTTTTTTT CTTACAAGGG
GGAGGAACAA CAGGATTTTC TTCTATTGCT TCCAACTTGA CTGCCTCCTT TGTTAAGAAA
ACTGGAAGGA AGGGCCGTGC TGCTTATGCA ATCACTGGAA CTTGGTCTAA GAAATCTGTT
GAAGAAGCCC AGCGTTTGGG TTTGGACGTT GACATTGTTT TGGATAGTAA AAAAGTTGAT
GGAAAATTTG GATCCATACC CCCAGTTGAC AAATGGAGCA TCCCCACTGA TCTATCAAAC
ACTTCTTATG TGTATTATTG TGATAATGAA ACTGTGAACG GTGTTGAGTT CAAGGAGTTC
CCATTTGAAA GTTTTCCTGG TGTTGAAGTA GTTGCAGATA TGTCATCCAA CATCTTATCA
AAGAGGCTGG ATGTCAGTAA ATATGGATTG ATAATGGCCG GTGCTCAAAA AAATATTGGA
TTAGCTGGAA TCACGATCTA TATTATCAAA AAGTCGTTGC TTGAGCAAGC TGATGATGCC
ACTTTGAAAG CTCTTGAAGT GCCTCTTTCA CCAATTGCGG TTCATTATCC TACAGTAGTC
AAGAACAACT CCGCTTACAA CACAATTCCC ATTTTCGCAG TTCATGTCGT CAATTTGGTC
CTCAGGCTAT TAGTAAAGAA CGGTGGTGTC GATGCCCAAC AGAAGATAAA CGAGGAGAAG
GCTTCCATAT TGTATGGTGC GTTGGAGAAG TTCCCACAAG TTTATGAGTT GCCTGCTTCC
ACCGATGCTC GTTCTCATAT GAATGTGGTT TTCACATTGA ATGGTGACTT GGATGCTGAA
TTCTTAGCCC AAGCTGCAGA AAGGAAGCTA AATGGATTGA AGGGTCACCG CTCGGTTGGT
GGAATGAGAG CCTCCATTTA CAATGCTGTT TCCTTGCACA GTGTCCAAGA GTTAACGAAA
TTCATCATCG AATTTGCTGA AGCTCATTCT TGA

TA4 - SER3 (SEQ ID No. 18)

ATGGCTTCTC CCCAAGATAT TAAGCAGTTA AGCAGTGGCT TCAATGCTGT CGGTTTGTCC
ACTTCCCCTG GAGTTGTTTC TGCTTCCCCT AATGACTCCT ACCTGTCCCA GGTCAGACAG
CGTAGTTTCT CTGCTGCCAA ACACGCTAAA CCATTGAAAC CATTCTCCAC TGGAGATATT
AAAATTTTGC TGCTAGAAAA TGTTAACGAA ACCGCCAGAG AGATCTTTGC TGAACAGGGA
TATCAAGTGG AGTTCCACAA GTCTTCGTTA CCAGAGGATG AGCTGATTGA AAAAATCAAA
GATGTTCATG CCATTGGTAT CAGGTCAAAA ACTAAGTTAA CCGAAAAGGT TTTGAAACAC
GCTAAAAACT TGATAATCAT CGGGTGTTTC TGTATTGGTA CCAATCAAGT CGACTTGGAA
TATGCGGCAA AATCGGGTGT TGCTGTGTTC AATTCCCCAT TTTCAAACTC CAGATCCGTT
GCTGAATTGG TTATTGCCGA GATTATTACT CTTGCCAGGC AACTAGGAGA TAGATCTTTG
GAGATGCACA CCGGAACCTG GAATAAAGTT TCCAACAAAT GTTGGGAGAT CAGAGGTAAA
ACGCTTGGAA TTGTGGGTTA CGGGCACATT GGTTCCCAGT TGTCCGTTCT GGCTGAAGCC
TTTGGTATGA ACGTTATCTA CTACGATGTC TTGATGATCA TGGCTCTGGG TTCTGCTAAA
CAGGTCCGTA CATTGAATGA CCTCCTTGCC CAAGCTGATT TTGTCACTTT GCATGTTCCC
GAAAATCCGG AGACTAAGAA CCTTATCAGC TCTCCTCAAC TGGCTGCTAT GAAGGATGGA
GCTTACCTGA TCAATGCTTC AAGAGGAACC GTGGTGGACA TTCCTGCCTT GGTTGAAGCG
ATGAAACTAG GTAAGCTGGC GGGTGCTGCT TTGGATGTTT ATCCACAAGA ACCAGGTAAG
AATGGTCCAA ACTTCACCAA CGAGTTGAAC TCTTGGGCTA CTGAGCTAAC TTCTTTGAGA
AACATTATTC TGACCCCACA TATTGGTGGT TCTACTGAAG AGGCCCAATC TGCAATTGGA
ATTGAAGTAG GAACTGCTCT CACAAAGTAC ATTAATGAGG GAACTTCTAC AGGTGCTGTC
AACTTCCCAG AAGTTGCTTT ACGTGCTCTA GATCTTGACC AAGAAAACTC TGTTAGAGTG
CTTTACATTC ATCAGAACGT GCCTGGTGTG TTGAAAACTG TGAACAACAT TTTGGGTAAC
CATAACATTG AAAAGCAGTT CTCTGACTCC AGAGGAGACA TTGCCTACTT GATGGCCGAT
ATTTCTGATG TGGATGCTTC AGACATCAAG TCTTTGTACG AACAACTGGA GCACACCCCT
TACAAAATCG TCACCCGTCT GCTATACTAG

## Figure 3 cont'd

TA5 - ARO3 (SEQ ID No. 19)

ATGTTCATTCAAAACGATCATGTCGGTGACAGATCCCGCTTAGAAGACTGGAGAATACGTGGATACGACC
CTCTGACGCCCCCCGACCTGTTGCAACATGAGTATCCACTTACCCCAAAAGCAGAGGAGAACATTCTCAA
GGGAAGAAACGAAGCTGTTGATATCCTGAAAGGTAAGGATGACAGGCTATTAGTCATTGTCGGACCTTGT
TCCATCCATGATCCTAAGGCAGCTTTAGACTACTGCGAGAGATTAGCCAAGTTTAACGAGACTATCAAAG
GTGAGCTTCATATCATAATGAGAGCTTATTTGGAGAAACCAAGAACCACTGTTGGTTGGAAAGGTCTGAT
AAATGACCCAGACATCGATGGTTCGTTTCAAATCAATAAAGGTCTTCGTATCTCGAGGGAGCTTTTCGTT
AAACTTACTGAACAGATTCCTATTGCTGGTGAGATGCTGGACACTATCTCCCCTCAATTCCTGTCTGATC
TCTTCTCCTTAGGAGCGATTGGCGCCAGAACCACAGAGTCTCAACTGCACAGAGAACTTGCTTCCGGTTT
GTCCTTCCCCGTGGGATTCAAGAATGGTACAGACGGTGGGCTCACAGTTGCTATAGATGCCATGAGAGCT
GCTTCGCATCCTCACCATTTCCTGTCTGTCACAAAACCAGGAGTTGTGGCTATTGTCGGTACCGAAGGAA
ATGAAGACACCTTTGTTATCCTAAGAGGTGGTAAGAACGGTACAAACTACGACGAGGCCTCTGTTGACGC
TGCCCACGCACAGTTAGAGAAGACCGGTATCCTGGGCTCTGGTCCTGCCATCATGGTGGACTGCTCTCAT
GGAAATTCCAACAAAGACCACCGTAACCAACCTAAGGTAGCCCAAGTTGTTGCTGACCAAATCCGTAAAG
GATCCCGAAAAATATGTGGCCTCATGATTGAGAGCAATATCGTTGAAGGTAGACAGGATGTTCCCAAAGA
GGGAAAGTCACATCTACGTTATGGATGTTCCATAACTGATGCCTGTATCTCCTGGGAGGATACTGAGAAG
GTTCTACGAGTCTTAGCAGATTCGGTCATTGAACGTCGTAATCTCAAGAATTAG

TA6 - ARO7 (SEQ ID No. 20)

ATGGAGTTCAAGAAACCCGCAACAGTGCTGAATCTGGCCAATATCAGACAGGCCTTGGTCCGAATGGAAG
ACACCGTGGTGTTCAGCTTCATCGAAAGGTCCCAGTTCTACGAATCTCCATCCGTGTACGCCCCCAATAA
GTACAAGATCCCCAACTTTTCAGGTTCTTTCTTGGACTGGTTGCTCTTGCAAAACGAGAAGACCCACTCG
CTTGTCAGGCGTTACGAGTCTCCAGACGAGACCCCCTTTTTCCCTGATCAACTGGAAGAATCGTTTCTTC
CAAGTCTAAACTACCCTCCAATTTTGGCTGACTACGCCGATGAGGTGAACGTAAATGACGAAATCAGAAC
TGTGTACATTGAAAAGATAGTTCCCCAAATTGCAGCTAAGAAAGGTGATCAAGATGAGAATATTGGATCC
ACAGCCTGTGCAGATGTTGATTGTTTGCAGGCTTTGAGTCGTCGAGTGCATTTTGGAAAATTTGTCGCAG
AAGCTAAGTTCCAAAATGAAATGGCCCGCTACACTAATTTGATCCGCAACAAGGACATTAAGGGCATTGA
AGAAGCTATTACAGATTCCGCAGTTGAAGCTAAGATTTTGGAACGTTTAATTGCCAAAGGTCATGCCTAT
GGTACGGACCCCACGCTTAGGTATTCGCAGAACCCTCAATCAAAAGTACAGCCAGAGGCAATTGCTAAGA
TATACAAAGAGGTAGTTATACCTCTGACCAAGAAGGTGGAGGTTGAATATCTGCTTAGAAGGTTAGGAGA
TGACGACGAAGCAGAAGCTCAAGACCCTCAACGCAAGACTCAGCTGATGAACAGACTATTATCCAACAGC
TTGTGGTAA

TA7 - ILV5 (SEQ ID No. 21)

ATGTCCGTAA GAAATGCCAC TTTTCGTTTA AGCCGTGCTG CTATTGCTAA GAGAACTTTG
GTCAGAATTG CTTCTCAAGC CTCATTAAGA CCAACGACAC TGAATTCTGC CACTGGAGTC
GCTCGTTCGC AGTTGACTTT TAGTCGTGGT GTGAAAACCA TTAATTTTGG TGGTGTCGAA
GAAGTTGTCC ACGAGAGATC TGATTGGCCA AGAGAGAAAC TGCTAGAGTA TTTCAAGAAT
GACACTTTAG CTTTAATTGG CTACGGATCT CAGGGTTATG GTCAAGGATT AAATCTAAGA
GATAATGGTC TCAATGTTAT TATTGGTGTC CGAAAGAACG GCGCTTCTTG GAAGGCTGCT
ATTGAGGATG GCTGGGTTCC TGGTGAAAAC CTTTTTGATG TCACTGAAGC TATAAAAAAG
GGTTCTATCA TCATGAATCT TTTGTCTGAC GCCGCCCAAT CTGAGACTTG GTCTACCGTC
AAACCCTTAT TGACTGAAGG TAAAACTTTA TACTTTTCTC ACGGATTTTC TCCAGTTTTC
TCAAACCTTA CCCATGTAGA GCCACCTTCC AACATTGACG TTATTTTAGC TGCTCCTAAA
GGTTCAGGTA GAACTGTTCG TTCCCTTTTT AAAGAGGGAC GTGGAATCAA CTCTTCCTAT
GCTGTTTGGA ACGACGTCAC CGGAAAAGCT GAAGAAAAAG CCATTGCTTT GGCTGTAGCA
GTCGGATCTG GATACGTTTA TCAGACCACA TTTGAGCGTG AAGTTAACTC CGACTTGTAC
GGTGAGAGAG GTTGTTTAAT GGGAGGTATC CATGGTATGT TTTTGGCACA ATACGAGGTT
CTAAGAGAGA ATGGACATAC GCCATCAGAA GCTTTCAACG AAACCGTCGA AGAGGCTACT
CAGTCTTTGT ATCCCTTGGT TGGAAAGTAT GGTATGGATT ACATGTATGA TGCCTGCTCC

## Figure 3 cont'd

ACTACTGCTC GTAGAGGTGC GTTAGACTGG TATCCAATTT TCAAGGATGC CCTCAAGCCA
GTGTTCGAAG ATCTTTACAA TTCAGTAAAG ACAGGTAAGG AAACTCAACG TTCTCTTGAC
TTCAATTCAC AACCTGATTA TAGGGAGAAA CTTGAAAAAG AACTGGAAAT TATCCGTAAT
ATGGAGATTT GGAAGGTTGG AAAGGAAGTC CGCAAACTAC GTCCAGAAAA CAACTGA

TA8 - LEU4 (SEQ ID No. 22)

ATGCCTATGC TAAAGGACCC CTCCAAGAAA TACGTTGCCT TCAAGCCGCT TAACCTGCCT
GACAGACAAT GGCCATCAAA AACTCTTCAA GCCCCTCCAA GATGGTTATC CACAGATTTG
AGAGATGGTA ACCAATCTCT GCCAGACCCC ATGAGTGTGG CCGAGAAAAA AGAATACTTC
AACAAGCTGG TTCAGATCGG GTTCAAGGAG ATTGAAGTTG CTTTCCCTTC AGCTTCGCAG
ACTGACTTTG ACTTTACCAG ATATGCGGTT GAGAATGCTC CAGCAGACGT AGCTATTCAA
GTTCTGTCCC CATGCAGACC TGAGTTGATC AAGAGAACTG TTGAGTCCTT GAAAGGTGCC
AAGAAAGCAA TTGTACACAT TTATTTGGCT ACTTCAGACT GTTTCAGAAA CATTATATTT
GGCCTTTCCC AGGAAGAATC TCTTGAAATG GCAGTAAACT GCACGAAACT GGTTAGAGAG
TTGACCAAGG ATGCTCCTGA AATGCAAGAT ACCGTATGGG GATTCGAATT CTCTCCGGAG
ACATTTAGTG ATACTGATCC GGACTATGCT CTGCGTGTTT GCGAGGCCGT GAAGGCTGCT
TGGGGCCCAT CAGAAGAAAA CCCAATCATA TTCAACCTTC CTGCTACTGT TGAAATGTCT
ACTCCAAACG TCTACGCTGA CCAAATTGAA TATTTTTCTA GAAACATCAG TGAGAGAAAG
ACAGTAGCAA TATCCCTGCA CCCACACAAT GACCGTGGTT GTGCCGTAGC TGCAGCTGAA
TTAGGACAGA TGGCTGGAGC TGACAGAATA GAGGGTTGTC TGTTCGGAAA CGGTGAAAGA
ACCGGTAACG TTGACTTGGT AACTCTGGCC CTTAACTTGT ACACTCAGGG AGTTTACCCT
CATTTGGACT TTTCAGACAT TACTTCAGTA ATTGACATCG TTGAGCGATG CAACAAGATC
CCAGTTCATC CAAGAGCCCC ATACGGAGGT CAACTGGTGG TCTGCGCATT CTCTGGGTCC
CACCAAGACG CTATTAAGAA GGGGTTCAAG AAACAGGAAG AAAGACAGGC CAAGGATCCC
AACGCCAGAT GGCAGCTACC TTATCTTCCA TTGGATCCTC AAGATATTGG AAGGACTTAC
GAGGCTATCA TCAGAGTCAA CTCTCAATCT GGAAAGGGTG GTGCTTCATG GGTTATTCTT
AGAAACTTGG AATTGGATCT TCCTCGTGCT TTGCAAGTTT CCTTCTCTAA GATCGTCCAA
AATGAGTCAG AAGTTAAAGG AAGAGAGTTG TACAACAAAG AGATCACAAA CCTGTTCAAG
AACTCTTACT TGTTGATGA TGATGACACC CAGCACGATC TTTACTTCCA ACTGGTAGAT
TACTCTATTA CAAATACATC CAAAAGCGCT AGACTGATCA ACGTTGAACT CCAAGTTGGA
AAAGACACTG TTTCCTTAAA AGGTATTGGT AACGGTCCAA TTTCCTCATT TGCCAGCGCT
ATTTCTTCTT ACCTGAAAAA AGATGTACTG GTCTTGAACT ACCATGAACA CTCTTTAGGA
AAAGATTCCA ACTCCAAGGC CGCTACATAC TTACACTGTG CCATTGGCGA TGACTGCAAG
GTTTGGGGTG TTGGTATCCA TGAAGATGTT TCTCAGGCAT CATTCTTATC TTTCATCAGT
CTTTTGAACT CTGCAAGGAG AGATGGTAAA ATATAA

TA9 - MAE1 (SEQ ID No. 23)

ATGAGCTCTC TTTACATGCC CTCAAAATCA ATGAAACGGC TTACTGTGAG CTGTCGTCGT
TTTATATCTT CGGCTCAACC AAAAATTGTT ACACACAAAA TACTCACACC AGTTGGGACA
GAAGCGGCAG CCAAAGTAGA TGTTCCCAAG ACGACCCGTC TTTCTGTGGA AGGCCCTATC
GAATGCCACT TGCAAGGATT TGAGCTATTA AACTCTCCAT TGTTTAACAA GGGTTCTGCG
TTCACTCCAG AGGAAAGAGC AGCCTTTGGG TTAGAAGGGT TACTTCCTCC AGTAGCCAAT
GACTTAAACG AACAAGCAGA GAGAGCTTAC AAGCAGCTGT CTTTCTTGAA AACCCCACTA
GCAAAGAATG ATTTTTGTAC CTCCATGAGA CAGCAAAATA AGGTTCTTTT CTACGAGTTG
GTGAGGCGAC ACATTAGAGA GCTACTGCCC ATCATCTATA CTCCTACGGA GGGGGATGCC
ATTGCCGCAT ATTCACATAG ATTCCGTAAA CCTGAGGGTT GTTTCCTTGA TGTCACGGAC
CCTGAATCCA TAGAGGATCG TTTATCTCGT TTCGGTGAGA GTAAAGATGT GGATTATATT
GTAGTTTCTG ACGGAGAAGG AATTTTGGGA ATTGGTGATC AGGGTATAGG TGGTGTTCGT
ATTGCCATTT CCAAGTTAGC TTTAATGACT CTTTGCGGTG GTATTCATCC TGGAAGAGTT
ATCCCTGTCG TTGTTGATGC CGGTACAAAC AATAAGGCTC TTCTGGATGA CGAACTATAC
ATGGGTAGCC GCTTCCCTCG AGTTCGTGGT GAAGAGTACT ATTCTTTTTT TGACAAGTTT

## Figure 3 cont'd

ATTGAAGTTG TCAAGCGTAG ATTCCCATCT GCAGTGTTGC ATTTTGAAGA TTTCGGTGTA
ACTAATGCCA AGCCCCTATT GGACAAGTAT CGTTCCGTTC TTCCTTGCTT CAATGACGAC
ATTCAAGGTA CTGGGGCCGT TGTAATGGCA TCTATGGCAG CCGCATTGAA ACTTACGAAT
CGAGATTTGT TAGATTCCAC CATCGTTATT TATGGCGCTG GTTCTGCTGG TCTTGGAATT
GCTTTCCAGA TCGTCAATCA CATGGTTAAC CATGGTGCCA CTAAAGAAGA AGCTTACTCA
AGGATTCATT TGCTAGATCG TCATGGCTTG ATTCTCACAA ACATGAGCGA TGTATCCAAC
AAAGATCAAA TGCTCTTTGC GGATGATCCT AGCAGCTGGG ATGGTATTAA TACCAAATCC
CTAGTTGATG TCATCGAGAA ACTCAAACCC ACCACATTAA TTGGTTGCTC CACCCAAGCC
GGAGCCTTCA CAGAGGAAGT CATTAAGACA ATGTACAAAT ACAACCCCAG GCCAATAGTG
TTCCCTCTTT CCAACCCTAC TAGACTTCAT GAAGCTGTTC CTGTTGATGT TATGCGATGG
ACGGATGATA ATGCCCTAAT TGCTACTGGT TCTCCCTTTA AGCCTGTTCG TGGTCATGTC
ATCAGTGAAA ACAATAACTG CTTCTCATTC CCAGGTATCG GATTGGGAGC CGTTCTTTCT
AGAGCTACAA CCATCTCCGA CACTATGATT TCCGCTGCAG TTGATGAACT TGCCAGTGGT
TCCCCTGCAT TCAAGGACCC TAAAGCAGGT TTGCTTGCCC CAGTCGAAGT GATAAATGAG
ACATCTGCTA AAGTGGCAGC CGCCTTAATC CTTCAAAGTA TCAAGGAAGG CACTGCCAGA
GTGGAAGAAG AGCTGTCTCC AAATGGAGAT AAAGTTTCTG TCCCTAGAGA CTTTGAGGGT
TGTGTTGCAT GGGTCAAATC CCAAATGTGG CGACCAGAAT ATAGACCCAT GGTGAAGGTG
GAACGAGTTC CCGAAATCCA TACGCATCAG TCATAG

TA10 - PRO1 (SEQ ID No. 24)

ATGGGTGAAA ATTGTTACAC TATCGTCGTT AAATTGGGAA CCAGTTCCGT GGTAGATGAA
GAAACCAGGG AACCAAGAAT CGCAACTATG TCTCGTATTG TCGAGACCCT TGTTAAGCTG
CGAAGGAAGG GCCATCGAAT TGTCATCGTC TCCTCAGGTG CCATCGCTGT GGGCATGAAG
AGAGTCAACA TGAAGCGGAA ACCAAAAAAG TTACAGCAAG TGCAGGCATT GGCTGCTATA
GGACAAGGCC GTTTGATAGG ACTTTGGGAC GACCTTTTCC GTCAGTTGAA TCAGCCTATT
GCGCAGATTT TACTGACTAG AACGGATTTG GTCGATTACA CCCAGTTTAA GAACGCTGAA
AATACATTGG AACAGCTTAT TAAAATGGGT ATTATTCCTA TTGTCAATGA GAATGACACC
CTATCCATTC AAGAAATCAA ATTTGGTGAC AATGACACCT TATCCGCCAT AACAGCTGGT
ATGTGTCATG CAGACTACCT GTTTTTGGTG ACTGATGTGG ACTGTCTTTA CACGGATAAC
CCTCGTACGA ATCCGGACGC TGAGCCAATC GTGTTAGTTA GAAATATGAG GAATCTAAAC
GTCAATACCG AAAGTGGAGG TTCCGCCGTA GGAACAGGAG GAATGACAAC TAAATTGATC
GCAGCTGATT TGGGTGTATC TGCAGGTGTT ACAACGATTA TTTGCAAAAG TGAACATCCC
GAGCAGATTT TGGACATTGT AGAGTACAGT ATCCGTGCTG ATAGAGTCGA AAATGAGGCT
AAATATCTGG TCATCAACGA AGAGGAAACT GTGGAACAAT TTCAAGAGAT CAATCGGTCA
GAACTGAGGG AGTTGAACAA GCTGGACATT CCTTTGCATA CACGTTTCGT TGGCCACAGT
TTTAATGCTG TTAATAACAA AGAGTTTTGG TTACTCCATG GACTAAAGGC CAACGGAGCC
ATTATCATTG ATCCAGGTTG TTATAAGGCT ATCACTAGAA AAAACAAAGC TGGTATTCTT
CCAGCTGGAA TTATTTCCGT AGAGGGTAAT TTCCATGAAT ACGAGTGTGT TGATGTTAAG
GTAGGACTAA GAGATCCAGA TGACCCACAT TCACTAGACC CCAATGAAGA ACTTTACGTC
GTTGGCCGTG CCCGTTGTAA TTACCCCAGC AATCAAATCA ACAAAATTAA GGGTCTACAA
AGCTCGCAGA TCGAGCAGGT TCTAGGTTAC GCTGACGGTG AGTATGTTGT TCACAGGGAC
AACTTGGCTT TCCCAGTATT TGCCGATCCA GAACTGTTGG ATGTTGTTGA GAGTACCCTG
TCTGAACAGG AGAGAGAATC CAAACCAAAT AAATAG

TA11 - PRO3 (SEQ ID No. 25)

ATGTCTTCAA TTGGTAACGA ATATACAATC ACTATCCTAG GGTGCGGAGT GATGGGAACC
GCAGTTTTAT CTGCTATTTT GAACTCAGAG TTCTCCCCTT ATCCCTCAAG AATTATCTGC
TGCACAGGCT CCCAGAAGTC CGCTGAAAAA TTGGCCAGCA CTTATTCCAA GATTGAAACT
AGCTACGGAT CTGAGCAAAA CATCAGAGCC GTCAAAGAGT CAGATATCAT AATTCTAGGG
TGCAAGCCGT TCTATTGTGA ACAAATCATG GAACAGGTCA AAGAGGGCTT ACACGATCAA
TTGCTAATTT CACTTTTGGC TGGCTGGACG TTAACCCAAC TAGAAAGGTA CTCTAAATAT

## Figure 3 cont'd

GTAGCAAGGG TAATGACCAA TACCCCGGCC AAATTTGGAT GTGGAATGGC TTGTGTATCT
CTATCTTCGC ACGCCGAGCA ATACGAAGAT CTAGTACTCA AGCTGGTTGG CCCAGTGGGC
AAGGCTATTA GATTGCCCGA AAAGAATATG GATGCCGCTA CAGCTCTTGT AGGTTCAGGT
CCTGCATTCT GCTTGTTGAT GATGGAATCT CTCATCGACG GAGCCGTCCA AAAGGGTTTA
CCATTTGCTA TTGCTAAAGA TGCAGCTGCT AAAGTTATGG AAGGCACAGC AAGGATGGTT
CTCGAGACTG GAGAACATCC TGCCGCACTA AAAAGTGCTG TGTGCACCCC AGGAGGTACC
ACTATTGGTG GTCTTATGGC CATGGAAGAC CGAGGAGTCA GAAGTGGTAT CGCTCGAGGT
GTGGAAGAAG CTGCCAACAT TGCCACTAGC TTAGGAAAGA AATAG

TA12 - MET17 (SEQ ID No. 26)

ATGCCTTCTC ACTTCGACAC TTTGCAGCTG CACGCCGGTC AGACCGCTGA AGCTCCACAC
AATGCCAGAG CTGTTCCTAT CTACGCTACC TCGTCTTACG TTTTCAGAGA CTCTGAGCAC
GGTGCCAAGC TGTTCGGTTT GGAGGAGCCA GGTTACATCT ACTCTCGTTT GATGAACCCT
ACTCAGAACG TCTTTGAAGA GAGAATTGCC GCTTTGGAGG GTGGTGCCGC TGCTTTGGCT
GTTGGATCCG GTCAAGCTGC TCAATTCCTG GCTATTGCTG GTTTGGCTCA CACTGGTGAC
AACGTCATCT CCACCTCTTT CTTGTACGGT GGAACTTACA ACCAATTCAA GGTCGCCTTC
AAGAGACTGG GAATTGAATC CAGATTTGTC CATGGTGATG ACCCAGCTGA ATTCGAGAGA
CTGATCGATG ATAAGACCAA GGCCATCTAC GTTGAGTCCA TTGGTAACCC AAAGTACAAT
ATTCCAGATT TTGAGGCTCT CGCAGAGCTT GCCCACAAGC ACGGTATCCC ATTAGTTGTT
GACAACACCT TTGGTGCCGG TGGTTACTAC GTTAGACCAA TCGAGCTTGG TGCTGACATC
GTCACCCACT CCACCACTAA GTGGATCAAT GGTCACGGTA ACACCATCGG TGGTGTTGTC
GTTGACTCTG GTAAGTTCCC ATGGAAAGAC TACCCAGAGA AGTACCCTCA ATTCTCCAAG
CCATCTGAGG GTTACCACGG TTTGATCTTG AATGACGCCT TTGGACCAGC TGCCTTCATT
GGTCACTTGA GAACTGAACT GCTAAGAGAT TTGGGTCCTG CTTCAAGTCC ATTCGGTAAC
TTCTTGAACA TAATCGGTTT GGAGACCTTA TCTCTGAGAG CTGAGAGACA CGCTGAGAAT
GCTTTGAAGC TGGCCAAATA CTTGGAAACC TCTCCATACG TCAGCTGGGT CTCTTACCCT
GGTTTGGAGT CTCACGACTA CCACGAGGCC GCTAAGAAGT ACTTGAAGAA CGGTTTCGGT
GCTGTATTGT CTTTTGGAGT CAAGGATCAT GGCAAGCCAG CGCTCACTCC CTTCGAAGAG
GCTGGTCCTA AGGTTGTAGA CTCCCTGAAG GTTTTCTCCA ACTTGGCTAA CGTTGGTGAC
TCCAAGTCTT TGATCATTGC TCCTTACTAC ACTACTCACC AACAGTTGTC TCACGAGGAG
AAGCTGGCTT CCGGTGTCAC CAAGGACTCT ATCCGTGTTT CTGTCGGAAC AGAGTTCATC
GATGATCTTA TTGCAGACCT TGAACAGGCC TTTGCCCTTG TTTACGAGGA GGCAAACACA
AAGTTGTGA

TA13 - CYS4 (SEQ ID No. 27)

ATGTCAGACA ACAACCAACC TTTGCCACCG GTTTCCAACT CCATTCTGGA GCACATTGGA
AAGACTCCAC TTGTTAAACT TAACAGAATA CCCAAAATTC TTAATTTGAA ACCACAGGTT
TATGCTAAAG TTGAACTGTT CAACTCAGGT GGTTCCATCA AGGACCGTAT TGCTCTGAGA
ATGGTTGAGC AAGCTGAGAA AGAGGGTAGA ATCAAGCCAG GGTACACGCT AATTGAGCCA
ACGTCAGGTA ACACTGGTAT TGGTCTGGCA TTAGTAGGTG CAGTCAAGGG CTACAGAACT
ATTATCACTC TTCCAGAGAA AATGTCTAAT GAAAAAGTTG CTGTGCTGAA AGCTCTCGGG
GCTGAAATTA TAAGAACTCC AACCGAGGCT GCTTGGGATT CCCCAGAGTC TCACATTGGT
GTTGCAAGAA GGTTGGAGAA AGAAATTCCA AATGCTGTAA TCTTAGACCA GTATGGTAAT
GTTAACAATC CAGATGCCCA TTTCTACACC ACTGGTGCGG AAATCTGGGA TCAGACTAAT
GGTAAAGTTA CCCACGTTGT TGCTGGTGCA GGAACTGGAG GTACGATTAC AGGTATTGCC
AAATTTTTGA AGTCTAAAAA CCCTAATATT CAAATTATCG GTGCTGATCC TCATGGTTCC
ATTCTAGCTC TTCCTGAATC ATTGAACACT AGTAATGAGC AATACAAAGT TGAAGGTATT
GGGTATGACT TTATCCCAGA AGTTTTGGAT AGAGAAATTG TGGACACTTG GTACAAAACA
GATGATAAGG ATTCCTTCAA GCTGGCCAGG CAATTGATTT CTGAAGAAGG AATTCTAGTT
GGAGGATCTT CCGGCTCTGC GCTTTCTGCT GCTGTTAAAA TAATCAATGA TGCCAAGCTT
GATGAAAGTG CTGTAGTTGT TGTCGTTTGT CCAGACAGTA TCAGATCTTA TCTAACCAAG

## Figure 3 cont'd

```
TTCGCTGATG ATGACTGGAT GAAACTCAAT GGATTCACTG AAGACGAGCC TGCACCTCTC
AAAAGGAAGT CCAGCTTTTC AAAGGACACT CTGTCGTCCT ATACGATCAA AGATTTGGCG
CTCAAACCTG TGGTCTCTGT CACCATTGAC GATTCAATTG AATCCACAAT TAATATCCTT
CAAACCAAGG GTTTTGATCA GTTACCAGTG TTGAAGAACG ATAAGCTTGC TGGTATTGTC
ACTCTGTCTC AATTGTTACG ATTACTTTCT AATAAGAAGA TTCATCTTAC TTCCAAGGTA
GAATCTGCCT ACTTTGATTT TTCCAAGCTT GAAAACTTTG AAAAATCTTA CAACATTAAC
AAGGAGTCAA CAAACAAGAG AAGAGAATAC CAGAAGATCA CTACTAGCAC CACGCTGGCC
AAATTGAACA AGTTTTTTGA GACCAGTTCA GCTGCTATAG TAGAAAATGA TAAGGGTCAA
CCGATCCACA TCGTTTCCAA GGTTGATCTG CTTTCTTTCT TAGCCTCCAA AGGAGAGTTT
AATTAA
```

TA14 - HOM2 (SEQ ID No. 28)

```
ATGAAGAAAG CAGGTGTTTT GGGCGCAACC GGAAGTGTTG GCCAACGTTT TATACTTTTA
CTAAGCGATC ATCCAGAGTT TGAGTTGTCT GTTCTCGGAG CTTCCTCTCG CTCTGCTGGA
AAGAGGTATG AAGATGCCGT CGTCTGGAAG CAAACTGATC TTTTGCCTGA GTCCGCAAGA
AATATCATTG TTCAAGAGTG CAAAGCTGAG GCTTTCAAGG ACTGTGACAT TATCTTCAGT
GGGTTAGACG CCGATTATGC TGGAGAAATT GAAAAGGAAT TTGTTTCTAG TGGTTTAGCT
GTTGTCTCTA ACGCAAAGAA CTACAGAAGA GAGGCAGACG TTCCTTTGAT CGTTCCTATT
GTCAATCCAG AGCATCTGGA AATTGTTGAA CAGAAAGTTA AGTCTGGACA AAAGGGATTC
CAAATTTGCA TTTCTAACTG TTCCACTGCT GGGCTCGTTG CTCCTTTGAA ACCTCTGGTG
GAGAAGTTTG GACCAATTGA CCTCCTGACA ACTACTACTT TACAGGCTAT TTCCGGAGCC
GGATTTTCTC CAGGTGTTCC AGGAATAGAT ATATTAGACA ATATTGTACC ATTCATCAGT
GGTGAAGAAG AGAAAATGGA ATGGGAGACC AGAAAGATTT TGGGAGGACT CTCCGAGGAT
AAGATGGAAT GCAGACTTGT TAGTGAAGAC GCAATGAAAG TTTCCGCTCA ATGTAATAGA
GTTCCTGTAA TTGATGGTCA TACTGAGTGC ATTTCCCTGA GATTCGAACG AAGACCCCCT
CCAAGTGTTG AGGCAGTCAA GCAATGTCTT AGAGACTACG TTTGTGAGGC CAGCAAATTG
GGCTGTCCAT CCGCTCCCAA GAACACCATT CACGTTTTGG AACAAAATGA CCGACCACAG
CCAAGGTTAG ACCGTGTACG TGATAATGGG TTTGGAGTTA GCGTCGGCAG AATCAGAGAG
GATCCAGTGT TAGATTTCAA GATGGTAGTA TTGTCCCACA ACACAATCAT CGGAGCTGCT
GGTTCTGGAG TACTCATTGG AGAGATCCTG TTGAAAAAGG GTCTCATTTA A
```

Europäisches Patentamt
European Patent Office
Office européen des brevets

# EUROPEAN SEARCH REPORT

Application Number
EP 14 19 0469

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2012/100621 A1 (NETT JUERGEN [US]) 26 April 2012 (2012-04-26) * the whole document * & DATABASE Geneseq [Online] 21 June 2012 (2012-06-21), "Pichia pastoris ARG9 protein, SEQ ID NO:6.", retrieved from EBI accession no. GSP:AZV50730 Database accession no. AZV50730 * sequence * ----- | 1-9,11, 12,14,15 | INV. C12N15/67 C12N15/81 |
| X | G. CHUA ET AL: "Identifying transcription factor functions and targets by phenotypic activation", PROCEEDINGS OF THE NATIONAL ACADEMY OF SCIENCES, vol. 103, no. 32, 8 August 2006 (2006-08-08), pages 12045-12050, XP055132698, ISSN: 0027-8424, DOI: 10.1073/pnas.0605140103 * the whole document * ----- | 12 | TECHNICAL FIELDS SEARCHED (IPC)<br>C12N |
| A | M.-J. HAN ET AL: "Engineering Escherichia coli for Increased Productivity of Serine-Rich Proteins Based on Proteome Profiling", APPLIED AND ENVIRONMENTAL MICROBIOLOGY, vol. 69, no. 10, 1 October 2003 (2003-10-01), pages 5772-5781, XP055132576, ISSN: 0099-2240, DOI: 10.1128/AEM.69.10.5772-5781.2003 * the whole document * ----- | 1-9,11, 12,14,15 | |
| | -/-- | | |

~~The present search report has been drawn up for all claims~~

1

| Place of search | Date of completion of the search | Examiner |
|---|---|---|
| Munich | 15 January 2015 | Rutz, Berthold |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 14 19 0469

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | KRUGER W D ET AL: "A yeast system for expression of human cystathionine beta-synthase: Structural and functional conservation of the human and yeast genes", PROCEEDINGS OF THE NATIONAL ACADEMY OF SCIENCES, NATIONAL ACADEMY OF SCIENCES, vol. 91, 1 July 1991 (1991-07-01), pages 6614-6618, XP002904262, ISSN: 0027-8424, DOI: 10.1073/PNAS.91.14.6614<br>----- | 1-9,11, 12,14,15 | |
| Y | GERHARD STADLMAYR ET AL: "Genome-scale analysis of library sorting (GALibSo): Isolation of secretion enhancing factors for recombinant protein production in Pichia pastoris", BIOTECHNOLOGY AND BIOENGINEERING, vol. 105, no. 3, 15 February 2010 (2010-02-15), pages 543-555, XP055131823, ISSN: 0006-3592, DOI: 10.1002/bit.22573<br>* the whole document *<br>----- | 1-9,11, 12,14,15 | |
| Y | KEITH EJ TYO ET AL: "Imbalance of heterologous protein folding and disulfide bond formation rates yields runaway oxidative stress", BMC BIOLOGY, BIOMED CENTRAL, LONDON, GB, GB, vol. 10, no. 1, 1 March 2012 (2012-03-01), page 16, XP021118800, ISSN: 1741-7007, DOI: 10.1186/1741-7007-10-16<br>* the whole document *<br>-----<br>-/-- | 1-9,11, 12,14,15 | TECHNICAL FIELDS SEARCHED (IPC) |

~~The present search report has been drawn up for all claims~~

1

| Place of search | Date of completion of the search | Examiner |
|---|---|---|
| Munich | 15 January 2015 | Rutz, Berthold |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 14 19 0469

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GASSER BRIGITTE ET AL: "Transcriptomics-based identification of novel factors enhancing heterologous protein secretion in Yeasts", APPLIED AND ENVIRONMENTAL MICROBIOLOGY, AMERICAN SOCIETY FOR MICROBIOLOGY, US, vol. 73, no. 20, 1 October 2007 (2007-10-01), pages 6499-6507, XP002499993, ISSN: 0099-2240, DOI: 10.1128/AEM.01196-07 * the whole document * ----- | 1-9,11, 12,14,15 | |
| A | JAN HEYLAND ET AL: "Carbon metabolism limits recombinant protein production in Pichia pastoris", BIOTECHNOLOGY AND BIOENGINEERING, vol. 108, no. 8, 11 March 2011 (2011-03-11), pages 1942-1953, XP055131881, ISSN: 0006-3592, DOI: 10.1002/bit.23114 * the whole document * ----- | 1-9,11, 12,14,15 | TECHNICAL FIELDS SEARCHED (IPC) |
| A | PARK S J ET AL: "Global physiological understanding and metabolic engineering of microorganisms based on omics studies", APPLIED MICROBIOLOGY AND BIOTECHNOLOGY, SPRINGER, BERLIN, DE, vol. 68, no. 5, 1 September 2005 (2005-09-01), pages 567-579, XP019331964, ISSN: 1432-0614, DOI: 10.1007/S00253-005-0081-Z ----- | 1-9,11, 12,14,15 | |

-/--

~~The present search report has been drawn up for all claims~~

1

| Place of search | Date of completion of the search | Examiner |
|---|---|---|
| Munich | 15 January 2015 | Rutz, Berthold |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 14 19 0469

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ALEXANDRA GRAF ET AL: "Yeast systems biotechnology for the production of heterologous proteins", FEMS YEAST RESEARCH, vol. 9, no. 3, 1 May 2009 (2009-05-01), pages 335-348, XP055131911, ISSN: 1567-1356, DOI: 10.1111/j.1567-1364.2009.00507.x<br>----- | 1-9,11, 12,14,15 | |
| A | Alan G Hinnebusch: "Translational regulation of GCN4 and the general amino acid control of yeast", Annual review of microbiology, 1 January 2005 (2005-01-01), pages 407-450, XP055132693, United States DOI: 10.1146/annurev.micro.59.031805.133833 Retrieved from the Internet: URL:http://search.proquest.com/docview/204725742<br>----- | 1-9,11, 12,14,15 | |
| | | | TECHNICAL FIELDS SEARCHED (IPC) |

~~The present search report has been drawn up for all claims~~

1

| Place of search | Date of completion of the search | Examiner |
|---|---|---|
| Munich | 15 January 2015 | Rutz, Berthold |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

Application Number
EP 14 19 0469

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-9, 11, 12, 14, 15(all partially)

☐ The present supplementary European search report has been drawn up for those parts of the European patent application which relate to the invention first mentioned in the claims (Rule 164 (1) EPC).

Europäisches Patentamt
European Patent Office
Office européen des brevets

**LACK OF UNITY OF INVENTION SHEET B**

Application Number
EP 14 19 0469

The Search Division considers that the present European patent application does not comply with the requirements of unity of invention and relates to several inventions or groups of inventions, namely:

1. claims: 1-9, 11, 12, 14, 15(all partially)

method of increasing the yield of a protein of interest in a fungal or yeast host cell, comprising overexpressing a polynucleotide encoding a protein which is involved in one or more proteinogenic amino acid biosynthetic pathways wherein said polynucleotide encodes a helper protein having an amino acid sequence as shown in SEQ ID NO: 1 or a functional homologue thereof having at least 30% sequence identity thereto, host cell engineered to overexpress said helper protein

---

2-14. claims: 1-15(partially)

method of increasing the yield of a protein of interest in a fungal or yeast host cell, comprising overexpressing a polynucleotide encoding a protein which is involved in one or more proteinogenic amino acid biosynthetic pathways wherein said polynucleotide encodes a helper protein having an amino acid sequence as shown in SEQ ID NO: 2, 3, .... or 14 or a functional homologue thereof having at least 30% sequence identity thereto, host cell engineered to overexpress said helper protein

---

## ANNEX TO THE EUROPEAN SEARCH REPORT ON EUROPEAN PATENT APPLICATION NO.

EP 14 19 0469

This annex lists the patent family members relating to the patent documents cited in the above-mentioned European search report. The members are as contained in the European Patent Office EDP file on
The European Patent Office is in no way liable for these particulars which are merely given for the purpose of information.

15-01-2015

| Patent document cited in search report | Publication date | Patent family member(s) | Publication date |
|---|---|---|---|
| US 2012100621 A1 | 26-04-2012 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20040171154 A
- WO 9517413 A
- WO 9522625 A
- US 5223409 A
- WO 9206204 A
- US 20130244243 A
- EP 0472869 A
- US 4601893 A
- WO 9615246 A
- JP 10229891 A
- US 5364780 A
- WO 9706269 A
- WO 9706268 A
- WO 2008128701 A
- EP 0317254 A
- US 5624659 A
- US 6187287 B
- US 4546082 A
- WO 2008128701 A2


### Non-patent literature cited in the description


- **HEYLAND et al.** *Biotechnol Bioeng,* 2010, vol. 107 (2), 357-68
- **MARX et al.** *FEMS Yeast Res.,* 2009, vol. 9 (8), 1260-70
- **GUERRASIO et al.** *Anal Bioanal Chem.,* 2014, vol. 406 (3), 915-22
- **KLAVINS et al.** *Anal Bioanal Chem.,* 2013, vol. 405 (15), 5159-69
- **GUERRASIO et al.** *Anal Bioanal Chem.,* 2013, vol. 405 (15), 5133-46
- **NEUBAUER et al.** *J Sep Sci.,* 2012, vol. 35 (22), 3091-105
- **HEYLAND et al.** *Biotechnol Bioeng.,* 2011, vol. 108 (8), 1942-53
- **JORDA et al.** *Microb Cell Fact.,* 2012, vol. 11, 57
- **NOCON et al.** *Metab Eng.,* 2014
- **HAN et al.** *Appl Env Microbiol,* 2003, vol. 69 (10), 5772-5781
- **SCHERER ; DAVIS.** *Proc. Natl. Acad. Sci. USA,* 1979, vol. 76, 4949-4955
- **BARTON.** *Nucleic Acids Res.,* 1990, vol. 18, 7349-4966
- **STORICI.** *Nature Biotechnol.,* 2001, vol. 19, 773-776
- **KREN.** *Nat. Med.,* 1998, vol. 4, 285-290
- **CALISSANO ; MACINO.** *Fungal Genet. Newslett.,* 1996, vol. 43, 15-16
- **TIAN et al.** *Nature,* 2004, vol. 432, 1050-1054
- **REIDHAAR-OLSON ; SAUER.** *Science,* 1988, vol. 241, 53-57
- **BOWIE ; SAUER.** *Proc. Natl. Acad. Sci. USA,* 1989, vol. 86, 2152-2156
- **LOWMAN et al.** *Biochemistry,* 1991, vol. 30, 10832-10837
- **DERBYSHIRE et al.** *Gene,* 1986, vol. 46, 145
- **NER et al.** *DNA,* 1988, vol. 7, 127
- **NESS.** *Nature Biotechnology,* 1999, vol. 17, 893-896
- **ALTSCHUL et al.** *Nucleic Acids Res.,* 1997, vol. 25, 3389-3402
- **YAMANISHI et al.** *Biosci. Biotechnol. Biochem.,* 2011, vol. 75, 2234
- **MARTIN et al.** *Bio/Technology,* 1987, vol. 5, 137-146
- **GUERRERO et al.** *Gene,* 1994, vol. 138, 35-41
- **TSUCHIYA ; MORINAGA.** *Bio/Technology,* 1988, vol. 6, 428-430
- **EIKMANNS et al.** *Gene,* 1991, vol. 102, 93-98
- **SCHWARZER ; PÜHLER.** *Bio/Technology,* 1991, vol. 9, 84-87
- **REINSCHEID et al.** *Applied and Environmental Microbiology,* 1994, vol. 60, 126-132
- **LABARRE et al.** *Journal of Bacteriology,* 1993, vol. 175, 1001-1007
- **MALUMBRES et al.** *Gene,* 1993, vol. 134, 15-24
- **JENSEN ; HAMMER.** *Biotechnology and Bioengineering,* 1998, vol. 58, 191-195
- **MAKRIDES.** *Microbiological Reviews,* 1996, vol. 60, 512-538
- **KÜBERL et al.** *J Biotechnol.,* 2011, vol. 154 (4), 312-20
- **ZAMIR et al.** *Proc. NatL Acad. Sci. USA,* 1981, vol. 78 (6), 3496-3500
- **VOTH et al.** *Nucleic Acids Res.,* 15 June 2001, vol. 29 (12), e59
- **MIRISOLA et al.** *Yeast,* 2007, vol. 24, 761-766

- **TAXIS et al.** *BioTechniques,* 2006, vol. 40, 73-78
- **J. SAMBROOK et al.** Molecular Cloning: A Laboratory Manual. Cold Spring Harbor Laboratory Press, 2001
- Molecular Cloning: A Laboratory Manual. Cold Spring Harbor Laboratory, 1989, vol. 1-3
- Current Protocols in Molecular Biology. John Wiley & Sons, Inc, 1997
- **CREGG et al.** *Mol Biotechnol.,* 2000, vol. 16 (1), 23-52
- **MANIATIS et al.** *Science,* 1987, vol. 236, 1237-1245
- **GOEDDEL.** Gene Expression Technology: Methods in Enzymology. Academic Press, 1990, vol. 185
- **MARX, H. ; MATTANOVICH, D. ; SAUER, M.** *Microb Cell Fact,* 2008, vol. 7, 23
- **PAN et al.** *FEMS Yeast Res.,* May 2011, 292-8
- **CREGG et al.** *Mol. Cell. Biol.,* 1985, vol. 5, 3376-3385
- **DATSENKO et al.** *Proc. Natl. Acad. Sci. U.S.A.,* 2000, vol. 97 (12), 6640-6645
- **GATZ.** *Curr. Op. Biotech.,* 1996, vol. 7, 168
- **GATZ.** *Ann. Rev. Plant. Physiol. Plant Mol. Biol.,* 1997, vol. 48, 89
- **MATTANOVICH et al.** *Methods Mol. Biol.,* 2012, vol. 824, 329-58
- **ROMANOS et al.** *Yeast,* 1992, vol. 8, 423-488
- **RUDONI et al.** *The International Journal of Biochemistry and Cell Biology,* 2000, vol. 32 (2), 215-224
- **SCHMIDT.** *Appl. Microbiol. Biotechnol.,* 2004, vol. 65, 363-372
- **KIRK et al.** *Curr. Opin. Biotechnol.,* 2002, vol. 13, 345-351
- **KIRK et al.** *Current Opinion in Biotechnology,* 2002, vol. 13, 345-351
- **SCHMIDT.** *Appl Microbiol Biotechnol,* 2004, vol. 65, 363-372
- **BOER et al.** *Appl Microbiol Biotechnol,* 2000, vol. 77, 513-523
- Principles of Gene Manipulation and Genomics by Primrose and Twyman. Blackwell Publishing, 2006
- **LANDY.** *Ann. Rev. Biochem.,* 1989, vol. 58, 913-949
- **KEOWN et al.** *Processes in Enzymology,* 1990, vol. 185, 527-537
- **JULIUS et al.** *Cell,* 1983, vol. 32, 839
- Manual of Methods for General Bacteriology. American Society for Bacteriology, 1981
- **GASSER et al.** *Future Microbiol.,* 2013, vol. 8 (2), 191-208

SEQUENCE LISTING

<110> Boehringer Ingelheim RCV GmbH & Co KG et al.
VTU Technology GmbH
BIOMIN Holding GmbH
Sandoz AG
Lonza Ltd.

<120> Improved protein production in fungi or yeasts

<130> BOE15077EP-A

<160> 61

<170> PatentIn version 3.5

<210> 1
<211> 263
<212> PRT
<213> Pichia pastoris

<400> 1

```
Met Ser Ala Ser Thr Tyr Ser Phe Asp Gln Ala Met Asp Phe Asp Ile
1               5                   10                  15

Val Gln Ser Val Thr Ser Thr Gln Asp His Ile Pro Met Val Leu Gly
            20                  25                  30

Glu Ser Val Leu Arg Ser Phe Val Gly Asn Asp Ala Asn Lys Ala Pro
        35                  40                  45

Ala Ile Lys Gln Glu Tyr Glu Ala Leu Pro Leu Asn Ala Gln Ile Val
    50                  55                  60

Asn Pro Glu Leu Thr Pro Ser Val Gly Thr Ile Ser Pro Leu Glu Ile
65                  70                  75                  80

His Thr Ser Val Leu Asp Ser Val Leu Ser Thr Asp Phe Thr Asp Ala
                85                  90                  95

Asp Asn Ser Pro Met Phe Glu Ser Pro Glu Ser Glu Asp Pro Asn Asn
            100                 105                 110

Trp Val Ser Leu Phe Ala Asp Glu Thr Thr Leu Ala Thr Thr Pro Ala
        115                 120                 125

Val Ser Arg Ala Pro Ala Ala Ser Ala Ser Pro Val Val Pro Ser Leu
    130                 135                 140

Lys Thr Thr Ser Gly Asp Glu Gln Gln Leu Thr Val Lys Gln Phe Val
145                 150                 155                 160
```

Glu Tyr Pro Ser Ala Lys Asp Lys Leu Ser Pro Lys Ser Val Glu Lys
165 170 175

Lys Ile Ser Phe Lys Lys Asp His Leu Gly Val Val Gly Tyr Thr Arg
180 185 190

Arg Gln Arg Ser Ser Pro Leu Ala Pro Ile Val Val Lys Asp Asp Asp
195 200 205

Pro Val Ser Met Lys Arg Ala Arg Asn Thr Glu Ala Ala Arg Arg Ser
210 215 220

Arg Ala Lys Lys Met Lys Arg Met Ser Gln Leu Glu Asp Lys Val Glu
225 230 235 240

Glu Leu Leu Ile Cys Lys Ser Glu Leu Glu Ala Glu Val Glu Arg Leu
245 250 255

Lys Ser Leu Val Lys His Gln
260

<210> 2
<211> 510
<212> PRT
<213> Pichia pastoris

<400> 2

Met Arg Val Asn Lys Phe Ile Arg Pro Cys Arg His Met Ser Ser Thr
1 5 10 15

Thr Val Val Ser Leu Gly Leu Lys Ile Lys Pro Asp Phe Gln Pro Ala
20 25 30

Pro Lys Leu Thr Val Thr Asp Leu Asn Pro His Thr Val Asn Ala Lys
35 40 45

Tyr Ala Val Arg Gly Lys Ile Pro Thr Lys Ala Glu Leu Leu Arg Asn
50 55 60

Gln Leu Asn Asp Val Asp His Lys Leu Pro Phe Thr Lys Ile Ile Ser
65 70 75 80

Ala Asn Ile Gly Asn Pro Gln Gln Leu Asp Gln Lys Pro Leu Thr Phe
85 90 95

Tyr Arg Gln Ile Leu Ala Leu Leu Gln Tyr Pro Pro Leu Leu Asp Asp
100 105 110

```
Pro Arg Val Ala Ser Ile Phe Pro Lys Asp Ile Ile Glu Arg Ala Arg
        115                 120                 125

Ser Leu Leu Lys Gln Ile Gly Ser Val Gly Ala Tyr Ser Gln Ser Gln
    130                 135                 140

Gly Val Pro Ser Ile Arg Gln Ser Ile Ala Asp Phe Ile Ser Arg Arg
145                 150                 155                 160

Asp Gly His Pro Cys Ala Arg Lys Glu Leu Ile Tyr Leu Thr Thr Gly
                165                 170                 175

Ala Ser Thr Ala Val Thr Tyr Leu Leu Thr Leu Leu Gly Gln Asn Glu
            180                 185                 190

Lys Thr Gly Phe Leu Ile Pro Ile Pro Gln Tyr Pro Leu Tyr Thr Ala
        195                 200                 205

Thr Leu Thr Leu Asn Asn Arg Thr Ala Leu Pro Tyr Tyr Leu Asn Glu
    210                 215                 220

Glu Asp Asn Trp Ser Ile Glu Cys Asp Glu Ile Glu Ser Ile Ile Leu
225                 230                 235                 240

Asp Ser Lys Ala Lys Gly Ile Asp Pro Arg Cys Leu Ile Val Ile Asn
                245                 250                 255

Pro Gly Asn Pro Thr Gly Ala Ile Leu Ser Tyr Glu Ala Ile Glu Asp
            260                 265                 270

Ile Leu Asn Val Cys Ala Lys Tyr Gly Leu Val Val Ile Ala Asp Glu
        275                 280                 285

Val Tyr Gln Glu Asn Ile Phe Asp Gly Lys Phe Ile Ser Val Arg Lys
    290                 295                 300

Val Leu Leu Asp Leu Leu Gln Gly Pro Lys Ala Asp Leu Tyr Lys Asn
305                 310                 315                 320

Ile Gln Leu Ala Ser Leu His Ser Thr Ser Lys Gly Ile Ser Gly Glu
                325                 330                 335

Cys Gly Gln Arg Gly Gly Tyr Met Glu Leu Ile Gly Phe Asp Glu Ser
            340                 345                 350

Val Leu Glu Gln Ile Thr Lys Leu Ser Ser Ile Ser Leu Cys Pro Val
        355                 360                 365
```

Val Thr Gly Gln Ala Leu Val Glu Leu Met Ile Asn Pro Pro Lys Glu
    370                 375                 380

Gly Asp Glu Ser Tyr Lys Leu Tyr Tyr Asp Glu Thr Arg Ala Ile His
385                 390                 395                 400

Asp Ser Leu Ala Glu Arg Ala Thr Lys Leu Tyr Asp Ala Phe Asn Ser
                405                 410                 415

Met Glu Gly Val Val Cys Arg Lys Pro Gln Gly Ala Met Tyr Cys Phe
            420                 425                 430

Pro Lys Ile Thr Ile Pro Glu Lys Ala Ile Lys Thr Ala Lys Glu Leu
        435                 440                 445

Asp Phe Glu Pro Asp Glu Phe Tyr Cys Asn Glu Leu Leu Glu Thr Thr
    450                 455                 460

Gly Ile Cys Ala Val Pro Gly Ser Gly Phe Gly Gln Lys Pro Gly Thr
465                 470                 475                 480

Tyr His Ile Arg Thr Thr Phe Leu Ala Pro Gly Ser Glu Trp Ile Asn
                485                 490                 495

Asp Trp Val Val Phe His Lys Lys Phe Met Gln Lys Tyr Lys
            500                 505                 510

<210> 3
<211> 390
<212> PRT
<213> Pichia pastoris

<400> 3

Met Ala Lys Thr Leu Glu Arg Glu Glu Pro His Tyr Phe Gly Ala Gly
1               5                   10                  15

Pro Ala Leu Leu Pro Thr Ser Val Leu Gln Glu Ala Ala Tyr Asp Leu
            20                  25                  30

Val Asn Tyr Gln Gly Val Gly Ile Gly Ile Gly Glu Ile Ser His Arg
        35                  40                  45

Ser Lys Gln Ala Ser Glu Val Ile Asp Asn Thr Lys Ala Asn Leu Val
    50                  55                  60

Ser Leu Leu Asn Ile Pro Asp Thr His Glu Val Phe Phe Leu Gln Gly
65                  70                  75                  80

```
Gly Gly Thr Thr Gly Phe Ser Ser Ile Ala Ser Asn Leu Thr Ala Ser
                85                  90                  95

Phe Val Lys Lys Thr Gly Arg Lys Gly Arg Ala Ala Tyr Ala Ile Thr
            100                 105                 110

Gly Thr Trp Ser Lys Lys Ser Val Glu Glu Ala Gln Arg Leu Gly Leu
        115                 120                 125

Asp Val Asp Ile Val Leu Asp Ser Lys Lys Val Asp Gly Lys Phe Gly
    130                 135                 140

Ser Ile Pro Pro Val Asp Lys Trp Ser Ile Pro Thr Asp Leu Ser Asn
145                 150                 155                 160

Thr Ser Tyr Val Tyr Tyr Cys Asp Asn Glu Thr Val Asn Gly Val Glu
                165                 170                 175

Phe Lys Glu Phe Pro Phe Glu Ser Phe Pro Gly Val Glu Val Val Ala
            180                 185                 190

Asp Met Ser Ser Asn Ile Leu Ser Lys Arg Leu Asp Val Ser Lys Tyr
        195                 200                 205

Gly Leu Ile Met Ala Gly Ala Gln Lys Asn Ile Gly Leu Ala Gly Ile
    210                 215                 220

Thr Ile Tyr Ile Ile Lys Lys Ser Leu Leu Glu Gln Ala Asp Asp Ala
225                 230                 235                 240

Thr Leu Lys Ala Leu Glu Val Pro Leu Ser Pro Ile Ala Val His Tyr
                245                 250                 255

Pro Thr Val Val Lys Asn Asn Ser Ala Tyr Asn Thr Ile Pro Ile Phe
            260                 265                 270

Ala Val His Val Val Asn Leu Val Leu Arg Leu Leu Val Lys Asn Gly
        275                 280                 285

Gly Val Asp Ala Gln Gln Lys Ile Asn Glu Glu Lys Ala Ser Ile Leu
    290                 295                 300

Tyr Gly Ala Leu Glu Lys Phe Pro Gln Val Tyr Glu Leu Pro Ala Ser
305                 310                 315                 320

Thr Asp Ala Arg Ser His Met Asn Val Val Phe Thr Leu Asn Gly Asp
                325                 330                 335
```

Leu Asp Ala Glu Phe Leu Ala Gln Ala Ala Glu Arg Lys Leu Asn Gly
340 345 350

Leu Lys Gly His Arg Ser Val Gly Gly Met Arg Ala Ser Ile Tyr Asn
355 360 365

Ala Val Ser Leu His Ser Val Gln Glu Leu Thr Lys Phe Ile Ile Glu
370 375 380

Phe Ala Glu Ala His Ser
385 390

<210> 4
<211> 469
<212> PRT
<213> Pichia pastoris

<400> 4

Met Ala Ser Pro Gln Asp Ile Lys Gln Leu Ser Ser Gly Phe Asn Ala
1 5 10 15

Val Gly Leu Ser Thr Ser Pro Gly Val Val Ser Ala Ser Pro Asn Asp
20 25 30

Ser Tyr Leu Ser Gln Val Arg Gln Arg Ser Phe Ser Ala Ala Lys His
35 40 45

Ala Lys Pro Leu Lys Pro Phe Ser Thr Gly Asp Ile Lys Ile Leu Leu
50 55 60

Leu Glu Asn Val Asn Glu Thr Ala Arg Glu Ile Phe Ala Glu Gln Gly
65 70 75 80

Tyr Gln Val Glu Phe His Lys Ser Ser Leu Pro Glu Asp Glu Leu Ile
85 90 95

Glu Lys Ile Lys Asp Val His Ala Ile Gly Ile Arg Ser Lys Thr Lys
100 105 110

Leu Thr Glu Lys Val Leu Lys His Ala Lys Asn Leu Ile Ile Ile Gly
115 120 125

Cys Phe Cys Ile Gly Thr Asn Gln Val Asp Leu Glu Tyr Ala Ala Lys
130 135 140

Ser Gly Val Ala Val Phe Asn Ser Pro Phe Ser Asn Ser Arg Ser Val
145 150 155 160

Ala Glu Leu Val Ile Ala Glu Ile Ile Thr Leu Ala Arg Gln Leu Gly
165 170 175

Asp Arg Ser Leu Glu Met His Thr Gly Thr Trp Asn Lys Val Ser Asn
180 185 190

Lys Cys Trp Glu Ile Arg Gly Lys Thr Leu Gly Ile Val Gly Tyr Gly
195 200 205

His Ile Gly Ser Gln Leu Ser Val Leu Ala Glu Ala Phe Gly Met Asn
210 215 220

Val Ile Tyr Tyr Asp Val Leu Met Ile Met Ala Leu Gly Ser Ala Lys
225 230 235 240

Gln Val Arg Thr Leu Asn Asp Leu Leu Ala Gln Ala Asp Phe Val Thr
245 250 255

Leu His Val Pro Glu Asn Pro Glu Thr Lys Asn Leu Ile Ser Ser Pro
260 265 270

Gln Leu Ala Ala Met Lys Asp Gly Ala Tyr Leu Ile Asn Ala Ser Arg
275 280 285

Gly Thr Val Val Asp Ile Pro Ala Leu Val Glu Ala Met Lys Leu Gly
290 295 300

Lys Leu Ala Gly Ala Ala Leu Asp Val Tyr Pro Gln Glu Pro Gly Lys
305 310 315 320

Asn Gly Pro Asn Phe Thr Asn Glu Leu Asn Ser Trp Ala Thr Glu Leu
325 330 335

Thr Ser Leu Arg Asn Ile Ile Leu Thr Pro His Ile Gly Gly Ser Thr
340 345 350

Glu Glu Ala Gln Ser Ala Ile Gly Ile Glu Val Gly Thr Ala Leu Thr
355 360 365

Lys Tyr Ile Asn Glu Gly Thr Ser Thr Gly Ala Val Asn Phe Pro Glu
370 375 380

Val Ala Leu Arg Ala Leu Asp Leu Asp Gln Glu Asn Ser Val Arg Val
385 390 395 400

Leu Tyr Ile His Gln Asn Val Pro Gly Val Leu Lys Thr Val Asn Asn

405 410 415

Ile Leu Gly Asn His Asn Ile Glu Lys Gln Phe Ser Asp Ser Arg Gly
420 425 430

Asp Ile Ala Tyr Leu Met Ala Asp Ile Ser Asp Val Asp Ala Ser Asp
435 440 445

Ile Lys Ser Leu Tyr Glu Gln Leu Glu His Thr Pro Tyr Lys Ile Val
450 455 460

Thr Arg Leu Leu Tyr
465

<210> 5
<211> 367
<212> PRT
<213> Pichia pastoris

<400> 5

Met Phe Ile Gln Asn Asp His Val Gly Asp Arg Ser Arg Leu Glu Asp
1 5 10 15

Trp Arg Ile Arg Gly Tyr Asp Pro Leu Thr Pro Pro Asp Leu Leu Gln
20 25 30

His Glu Tyr Pro Leu Thr Pro Lys Ala Glu Glu Asn Ile Leu Lys Gly
35 40 45

Arg Asn Glu Ala Val Asp Ile Leu Lys Gly Lys Asp Asp Arg Leu Leu
50 55 60

Val Ile Val Gly Pro Cys Ser Ile His Asp Pro Lys Ala Ala Leu Asp
65 70 75 80

Tyr Cys Glu Arg Leu Ala Lys Phe Asn Glu Thr Ile Lys Gly Glu Leu
85 90 95

His Ile Ile Met Arg Ala Tyr Leu Glu Lys Pro Arg Thr Thr Val Gly
100 105 110

Trp Lys Gly Leu Ile Asn Asp Pro Asp Ile Asp Gly Ser Phe Gln Ile
115 120 125

Asn Lys Gly Leu Arg Ile Ser Arg Glu Leu Phe Val Lys Leu Thr Glu
130 135 140

Gln Ile Pro Ile Ala Gly Glu Met Leu Asp Thr Ile Ser Pro Gln Phe

```
145                 150                 155                 160

Leu Ser Asp Leu Phe Ser Leu Gly Ala Ile Gly Ala Arg Thr Thr Glu
                165                 170                 175

Ser Gln Leu His Arg Glu Leu Ala Ser Gly Leu Ser Phe Pro Val Gly
            180                 185                 190

Phe Lys Asn Gly Thr Asp Gly Gly Leu Thr Val Ala Ile Asp Ala Met
        195                 200                 205

Arg Ala Ala Ser His Pro His His Phe Leu Ser Val Thr Lys Pro Gly
    210                 215                 220

Val Val Ala Ile Val Gly Thr Glu Gly Asn Glu Asp Thr Phe Val Ile
225                 230                 235                 240

Leu Arg Gly Gly Lys Asn Gly Thr Asn Tyr Asp Glu Ala Ser Val Asp
                245                 250                 255

Ala Ala His Ala Gln Leu Glu Lys Thr Gly Ile Leu Gly Ser Gly Pro
            260                 265                 270

Ala Ile Met Val Asp Cys Ser His Gly Asn Ser Asn Lys Asp His Arg
        275                 280                 285

Asn Gln Pro Lys Val Ala Gln Val Val Ala Asp Gln Ile Arg Lys Gly
    290                 295                 300

Ser Arg Lys Ile Cys Gly Leu Met Ile Glu Ser Asn Ile Val Glu Gly
305                 310                 315                 320

Arg Gln Asp Val Pro Lys Glu Gly Lys Ser His Leu Arg Tyr Gly Cys
                325                 330                 335

Ser Ile Thr Asp Ala Cys Ile Ser Trp Glu Asp Thr Glu Lys Val Leu
            340                 345                 350

Arg Val Leu Ala Asp Ser Val Ile Glu Arg Arg Asn Leu Lys Asn
        355                 360                 365


<210>  6
<211>  282
<212>  PRT
<213>  Pichia pastoris

<400>  6

Met Glu Phe Lys Lys Pro Ala Thr Val Leu Asn Leu Ala Asn Ile Arg
```

```
1               5                   10                  15

Gln Ala Leu Val Arg Met Glu Asp Thr Val Val Phe Ser Phe Ile Glu
            20                  25                  30

Arg Ser Gln Phe Tyr Glu Ser Pro Ser Val Tyr Ala Pro Asn Lys Tyr
        35                  40                  45

Lys Ile Pro Asn Phe Ser Gly Ser Phe Leu Asp Trp Leu Leu Leu Gln
    50                  55                  60

Asn Glu Lys Thr His Ser Leu Val Arg Arg Tyr Glu Ser Pro Asp Glu
65                  70                  75                  80

Thr Pro Phe Phe Pro Asp Gln Leu Glu Glu Ser Phe Leu Pro Ser Leu
                85                  90                  95

Asn Tyr Pro Pro Ile Leu Ala Asp Tyr Ala Asp Glu Val Asn Val Asn
            100                 105                 110

Asp Glu Ile Arg Thr Val Tyr Ile Glu Lys Ile Val Pro Gln Ile Ala
        115                 120                 125

Ala Lys Lys Gly Asp Gln Asp Glu Asn Ile Gly Ser Thr Ala Cys Ala
    130                 135                 140

Asp Val Asp Cys Leu Gln Ala Leu Ser Arg Arg Val His Phe Gly Lys
145                 150                 155                 160

Phe Val Ala Glu Ala Lys Phe Gln Asn Glu Met Ala Arg Tyr Thr Asn
                165                 170                 175

Leu Ile Arg Asn Lys Asp Ile Lys Gly Ile Glu Glu Ala Ile Thr Asp
            180                 185                 190

Ser Ala Val Glu Ala Lys Ile Leu Glu Arg Leu Ile Ala Lys Gly His
        195                 200                 205

Ala Tyr Gly Thr Asp Pro Thr Leu Arg Tyr Ser Gln Asn Pro Gln Ser
    210                 215                 220

Lys Val Gln Pro Glu Ala Ile Ala Lys Ile Tyr Lys Glu Val Val Ile
225                 230                 235                 240

Pro Leu Thr Lys Lys Val Glu Val Glu Tyr Leu Leu Arg Arg Leu Gly
                245                 250                 255
```

```
Asp Asp Asp Glu Ala Glu Ala Gln Asp Pro Gln Arg Lys Thr Gln Leu
            260                 265                 270

Met Asn Arg Leu Leu Ser Asn Ser Leu Trp
        275                 280
```

<210> 7
<211> 398
<212> PRT
<213> Pichia pastoris

<400> 7

```
Met Ser Val Arg Asn Ala Thr Phe Arg Leu Ser Arg Ala Ala Ile Ala
1               5                   10                  15

Lys Arg Thr Leu Val Arg Ile Ala Ser Gln Ala Ser Leu Arg Pro Thr
            20                  25                  30

Thr Leu Asn Ser Ala Thr Gly Val Ala Arg Ser Gln Leu Thr Phe Ser
        35                  40                  45

Arg Gly Val Lys Thr Ile Asn Phe Gly Gly Val Glu Glu Val Val His
    50                  55                  60

Glu Arg Ser Asp Trp Pro Arg Glu Lys Leu Leu Glu Tyr Phe Lys Asn
65                  70                  75                  80

Asp Thr Leu Ala Leu Ile Gly Tyr Gly Ser Gln Gly Tyr Gly Gln Gly
                85                  90                  95

Leu Asn Leu Arg Asp Asn Gly Leu Asn Val Ile Ile Gly Val Arg Lys
            100                 105                 110

Asn Gly Ala Ser Trp Lys Ala Ala Ile Glu Asp Gly Trp Val Pro Gly
        115                 120                 125

Glu Asn Leu Phe Asp Val Thr Glu Ala Ile Lys Lys Gly Ser Ile Ile
    130                 135                 140

Met Asn Leu Leu Ser Asp Ala Ala Gln Ser Glu Thr Trp Ser Thr Val
145                 150                 155                 160

Lys Pro Leu Leu Thr Glu Gly Lys Thr Leu Tyr Phe Ser His Gly Phe
                165                 170                 175

Ser Pro Val Phe Ser Asn Leu Thr His Val Glu Pro Pro Ser Asn Ile
            180                 185                 190
```

```
Asp Val Ile Leu Ala Ala Pro Lys Gly Ser Gly Arg Thr Val Arg Ser
        195                 200                 205

Leu Phe Lys Glu Gly Arg Gly Ile Asn Ser Ser Tyr Ala Val Trp Asn
    210                 215                 220

Asp Val Thr Gly Lys Ala Glu Glu Lys Ala Ile Ala Leu Ala Val Ala
225                 230                 235                 240

Val Gly Ser Gly Tyr Val Tyr Gln Thr Thr Phe Glu Arg Glu Val Asn
                245                 250                 255

Ser Asp Leu Tyr Gly Glu Arg Gly Cys Leu Met Gly Gly Ile His Gly
            260                 265                 270

Met Phe Leu Ala Gln Tyr Glu Val Leu Arg Glu Asn Gly His Thr Pro
        275                 280                 285

Ser Glu Ala Phe Asn Glu Thr Val Glu Glu Ala Thr Gln Ser Leu Tyr
    290                 295                 300

Pro Leu Val Gly Lys Tyr Gly Met Asp Tyr Met Tyr Asp Ala Cys Ser
305                 310                 315                 320

Thr Thr Ala Arg Arg Gly Ala Leu Asp Trp Tyr Pro Ile Phe Lys Asp
                325                 330                 335

Ala Leu Lys Pro Val Phe Glu Asp Leu Tyr Asn Ser Val Lys Thr Gly
            340                 345                 350

Lys Glu Thr Gln Arg Ser Leu Asp Phe Asn Ser Gln Pro Asp Tyr Arg
        355                 360                 365

Glu Lys Leu Glu Lys Glu Leu Glu Ile Ile Arg Asn Met Glu Ile Trp
    370                 375                 380

Lys Val Gly Lys Glu Val Arg Lys Leu Arg Pro Glu Asn Asn
385                 390                 395

<210>  8
<211>  571
<212>  PRT
<213>  Pichia pastoris

<400>  8

Met Pro Met Leu Lys Asp Pro Ser Lys Lys Tyr Val Ala Phe Lys Pro
1               5                   10                  15
```

```
Leu Asn Leu Pro Asp Arg Gln Trp Pro Ser Lys Thr Leu Gln Ala Pro
            20                  25                  30

Pro Arg Trp Leu Ser Thr Asp Leu Arg Asp Gly Asn Gln Ser Leu Pro
        35                  40                  45

Asp Pro Met Ser Val Ala Glu Lys Lys Glu Tyr Phe Asn Lys Leu Val
    50                  55                  60

Gln Ile Gly Phe Lys Glu Ile Glu Val Ala Phe Pro Ser Ala Ser Gln
65                  70                  75                  80

Thr Asp Phe Asp Phe Thr Arg Tyr Ala Val Glu Asn Ala Pro Ala Asp
                85                  90                  95

Val Ala Ile Gln Val Leu Ser Pro Cys Arg Pro Glu Leu Ile Lys Arg
            100                 105                 110

Thr Val Glu Ser Leu Lys Gly Ala Lys Lys Ala Ile Val His Ile Tyr
        115                 120                 125

Leu Ala Thr Ser Asp Cys Phe Arg Asn Ile Ile Phe Gly Leu Ser Gln
    130                 135                 140

Glu Glu Ser Leu Glu Met Ala Val Asn Cys Thr Lys Leu Val Arg Glu
145                 150                 155                 160

Leu Thr Lys Asp Ala Pro Glu Met Gln Asp Thr Val Trp Gly Phe Glu
                165                 170                 175

Phe Ser Pro Glu Thr Phe Ser Asp Thr Asp Pro Asp Tyr Ala Leu Arg
            180                 185                 190

Val Cys Glu Ala Val Lys Ala Ala Trp Gly Pro Ser Glu Glu Asn Pro
        195                 200                 205

Ile Ile Phe Asn Leu Pro Ala Thr Val Glu Met Ser Thr Pro Asn Val
    210                 215                 220

Tyr Ala Asp Gln Ile Glu Tyr Phe Ser Arg Asn Ile Ser Glu Arg Lys
225                 230                 235                 240

Thr Val Ala Ile Ser Leu His Pro His Asn Asp Arg Gly Cys Ala Val
                245                 250                 255

Ala Ala Ala Glu Leu Gly Gln Met Ala Gly Ala Asp Arg Ile Glu Gly
            260                 265                 270
```

```
Cys Leu Phe Gly Asn Gly Glu Arg Thr Gly Asn Val Asp Leu Val Thr
        275                 280                 285

Leu Ala Leu Asn Leu Tyr Thr Gln Gly Val Tyr Pro His Leu Asp Phe
    290                 295                 300

Ser Asp Ile Thr Ser Val Ile Asp Ile Val Glu Arg Cys Asn Lys Ile
305                 310                 315                 320

Pro Val His Pro Arg Ala Pro Tyr Gly Gly Gln Leu Val Val Cys Ala
                325                 330                 335

Phe Ser Gly Ser His Gln Asp Ala Ile Lys Lys Gly Phe Lys Lys Gln
            340                 345                 350

Glu Glu Arg Gln Ala Lys Asp Pro Asn Ala Arg Trp Gln Leu Pro Tyr
        355                 360                 365

Leu Pro Leu Asp Pro Gln Asp Ile Gly Arg Thr Tyr Glu Ala Ile Ile
    370                 375                 380

Arg Val Asn Ser Gln Ser Gly Lys Gly Gly Ala Ser Trp Val Ile Leu
385                 390                 395                 400

Arg Asn Leu Glu Leu Asp Leu Pro Arg Ala Leu Gln Val Ser Phe Ser
                405                 410                 415

Lys Ile Val Gln Asn Glu Ser Glu Val Lys Gly Arg Glu Leu Tyr Asn
            420                 425                 430

Lys Glu Ile Thr Asn Leu Phe Lys Asn Ser Tyr Phe Val Asp Asp Asp
        435                 440                 445

Asp Thr Gln His Asp Leu Tyr Phe Gln Leu Val Asp Tyr Ser Ile Thr
    450                 455                 460

Asn Thr Ser Lys Ser Ala Arg Leu Ile Asn Val Glu Leu Gln Val Gly
465                 470                 475                 480

Lys Asp Thr Val Ser Leu Lys Gly Ile Gly Asn Gly Pro Ile Ser Ser
                485                 490                 495

Phe Ala Ser Ala Ile Ser Ser Tyr Leu Lys Lys Asp Val Leu Val Leu
            500                 505                 510

Asn Tyr His Glu His Ser Leu Gly Lys Asp Ser Asn Ser Lys Ala Ala
        515                 520                 525
```

Thr Tyr Leu His Cys Ala Ile Gly Asp Asp Cys Lys Val Trp Gly Val
530 535 540

Gly Ile His Glu Asp Val Ser Gln Ala Ser Phe Leu Ser Phe Ile Ser
545 550 555 560

Leu Leu Asn Ser Ala Arg Arg Asp Gly Lys Ile
565 570

<210> 9
<211> 631
<212> PRT
<213> Pichia pastoris

<400> 9

Met Ser Ser Leu Tyr Met Pro Ser Lys Ser Met Lys Arg Leu Thr Val
1 5 10 15

Ser Cys Arg Arg Phe Ile Ser Ser Ala Gln Pro Lys Ile Val Thr His
20 25 30

Lys Ile Leu Thr Pro Val Gly Thr Glu Ala Ala Ala Lys Val Asp Val
35 40 45

Pro Lys Thr Thr Arg Leu Ser Val Glu Gly Pro Ile Glu Cys His Leu
50 55 60

Gln Gly Phe Glu Leu Leu Asn Ser Pro Leu Phe Asn Lys Gly Ser Ala
65 70 75 80

Phe Thr Pro Glu Glu Arg Ala Ala Phe Gly Leu Glu Gly Leu Leu Pro
85 90 95

Pro Val Ala Asn Asp Leu Asn Glu Gln Ala Glu Arg Ala Tyr Lys Gln
100 105 110

Leu Ser Phe Leu Lys Thr Pro Leu Ala Lys Asn Asp Phe Cys Thr Ser
115 120 125

Met Arg Gln Gln Asn Lys Val Leu Phe Tyr Glu Leu Val Arg Arg His
130 135 140

Ile Arg Glu Leu Leu Pro Ile Ile Tyr Thr Pro Thr Glu Gly Asp Ala
145 150 155 160

Ile Ala Ala Tyr Ser His Arg Phe Arg Lys Pro Glu Gly Cys Phe Leu
165 170 175

```
Asp Val Thr Asp Pro Glu Ser Ile Glu Asp Arg Leu Ser Arg Phe Gly
            180                 185                 190

Glu Ser Lys Asp Val Asp Tyr Ile Val Val Ser Asp Gly Glu Gly Ile
        195                 200                 205

Leu Gly Ile Gly Asp Gln Gly Ile Gly Gly Val Arg Ile Ala Ile Ser
    210                 215                 220

Lys Leu Ala Leu Met Thr Leu Cys Gly Gly Ile His Pro Gly Arg Val
225                 230                 235                 240

Ile Pro Val Val Val Asp Ala Gly Thr Asn Asn Lys Ala Leu Leu Asp
                245                 250                 255

Asp Glu Leu Tyr Met Gly Ser Arg Phe Pro Arg Val Arg Gly Glu Glu
            260                 265                 270

Tyr Tyr Ser Phe Phe Asp Lys Phe Ile Glu Val Val Lys Arg Arg Phe
        275                 280                 285

Pro Ser Ala Val Leu His Phe Glu Asp Phe Gly Val Thr Asn Ala Lys
    290                 295                 300

Pro Leu Leu Asp Lys Tyr Arg Ser Val Leu Pro Cys Phe Asn Asp Asp
305                 310                 315                 320

Ile Gln Gly Thr Gly Ala Val Val Met Ala Ser Met Ala Ala Ala Leu
                325                 330                 335

Lys Leu Thr Asn Arg Asp Leu Leu Asp Ser Thr Ile Val Ile Tyr Gly
            340                 345                 350

Ala Gly Ser Ala Gly Leu Gly Ile Ala Phe Gln Ile Val Asn His Met
        355                 360                 365

Val Asn His Gly Ala Thr Lys Glu Glu Ala Tyr Ser Arg Ile His Leu
    370                 375                 380

Leu Asp Arg His Gly Leu Ile Leu Thr Asn Met Ser Asp Val Ser Asn
385                 390                 395                 400

Lys Asp Gln Met Leu Phe Ala Asp Asp Pro Ser Ser Trp Asp Gly Ile
                405                 410                 415

Asn Thr Lys Ser Leu Val Asp Val Ile Glu Lys Leu Lys Pro Thr Thr
```

420 425 430

Leu Ile Gly Cys Ser Thr Gln Ala Gly Ala Phe Thr Glu Glu Val Ile
435 440 445

Lys Thr Met Tyr Lys Tyr Asn Pro Arg Pro Ile Val Phe Pro Leu Ser
450 455 460

Asn Pro Thr Arg Leu His Glu Ala Val Pro Val Asp Val Met Arg Trp
465 470 475 480

Thr Asp Asp Asn Ala Leu Ile Ala Thr Gly Ser Pro Phe Lys Pro Val
485 490 495

Arg Gly His Val Ile Ser Glu Asn Asn Asn Cys Phe Ser Phe Pro Gly
500 505 510

Ile Gly Leu Gly Ala Val Leu Ser Arg Ala Thr Thr Ile Ser Asp Thr
515 520 525

Met Ile Ser Ala Ala Val Asp Glu Leu Ala Ser Gly Ser Pro Ala Phe
530 535 540

Lys Asp Pro Lys Ala Gly Leu Leu Ala Pro Val Glu Val Ile Asn Glu
545 550 555 560

Thr Ser Ala Lys Val Ala Ala Ala Leu Ile Leu Gln Ser Ile Lys Glu
565 570 575

Gly Thr Ala Arg Val Glu Glu Glu Leu Ser Pro Asn Gly Asp Lys Val
580 585 590

Ser Val Pro Arg Asp Phe Glu Gly Cys Val Ala Trp Val Lys Ser Gln
595 600 605

Met Trp Arg Pro Glu Tyr Arg Pro Met Val Lys Val Glu Arg Val Pro
610 615 620

Glu Ile His Thr His Gln Ser
625 630

<210> 10
<211> 451
<212> PRT
<213> Pichia pastoris

<400> 10

Met Gly Glu Asn Cys Tyr Thr Ile Val Val Lys Leu Gly Thr Ser Ser

```
1               5                   10                  15

Val Val Asp Glu Glu Thr Arg Glu Pro Arg Ile Ala Thr Met Ser Arg
            20                  25                  30

Ile Val Glu Thr Leu Val Lys Leu Arg Arg Lys Gly His Arg Ile Val
        35                  40                  45

Ile Val Ser Ser Gly Ala Ile Ala Val Gly Met Lys Arg Val Asn Met
    50                  55                  60

Lys Arg Lys Pro Lys Lys Leu Gln Gln Val Gln Ala Leu Ala Ala Ile
65                  70                  75                  80

Gly Gln Gly Arg Leu Ile Gly Leu Trp Asp Asp Leu Phe Arg Gln Leu
                85                  90                  95

Asn Gln Pro Ile Ala Gln Ile Leu Leu Thr Arg Thr Asp Leu Val Asp
            100                 105                 110

Tyr Thr Gln Phe Lys Asn Ala Glu Asn Thr Leu Glu Gln Leu Ile Lys
        115                 120                 125

Met Gly Ile Ile Pro Ile Val Asn Glu Asn Asp Thr Leu Ser Ile Gln
    130                 135                 140

Glu Ile Lys Phe Gly Asp Asn Asp Thr Leu Ser Ala Ile Thr Ala Gly
145                 150                 155                 160

Met Cys His Ala Asp Tyr Leu Phe Leu Val Thr Asp Val Asp Cys Leu
                165                 170                 175

Tyr Thr Asp Asn Pro Arg Thr Asn Pro Asp Ala Glu Pro Ile Val Leu
            180                 185                 190

Val Arg Asn Met Arg Asn Leu Asn Val Asn Thr Glu Ser Gly Gly Ser
        195                 200                 205

Ala Val Gly Thr Gly Gly Met Thr Thr Lys Leu Ile Ala Ala Asp Leu
    210                 215                 220

Gly Val Ser Ala Gly Val Thr Thr Ile Ile Cys Lys Ser Glu His Pro
225                 230                 235                 240

Glu Gln Ile Leu Asp Ile Val Glu Tyr Ser Ile Arg Ala Asp Arg Val
                245                 250                 255
```

```
Glu Asn Glu Ala Lys Tyr Leu Val Ile Asn Glu Glu Glu Thr Val Glu
            260                 265                 270

Gln Phe Gln Glu Ile Asn Arg Ser Glu Leu Arg Glu Leu Asn Lys Leu
        275                 280                 285

Asp Ile Pro Leu His Thr Arg Phe Val Gly His Ser Phe Asn Ala Val
    290                 295                 300

Asn Asn Lys Glu Phe Trp Leu Leu His Gly Leu Lys Ala Asn Gly Ala
305                 310                 315                 320

Ile Ile Ile Asp Pro Gly Cys Tyr Lys Ala Ile Thr Arg Lys Asn Lys
                325                 330                 335

Ala Gly Ile Leu Pro Ala Gly Ile Ile Ser Val Glu Gly Asn Phe His
            340                 345                 350

Glu Tyr Glu Cys Val Asp Val Lys Val Gly Leu Arg Asp Pro Asp Asp
        355                 360                 365

Pro His Ser Leu Asp Pro Asn Glu Glu Leu Tyr Val Val Gly Arg Ala
    370                 375                 380

Arg Cys Asn Tyr Pro Ser Asn Gln Ile Asn Lys Ile Lys Gly Leu Gln
385                 390                 395                 400

Ser Ser Gln Ile Glu Gln Val Leu Gly Tyr Ala Asp Gly Glu Tyr Val
                405                 410                 415

Val His Arg Asp Asn Leu Ala Phe Pro Val Phe Ala Asp Pro Glu Leu
            420                 425                 430

Leu Asp Val Val Glu Ser Thr Leu Ser Glu Gln Glu Arg Glu Ser Lys
        435                 440                 445

Pro Asn Lys
    450


<210>  11
<211>  274
<212>  PRT
<213>  Pichia pastoris

<400>  11

Met Ser Ser Ile Gly Asn Glu Tyr Thr Ile Thr Ile Leu Gly Cys Gly
1               5                   10                  15
```

```
Val Met Gly Thr Ala Val Leu Ser Ala Ile Leu Asn Ser Glu Phe Ser
            20                  25                  30

Pro Tyr Pro Ser Arg Ile Ile Cys Cys Thr Gly Ser Gln Lys Ser Ala
        35                  40                  45

Glu Lys Leu Ala Ser Thr Tyr Ser Lys Ile Glu Thr Ser Tyr Gly Ser
    50                  55                  60

Glu Gln Asn Ile Arg Ala Val Lys Glu Ser Asp Ile Ile Ile Leu Gly
65                  70                  75                  80

Cys Lys Pro Phe Tyr Cys Glu Gln Ile Met Glu Gln Val Lys Glu Gly
                85                  90                  95

Leu His Asp Gln Leu Leu Ile Ser Leu Leu Ala Gly Trp Thr Leu Thr
            100                 105                 110

Gln Leu Glu Arg Tyr Ser Lys Tyr Val Ala Arg Val Met Thr Asn Thr
        115                 120                 125

Pro Ala Lys Phe Gly Cys Gly Met Ala Cys Val Ser Leu Ser Ser His
    130                 135                 140

Ala Glu Gln Tyr Glu Asp Leu Val Leu Lys Leu Val Gly Pro Val Gly
145                 150                 155                 160

Lys Ala Ile Arg Leu Pro Glu Lys Asn Met Asp Ala Ala Thr Ala Leu
                165                 170                 175

Val Gly Ser Gly Pro Ala Phe Cys Leu Leu Met Met Glu Ser Leu Ile
            180                 185                 190

Asp Gly Ala Val Gln Lys Gly Leu Pro Phe Ala Ile Ala Lys Asp Ala
        195                 200                 205

Ala Ala Lys Val Met Glu Gly Thr Ala Arg Met Val Leu Glu Thr Gly
    210                 215                 220

Glu His Pro Ala Ala Leu Lys Ser Ala Val Cys Thr Pro Gly Gly Thr
225                 230                 235                 240

Thr Ile Gly Gly Leu Met Ala Met Glu Asp Arg Gly Val Arg Ser Gly
                245                 250                 255

Ile Ala Arg Gly Val Glu Glu Ala Ala Asn Ile Ala Thr Ser Leu Gly
            260                 265                 270
```

Lys Lys

<210> 12
<211> 442
<212> PRT
<213> Pichia pastoris

<400> 12

Met Pro Ser His Phe Asp Thr Leu Gln Leu His Ala Gly Gln Thr Ala
1               5                   10                  15

Glu Ala Pro His Asn Ala Arg Ala Val Pro Ile Tyr Ala Thr Ser Ser
            20                  25                  30

Tyr Val Phe Arg Asp Ser Glu His Gly Ala Lys Leu Phe Gly Leu Glu
        35                  40                  45

Glu Pro Gly Tyr Ile Tyr Ser Arg Leu Met Asn Pro Thr Gln Asn Val
    50                  55                  60

Phe Glu Glu Arg Ile Ala Ala Leu Glu Gly Gly Ala Ala Ala Leu Ala
65                  70                  75                  80

Val Gly Ser Gly Gln Ala Ala Gln Phe Leu Ala Ile Ala Gly Leu Ala
                85                  90                  95

His Thr Gly Asp Asn Val Ile Ser Thr Ser Phe Leu Tyr Gly Gly Thr
            100                 105                 110

Tyr Asn Gln Phe Lys Val Ala Phe Lys Arg Leu Gly Ile Glu Ser Arg
        115                 120                 125

Phe Val His Gly Asp Asp Pro Ala Glu Phe Glu Arg Leu Ile Asp Asp
    130                 135                 140

Lys Thr Lys Ala Ile Tyr Val Glu Ser Ile Gly Asn Pro Lys Tyr Asn
145                 150                 155                 160

Ile Pro Asp Phe Glu Ala Leu Ala Glu Leu Ala His Lys His Gly Ile
                165                 170                 175

Pro Leu Val Val Asp Asn Thr Phe Gly Ala Gly Gly Tyr Tyr Val Arg
            180                 185                 190

Pro Ile Glu Leu Gly Ala Asp Ile Val Thr His Ser Thr Thr Lys Trp
        195                 200                 205

```
Ile Asn Gly His Gly Asn Thr Ile Gly Gly Val Val Val Asp Ser Gly
    210                 215                 220

Lys Phe Pro Trp Lys Asp Tyr Pro Glu Lys Tyr Pro Gln Phe Ser Lys
225                 230                 235                 240

Pro Ser Glu Gly Tyr His Gly Leu Ile Leu Asn Asp Ala Phe Gly Pro
                245                 250                 255

Ala Ala Phe Ile Gly His Leu Arg Thr Glu Leu Leu Arg Asp Leu Gly
            260                 265                 270

Pro Ala Ser Ser Pro Phe Gly Asn Phe Leu Asn Ile Ile Gly Leu Glu
        275                 280                 285

Thr Leu Ser Leu Arg Ala Glu Arg His Ala Glu Asn Ala Leu Lys Leu
    290                 295                 300

Ala Lys Tyr Leu Glu Thr Ser Pro Tyr Val Ser Trp Val Ser Tyr Pro
305                 310                 315                 320

Gly Leu Glu Ser His Asp Tyr His Glu Ala Ala Lys Lys Tyr Leu Lys
                325                 330                 335

Asn Gly Phe Gly Ala Val Leu Ser Phe Gly Val Lys Asp His Gly Lys
            340                 345                 350

Pro Ala Leu Thr Pro Phe Glu Glu Ala Gly Pro Lys Val Val Asp Ser
        355                 360                 365

Leu Lys Val Phe Ser Asn Leu Ala Asn Val Gly Asp Ser Lys Ser Leu
    370                 375                 380

Ile Ile Ala Pro Tyr Tyr Thr Thr His Gln Gln Leu Ser His Glu Glu
385                 390                 395                 400

Lys Leu Ala Ser Gly Val Thr Lys Asp Ser Ile Arg Val Ser Val Gly
                405                 410                 415

Thr Glu Phe Ile Asp Asp Leu Ile Ala Asp Leu Glu Gln Ala Phe Ala
            420                 425                 430

Leu Val Tyr Glu Glu Ala Asn Thr Lys Leu
        435                 440
```

<210> 13
<211> 501

<212> PRT
<213> Pichia pastoris

<400> 13

```
Met Ser Asp Asn Asn Gln Pro Leu Pro Pro Val Ser Asn Ser Ile Leu
1               5                   10                  15

Glu His Ile Gly Lys Thr Pro Leu Val Lys Leu Asn Arg Ile Pro Lys
            20                  25                  30

Ile Leu Asn Leu Lys Pro Gln Val Tyr Ala Lys Val Glu Leu Phe Asn
        35                  40                  45

Ser Gly Gly Ser Ile Lys Asp Arg Ile Ala Leu Arg Met Val Glu Gln
    50                  55                  60

Ala Glu Lys Glu Gly Arg Ile Lys Pro Gly Tyr Thr Leu Ile Glu Pro
65                  70                  75                  80

Thr Ser Gly Asn Thr Gly Ile Gly Leu Ala Leu Val Gly Ala Val Lys
                85                  90                  95

Gly Tyr Arg Thr Ile Ile Thr Leu Pro Glu Lys Met Ser Asn Glu Lys
            100                 105                 110

Val Ala Val Leu Lys Ala Leu Gly Ala Glu Ile Ile Arg Thr Pro Thr
        115                 120                 125

Glu Ala Ala Trp Asp Ser Pro Glu Ser His Ile Gly Val Ala Arg Arg
    130                 135                 140

Leu Glu Lys Glu Ile Pro Asn Ala Val Ile Leu Asp Gln Tyr Gly Asn
145                 150                 155                 160

Val Asn Asn Pro Asp Ala His Phe Tyr Thr Thr Gly Ala Glu Ile Trp
                165                 170                 175

Asp Gln Thr Asn Gly Lys Val Thr His Val Val Ala Gly Ala Gly Thr
            180                 185                 190

Gly Gly Thr Ile Thr Gly Ile Ala Lys Phe Leu Lys Ser Lys Asn Pro
        195                 200                 205

Asn Ile Gln Ile Ile Gly Ala Asp Pro His Gly Ser Ile Leu Ala Leu
    210                 215                 220

Pro Glu Ser Leu Asn Thr Ser Asn Glu Gln Tyr Lys Val Glu Gly Ile
225                 230                 235                 240
```

Gly Tyr Asp Phe Ile Pro Glu Val Leu Asp Arg Glu Ile Val Asp Thr
245 250 255

Trp Tyr Lys Thr Asp Asp Lys Asp Ser Phe Lys Leu Ala Arg Gln Leu
260 265 270

Ile Ser Glu Glu Gly Ile Leu Val Gly Gly Ser Ser Gly Ser Ala Leu
275 280 285

Ser Ala Ala Val Lys Ile Ile Asn Asp Ala Lys Leu Asp Glu Ser Ala
290 295 300

Val Val Val Val Val Cys Pro Asp Ser Ile Arg Ser Tyr Leu Thr Lys
305 310 315 320

Phe Ala Asp Asp Asp Trp Met Lys Leu Asn Gly Phe Thr Glu Asp Glu
325 330 335

Pro Ala Pro Leu Lys Arg Lys Ser Ser Phe Ser Lys Asp Thr Leu Ser
340 345 350

Ser Tyr Thr Ile Lys Asp Leu Ala Leu Lys Pro Val Val Ser Val Thr
355 360 365

Ile Asp Asp Ser Ile Glu Ser Thr Ile Asn Ile Leu Gln Thr Lys Gly
370 375 380

Phe Asp Gln Leu Pro Val Leu Lys Asn Asp Lys Leu Ala Gly Ile Val
385 390 395 400

Thr Leu Ser Gln Leu Leu Arg Leu Leu Ser Asn Lys Lys Ile His Leu
405 410 415

Thr Ser Lys Val Glu Ser Ala Tyr Phe Asp Phe Ser Lys Leu Glu Asn
420 425 430

Phe Glu Lys Ser Tyr Asn Ile Asn Lys Glu Ser Thr Asn Lys Arg Arg
435 440 445

Glu Tyr Gln Lys Ile Thr Thr Ser Thr Thr Leu Ala Lys Leu Asn Lys
450 455 460

Phe Phe Glu Thr Ser Ser Ala Ala Ile Val Glu Asn Asp Lys Gly Gln
465 470 475 480

Pro Ile His Ile Val Ser Lys Val Asp Leu Leu Ser Phe Leu Ala Ser

```
                485                 490                 495

Lys Gly Glu Phe Asn
            500

<210>  14
<211>  356
<212>  PRT
<213>  Pichia pastoris

<400>  14

Met Lys Lys Ala Gly Val Leu Gly Ala Thr Gly Ser Val Gly Gln Arg
1               5                   10                  15

Phe Ile Leu Leu Leu Ser Asp His Pro Glu Phe Glu Leu Ser Val Leu
            20                  25                  30

Gly Ala Ser Ser Arg Ser Ala Gly Lys Arg Tyr Glu Asp Ala Val Val
        35                  40                  45

Trp Lys Gln Thr Asp Leu Leu Pro Glu Ser Ala Arg Asn Ile Ile Val
    50                  55                  60

Gln Glu Cys Lys Ala Glu Ala Phe Lys Asp Cys Asp Ile Ile Phe Ser
65                  70                  75                  80

Gly Leu Asp Ala Asp Tyr Ala Gly Glu Ile Glu Lys Glu Phe Val Ser
                85                  90                  95

Ser Gly Leu Ala Val Val Ser Asn Ala Lys Asn Tyr Arg Arg Glu Ala
            100                 105                 110

Asp Val Pro Leu Ile Val Pro Ile Val Asn Pro Glu His Leu Glu Ile
        115                 120                 125

Val Glu Gln Lys Val Lys Ser Gly Gln Lys Gly Phe Gln Ile Cys Ile
    130                 135                 140

Ser Asn Cys Ser Thr Ala Gly Leu Val Ala Pro Leu Lys Pro Leu Val
145                 150                 155                 160

Glu Lys Phe Gly Pro Ile Asp Leu Leu Thr Thr Thr Thr Leu Gln Ala
                165                 170                 175

Ile Ser Gly Ala Gly Phe Ser Pro Gly Val Pro Gly Ile Asp Ile Leu
            180                 185                 190

Asp Asn Ile Val Pro Phe Ile Ser Gly Glu Glu Glu Lys Met Glu Trp
```

```
        195                 200                 205

Glu Thr Arg Lys Ile Leu Gly Gly Leu Ser Glu Asp Lys Met Glu Cys
    210                 215                 220

Arg Leu Val Ser Glu Asp Ala Met Lys Val Ser Ala Gln Cys Asn Arg
225                 230                 235                 240

Val Pro Val Ile Asp Gly His Thr Glu Cys Ile Ser Leu Arg Phe Glu
                245                 250                 255

Arg Arg Pro Pro Pro Ser Val Glu Ala Val Lys Gln Cys Leu Arg Asp
            260                 265                 270

Tyr Val Cys Glu Ala Ser Lys Leu Gly Cys Pro Ser Ala Pro Lys Asn
        275                 280                 285

Thr Ile His Val Leu Glu Gln Asn Asp Arg Pro Gln Pro Arg Leu Asp
    290                 295                 300

Arg Val Arg Asp Asn Gly Phe Gly Val Ser Val Gly Arg Ile Arg Glu
305                 310                 315                 320

Asp Pro Val Leu Asp Phe Lys Met Val Val Leu Ser His Asn Thr Ile
                325                 330                 335

Ile Gly Ala Ala Gly Ser Gly Val Leu Ile Gly Glu Ile Leu Leu Lys
            340                 345                 350

Lys Gly Leu Ile
        355

<210>  15
<211>  792
<212>  DNA
<213>  Pichia pastoris

<400>  15
atgtctgcaa gtacttacag ttttgaccaa gcaatggact ttgacatcgt tcagtccgtg        60

acgtcgaccc aggaccatat ccccatggtt cttggcgagt cagtgcttcg ttcttttgtt       120

ggaaatgatg ccaacaaggc tcctgctatc aagcaggaat atgaggcctt gccgctaaac       180

gctcaaatcg tcaatcctga gctgacacca tccgtcggaa ctatttctcc tttggagatc       240

catacttccg ttttggattc tgtattgtcg acagacttta ctgatgctga caactccccc       300

atgtttgaat ctccagagtc tgaagatcca aacaactggg tttcgttgtt tgcagatgaa       360

actactcttg ctaccactcc agccgtttct cgtgcaccag cagcctctgc ctctccagta       420
```

gtcccttcgt tgaagaccac ctctggagac gagcaacagc tgactgttaa acaattcgtc 480

gagtatcctt ccgctaagga taagctttct cccaagtcag tggagaaaaa gatttctttc 540

aagaaggacc acttaggtgt tgttggctac actagaagac agcgttcctc tcccttggct 600

ccaatcgtgg tcaaggatga cgatcctgtt tctatgaagc gtgcccgtaa cacggaagcc 660

gctcgtcgtt ccagggctaa gaagatgaag agaatgagtc aactggaaga caaagttgag 720

gagttactga tttgcaagag cgagttggaa gctgaggttg agcgtttaaa gagtttagtg 780

aaacatcagt ga 792

<210> 16
<211> 1533
<212> DNA
<213> Pichia pastoris

<400> 16
atgagagtta ataaatttat tcgtccttgt cgtcatatgt cttcgacaac tgttgtttcg 60

ttggggctga agatcaaacc agattttcag cccgcaccaa agttgaccgt cactgatttg 120

aaccctcata cagtcaatgc caaatatgcc gtaagaggaa aaattcctac taaggcagaa 180

cttttaagaa atcagcttaa cgatgtagac cataaattgc ctttcaccaa aattattagt 240

gccaacattg gtaatccaca gcaattagac caaaagccat taacattcta cagacaaatc 300

ctggccctgc tgcagtaccc gcccttgttg gacgatcctc gtgttgcttc aattttcccc 360

aaggatatta ttgagcgtgc tagaagctta ttgaaacaaa ttggttccgt gggtgcctac 420

tcccaatctc aaggtgtccc ttctatcaga cagtctatcg cggacttcat cagcagaaga 480

gacggccatc cttgtgccag gaaagagcta atatatctta ccaccggtgc atccaccgct 540

gtcacttatc tgttaactct actgggacaa aatgagaaga ccggtttctt gattccgatt 600

cctcaatatc ctctctatac tgccacactg accctcaaca accgtaccgc attgccatac 660

tacttgaacg aggaagacaa ctggtccatc gagtgcgatg aaatagagtc cattattctg 720

gattccaaag ctaaaggaat cgaccctcgt tgcctgatag taatcaatcc tggaaacccc 780

actggtgcca ttctttccta tgaagccatt gaggatatct tgaatgtttg tgccaagtat 840

ggtttggttg ttattgccga tgaagtgtat caggaaaaca tttttgacgg taagttcatt 900

tccgttagaa aagtgttgct agatctatta caaggcccaa aggccgactt gtataagaac 960

atccagttgg catcgttgca ttcgacctcc aagggtattt ctggagaatg tgggcaaaga 1020

ggtggctata tggaactcat aggtttcgac gaatctgtgt tagaacagat caccaagctt 1080

tcttctattt ctctgtgccc agtggtgaca ggccaagctc tggtagaact aatgattaac 1140

ccgcccaagg aaggagatga atcttacaaa ctgtattacg atgagacccg tgctattcac 1200

gattccctag ccgaaagggc cacgaaattg tacgatgcgt tcaacagcat ggagggtgtt 1260

gtttgccgca aaccccaagg ggccatgtat tgtttcccca aaatcactat tcctgagaag 1320

gcgatcaaga ccgccaagga gcttgacttt gaacctgatg aattttactg caatgaattg 1380

ttagagacta ctggtatttg tgctgttcca ggctccggat ttggacagaa gccgggaaca 1440

tatcacatta gaacaacttt ccttgctcca gggtccgagt ggatcaacga ctgggtcgtc 1500

ttccacaaga agttcatgca aaagtataaa tga 1533

<210> 17
<211> 1173
<212> DNA
<213> Pichia pastoris

<400> 17
atggcaaaaa ctttggaaag agaagaacca cattattttg gggctgggcc agcgctactg 60

cctacttcag tattgcaaga ggctgcctat gacttggtca actaccaggg tgttggtata 120

ggtattggtg aaatttctca tcgttctaag caagctagtg aagtcattga taacacgaag 180

gccaatctag ttagtttgtt gaacatccct gatacacatg aggttttttt cttacaaggg 240

ggaggaacaa caggattttc ttctattgct tccaacttga ctgcctcctt tgttaagaaa 300

actggaagga agggccgtgc tgcttatgca atcactggaa cttggtctaa gaaatctgtt 360

gaagaagccc agcgtttggg tttggacgtt gacattgttt tggatagtaa aaaagttgat 420

ggaaaatttg gatccatacc cccagttgac aaatggagca tccccactga tctatcaaac 480

acttcttatg tgtattattg tgataatgaa actgtgaacg gtgttgagtt caaggagttc 540

ccatttgaaa gttttcctgg tgttgaagta gttgcagata tgtcatccaa catcttatca 600

aagaggctgg atgtcagtaa atatggattg ataatggccg gtgctcaaaa aaatattgga 660

ttagctggaa tcacgatcta tattatcaaa aagtcgttgc ttgagcaagc tgatgatgcc 720

actttgaaag ctcttgaagt gcctctttca ccaattgcgg ttcattatcc tacagtagtc 780

aagaacaact ccgcttacaa cacaattccc attttcgcag ttcatgtcgt caatttggtc 840

ctcaggctat tagtaaagaa cggtggtgtc gatgcccaac agaagataaa cgaggagaag 900

gcttccatat tgtatggtgc gttggagaag ttcccacaag tttatgagtt gcctgcttcc 960

accgatgctc gttctcatat gaatgtggtt ttcacattga atggtgactt ggatgctgaa 1020

ttcttagccc aagctgcaga aaggaagcta aatggattga agggtcaccg ctcggttggt 1080

ggaatgagag cctccattta caatgctgtt tccttgcaca gtgtccaaga gttaacgaaa 1140

ttcatcatcg aatttgctga agctcattct tga 1173

<210> 18
<211> 1410
<212> DNA
<213> Pichia pastoris

<400> 18
atggcttctc cccaagatat taagcagtta agcagtggct tcaatgctgt cggtttgtcc 60

acttcccctg gagttgtttc tgcttcccct aatgactcct acctgtccca ggtcagacag 120

cgtagtttct ctgctgccaa acacgctaaa ccattgaaac cattctccac tggagatatt 180

aaaattttgc tgctagaaaa tgttaacgaa accgccagag agatctttgc tgaacaggga 240

tatcaagtgg agttccacaa gtcttcgtta ccagaggatg agctgattga aaaaatcaaa 300

gatgttcatg ccattggtat caggtcaaaa actaagttaa ccgaaaaggt tttgaaacac 360

gctaaaaact tgataatcat cgggtgtttc tgtattggta ccaatcaagt cgacttggaa 420

tatgcggcaa aatcgggtgt tgctgtgttc aattccccat tttcaaactc cagatccgtt 480

gctgaattgg ttattgccga gattattact cttgccaggc aactaggaga tagatctttg 540

gagatgcaca ccggaacctg gaataaagtt tccaacaaat gttgggagat cagaggtaaa 600

acgcttggaa ttgtgggtta cgggcacatt ggttcccagt tgtccgttct ggctgaagcc 660

tttggtatga acgttatcta ctacgatgtc ttgatgatca tggctctggg ttctgctaaa 720

caggtccgta cattgaatga cctccttgcc caagctgatt ttgtcacttt gcatgttccc 780

gaaaatccgg agactaagaa ccttatcagc tctcctcaac tggctgctat gaaggatgga 840

gcttacctga tcaatgcttc aagaggaacc gtggtggaca ttcctgcctt ggttgaagcg 900

atgaaactag gtaagctggc gggtgctgct ttggatgttt atccacaaga accaggtaag 960

aatggtccaa acttcaccaa cgagttgaac tcttgggcta ctgagctaac ttctttgaga 1020

aacattattc tgaccccaca tattggtggt tctactgaag aggcccaatc tgcaattgga 1080

attgaagtag gaactgctct cacaaagtac attaatgagg gaacttctac aggtgctgtc 1140

aacttcccag aagttgcttt acgtgctcta gatcttgacc aagaaaactc tgttagagtg 1200

ctttacattc atcagaacgt gcctggtgtg ttgaaaactg tgaacaacat tttgggtaac 1260

cataacattg aaaagcagtt ctctgactcc agaggagaca ttgcctactt gatggccgat 1320

atttctgatg tggatgcttc agacatcaag tctttgtacg aacaactgga gcacacccct 1380

tacaaaatcg tcacccgtct gctatactag 1410

<210> 19
<211> 1104
<212> DNA
<213> Pichia pastoris

<400> 19
atgttcattc aaaacgatca tgtcggtgac agatcccgct tagaagactg gagaatacgt 60

ggatacgacc ctctgacgcc ccccgacctg ttgcaacatg agtatccact taccccaaaa 120

gcagaggaga acattctcaa gggaagaaac gaagctgttg atatcctgaa aggtaaggat 180

gacaggctat tagtcattgt cggaccttgt tccatccatg atcctaaggc agctttagac 240

tactgcgaga gattagccaa gtttaacgag actatcaaag gtgagcttca tatcataatg 300

agagcttatt tggagaaacc aagaaccact gttggttgga aaggtctgat aaatgaccca 360

gacatcgatg gttcgtttca aatcaataaa ggtcttcgta tctcgaggga gcttttcgtt 420

aaacttactg aacagattcc tattgctggt gagatgctgg acactatctc ccctcaattc 480

ctgtctgatc tcttctcctt aggagcgatt ggcgccagaa ccacagagtc tcaactgcac 540

agagaacttg cttccggttt gtccttcccc gtgggattca agaatggtac agacggtggg 600

ctcacagttg ctatagatgc catgagagct gcttcgcatc ctcaccattt cctgtctgtc 660

acaaaaccag gagttgtggc tattgtcggt accgaaggaa atgaagacac ctttgttatc 720

ctaagaggtg gtaagaacgg tacaaactac gacgaggcct ctgttgacgc tgcccacgca 780

cagttagaga agaccggtat cctgggctct ggtcctgcca tcatggtgga ctgctctcat 840

ggaaattcca acaaagacca ccgtaaccaa cctaaggtag cccaagttgt tgctgaccaa 900

atccgtaaag gatcccgaaa aatatgtggc ctcatgattg agagcaatat cgttgaaggt 960

agacaggatg ttcccaaaga gggaaagtca catctacgtt atggatgttc cataactgat 1020

gcctgtatct cctgggagga tactgagaag gttctacgag tcttagcaga ttcggtcatt 1080

gaacgtcgta atctcaagaa ttag 1104

<210> 20
<211> 849
<212> DNA
<213> Pichia pastoris

<400> 20
atggagttca agaaacccgc aacagtgctg aatctggcca atatcagaca ggccttggtc 60

cgaatggaag acaccgtggt gttcagcttc atcgaaaggt cccagttcta cgaatctcca 120

tccgtgtacg cccccaataa gtacaagatc cccaactttt caggttcttt cttggactgg 180

ttgctcttgc aaaacgagaa gacccactcg cttgtcaggc gttacgagtc tccagacgag 240

accccctttt tccctgatca actggaagaa tcgtttcttc caagtctaaa ctaccctcca 300

attttggctg actacgccga tgaggtgaac gtaaatgacg aaatcagaac tgtgtacatt 360

gaaaagatag ttccccaaat tgcagctaag aaaggtgatc aagatgagaa tattggatcc 420

acagcctgtg cagatgttga ttgtttgcag gctttgagtc gtcgagtgca ttttggaaaa 480

tttgtcgcag aagctaagtt ccaaaatgaa atggcccgct acactaattt gatccgcaac 540

aaggacatta agggcattga agaagctatt acagattccg cagttgaagc taagattttg 600

gaacgtttaa ttgccaaagg tcatgcctat ggtacggacc ccacgcttag gtattcgcag 660

aaccctcaat caaaagtaca gccagaggca attgctaaga tatacaaaga ggtagttata 720

cctctgacca agaaggtgga ggttgaatat ctgcttagaa ggttaggaga tgacgacgaa 780

gcagaagctc aagaccctca acgcaagact cagctgatga acagactatt atccaacagc 840

ttgtggtaa 849

<210> 21
<211> 1197
<212> DNA
<213> Pichia pastoris

<400> 21
atgtccgtaa gaaatgccac ttttcgttta agccgtgctg ctattgctaa gagaactttg 60

gtcagaattg cttctcaagc ctcattaaga ccaacgacac tgaattctgc cactggagtc 120

gctcgttcgc agttgacttt tagtcgtggt gtgaaaacca ttaattttgg tggtgtcgaa 180

gaagttgtcc acgagagatc tgattggcca agagagaaac tgctagagta tttcaagaat 240

gacactttag ctttaattgg ctacggatct cagggttatg gtcaaggatt aaatctaaga 300

gataatggtc tcaatgttat tattggtgtc cgaaagaacg gcgcttcttg gaaggctgct 360

attgaggatg gctgggttcc tggtgaaaac ctttttgatg tcactgaagc tataaaaaag 420

ggttctatca tcatgaatct tttgtctgac gccgcccaat ctgagacttg gtctaccgtc 480

aaacccttat tgactgaagg taaaacttta tacttttctc acggattttc tccagttttc 540

tcaaacctta cccatgtaga gccaccttcc aacattgacg ttattttagc tgctcctaaa 600

ggttcaggta gaactgttcg ttcccttttt aaagagggac gtggaatcaa ctcttcctat 660

gctgtttgga acgacgtcac cggaaaagct gaagaaaaag ccattgcttt ggctgtagca 720

gtcggatctg gatacgttta tcagaccaca tttgagcgtg aagttaactc cgacttgtac 780

ggtgagagag gttgtttaat gggaggtatc catggtatgt tttggcaca atacgaggtt 840

ctaagagaga atggacatac gccatcagaa gctttcaacg aaaccgtcga agaggctact 900

cagtctttgt atcccttggt tggaaagtat ggtatggatt acatgtatga tgcctgctcc 960

actactgctc gtagaggtgc gttagactgg tatccaattt tcaaggatgc cctcaagcca 1020

gtgttcgaag atctttacaa ttcagtaaag acaggtaagg aaactcaacg ttctcttgac 1080

ttcaattcac aacctgatta tagggagaaa cttgaaaaag aactggaaat tatccgtaat 1140

atggagattt ggaaggttgg aaaggaagtc cgcaaactac gtccagaaaa caactga 1197

<210> 22
<211> 1716
<212> DNA
<213> Pichia pastoris

<400> 22
atgcctatgc taaaggaccc ctccaagaaa tacgttgcct tcaagccgct taacctgcct 60

gacagacaat ggccatcaaa aactcttcaa gcccctccaa gatggttatc cacagatttg 120

agagatggta accaatctct gccagacccc atgagtgtgg ccgagaaaaa agaatacttc 180
aacaagctgg ttcagatcgg gttcaaggag attgaagttg ctttcccttc agcttcgcag 240
actgactttg actttaccag atatgcggtt gagaatgctc cagcagacgt agctattcaa 300
gttctgtccc catgcagacc tgagttgatc aagagaactg ttgagtcctt gaaaggtgcc 360
aagaaagcaa ttgtacacat ttatttggct acttcagact gtttcagaaa cattatattt 420
ggcctttccc aggaagaatc tcttgaaatg gcagtaaact gcacgaaact ggttagagag 480
ttgaccaagg atgctcctga aatgcaagat accgtatggg gattcgaatt ctctccggag 540
acatttagtg atactgatcc ggactatgct ctgcgtgttt gcgaggccgt gaaggctgct 600
tggggcccat cagaagaaaa cccaatcata ttcaaccttc ctgctactgt tgaaatgtct 660
actccaaacg tctacgctga ccaaattgaa tatttttcta gaaacatcag tgagagaaag 720
acagtagcaa tatccctgca cccacacaat gaccgtggtt gtgccgtagc tgcagctgaa 780
ttaggacaga tggctggagc tgacagaata gagggttgtc tgttcggaaa cggtgaaaga 840
accggtaacg ttgacttggt aactctggcc cttaacttgt acactcaggg agtttaccct 900
catttggact tttcagacat tacttcagta attgacatcg ttgagcgatg caacaagatc 960
ccagttcatc caagagcccc atacggaggt caactggtgg tctgcgcatt ctctgggtcc 1020
caccaagacg ctattaagaa ggggttcaag aaacaggaag aaagacaggc caaggatccc 1080
aacgccagat ggcagctacc ttatcttcca ttggatcctc aagatattgg aaggacttac 1140
gaggctatca tcagagtcaa ctctcaatct ggaaagggtg gtgcttcatg ggttattctt 1200
agaaacttgg aattggatct tcctcgtgct ttgcaagttt ccttctctaa gatcgtccaa 1260
aatgagtcag aagttaaagg aagagagttg tacaacaaag agatcacaaa cctgttcaag 1320
aactcttact ttgttgatga tgatgacacc cagcacgatc tttacttcca actggtagat 1380
tactctatta caaatacatc caaaagcgct agactgatca acgttgaact ccaagttgga 1440
aaagacactg tttccttaaa aggtattggt aacggtccaa tttcctcatt tgccagcgct 1500
atttcttctt acctgaaaaa agatgtactg gtcttgaact accatgaaca ctctttagga 1560
aaagattcca actccaaggc cgctacatac ttacactgtg ccattggcga tgactgcaag 1620
gtttggggtg ttggtatcca tgaagatgtt tctcaggcat cattcttatc tttcatcagt 1680
cttttgaact ctgcaaggag agatggtaaa atataa 1716

<210> 23
<211> 1896
<212> DNA
<213> Pichia pastoris

<400> 23
atgagctctc tttacatgcc ctcaaaatca atgaaacggc ttactgtgag ctgtcgtcgt 60

tttatatctt cggctcaacc aaaaattgtt acacacaaaa tactcacacc agttgggaca 120
gaagcggcag ccaaagtaga tgttcccaag acgacccgtc tttctgtgga aggccctatc 180
gaatgccact tgcaaggatt tgagctatta aactctccat tgtttaacaa gggttctgcg 240
ttcactccag aggaaagagc agcctttggg ttagaagggt tacttcctcc agtagccaat 300
gacttaaacg aacaagcaga gagagcttac aagcagctgt ctttcttgaa aaccccacta 360
gcaaagaatg atttttgtac ctccatgaga cagcaaaata aggttctttt ctacgagttg 420
gtgaggcgac acattagaga gctactgccc atcatctata ctcctacgga gggggatgcc 480
attgccgcat attcacatag attccgtaaa cctgagggtt gtttccttga tgtcacggac 540
cctgaatcca tagaggatcg tttatctcgt ttcggtgaga gtaaagatgt ggattatatt 600
gtagtttctg acggagaagg aattttggga attggtgatc agggtatagg tggtgttcgt 660
attgccattt ccaagttagc tttaatgact ctttgcggtg gtattcatcc tggaagagtt 720
atccctgtcg ttgttgatgc cggtacaaac aataaggctc ttctggatga cgaactatac 780
atgggtagcc gcttccctcg agttcgtggt gaagagtact attctttttt tgacaagttt 840
attgaagttg tcaagcgtag attcccatct gcagtgttgc attttgaaga tttcggtgta 900
actaatgcca agcccctatt ggacaagtat cgttccgttc ttccttgctt caatgacgac 960
attcaaggta ctggggccgt tgtaatggca tctatggcag ccgcattgaa acttacgaat 1020
cgagatttgt tagattccac catcgttatt tatggcgctg gttctgctgg tcttggaatt 1080
gctttccaga tcgtcaatca catggttaac catggtgcca ctaaagaaga agcttactca 1140
aggattcatt tgctagatcg tcatggcttg attctcacaa acatgagcga tgtatccaac 1200
aaagatcaaa tgctctttgc ggatgatcct agcagctggg atggtattaa taccaaatcc 1260
ctagttgatg tcatcgagaa actcaaaccc accacattaa ttggttgctc cacccaagcc 1320
ggagccttca cagaggaagt cattaagaca atgtacaaat acaaccccag gccaatagtg 1380
ttccctcttt ccaaccctac tagacttcat gaagctgttc ctgttgatgt tatgcgatgg 1440
acggatgata atgccctaat tgctactggt tctcccttta agcctgttcg tggtcatgtc 1500
atcagtgaaa acaataactg cttctcattc ccaggtatcg gattgggagc cgttctttct 1560
agagctacaa ccatctccga cactatgatt tccgctgcag ttgatgaact tgccagtggt 1620
tcccctgcat tcaaggaccc taaagcaggt ttgcttgccc cagtcgaagt gataaatgag 1680
acatctgcta aagtggcagc cgccttaatc cttcaaagta tcaaggaagg cactgccaga 1740
gtggaagaag agctgtctcc aaatggagat aaagtttctg tccctagaga ctttgagggt 1800
tgtgttgcat gggtcaaatc ccaaatgtgg cgaccagaat atagacccat ggtgaaggtg 1860
gaacgagttc ccgaaatcca tacgcatcag tcatag 1896

<210> 24
<211> 1356
<212> DNA
<213> Pichia pastoris

<400> 24
atgggtgaaa attgttacac tatcgtcgtt aaattgggaa ccagttccgt ggtagatgaa 60

gaaaccaggg aaccaagaat cgcaactatg tctcgtattg tcgagaccct tgttaagctg 120

cgaaggaagg gccatcgaat tgtcatcgtc tcctcaggtg ccatcgctgt gggcatgaag 180

agagtcaaca tgaagcggaa accaaaaaag ttacagcaag tgcaggcatt ggctgctata 240

ggacaaggcc gtttgatagg actttgggac gaccttttcc gtcagttgaa tcagcctatt 300

gcgcagattt tactgactag aacggatttg gtcgattaca cccagtttaa gaacgctgaa 360

aatacattgg aacagcttat taaaatgggt attattccta ttgtcaatga gaatgacacc 420

ctatccattc aagaaatcaa atttggtgac aatgacacct tatccgccat aacagctggt 480

atgtgtcatg cagactacct gtttttggtg actgatgtgg actgtcttta cacggataac 540

cctcgtacga atccggacgc tgagccaatc gtgttagtta gaaatatgag gaatctaaac 600

gtcaataccg aaagtggagg ttccgccgta ggaacaggag gaatgacaac taaattgatc 660

gcagctgatt tgggtgtatc tgcaggtgtt acaacgatta tttgcaaaag tgaacatccc 720

gagcagattt tggacattgt agagtacagt atccgtgctg atagagtcga aaatgaggct 780

aaatatctgg tcatcaacga agaggaaact gtggaacaat ttcaagagat caatcggtca 840

gaactgaggg agttgaacaa gctggacatt cctttgcata cacgtttcgt tggccacagt 900

tttaatgctg ttaataacaa agagttttgg ttactccatg gactaaaggc caacggagcc 960

attatcattg atccaggttg ttataaggct atcactagaa aaaacaaagc tggtattctt 1020

ccagctggaa ttatttccgt agagggtaat ttccatgaat acgagtgtgt tgatgttaag 1080

gtaggactaa gagatccaga tgacccacat tcactagacc ccaatgaaga actttacgtc 1140

gttggccgtg cccgttgtaa ttaccccagc aatcaaatca acaaaattaa gggtctacaa 1200

agctcgcaga tcgagcaggt tctaggttac gctgacggtg agtatgttgt tcacagggac 1260

aacttggctt tcccagtatt tgccgatcca gaactgttgg atgttgttga gagtaccctg 1320

tctgaacagg agagagaatc caaaccaaat aaatag 1356

<210> 25
<211> 825
<212> DNA
<213> Pichia pastoris

<400> 25
atgtcttcaa ttggtaacga atatacaatc actatcctag ggtgcggagt gatgggaacc 60

gcagttttat ctgctatttt gaactcagag ttctcccctt atccctcaag aattatctgc 120

tgcacaggct cccagaagtc cgctgaaaaa ttggccagca cttattccaa gattgaaact 180

agctacggat ctgagcaaaa catcagagcc gtcaaagagt cagatatcat aattctaggg 240

tgcaagccgt tctattgtga acaaatcatg gaacaggtca aagagggctt acacgatcaa 300

ttgctaattt cacttttggc tggctggacg ttaacccaac tagaaaggta ctctaaatat 360

gtagcaaggg taatgaccaa taccccggcc aaatttggat gtggaatggc ttgtgtatct 420

ctatcttcgc acgccgagca atacgaagat ctagtactca agctggttgg cccagtgggc 480

aaggctatta gattgcccga aaagaatatg gatgccgcta cagctcttgt aggttcaggt 540

cctgcattct gcttgttgat gatggaatct ctcatcgacg gagccgtcca aaagggttta 600

ccatttgcta ttgctaaaga tgcagctgct aaagttatgg aaggcacagc aaggatggtt 660

ctcgagactg gagaacatcc tgccgcacta aaaagtgctg tgtgcacccc aggaggtacc 720

actattggtg gtcttatggc catggaagac cgaggagtca gaagtggtat cgctcgaggt 780

gtggaagaag ctgccaacat tgccactagc ttaggaaaga aatag 825

<210> 26
<211> 1329
<212> DNA
<213> Pichia pastoris

<400> 26
atgccttctc acttcgacac tttgcagctg cacgccggtc agaccgctga agctccacac 60

aatgccagag ctgttcctat ctacgctacc tcgtcttacg ttttcagaga ctctgagcac 120

ggtgccaagc tgttcggttt ggaggagcca ggttacatct actctcgttt gatgaaccct 180

actcagaacg tctttgaaga gagaattgcc gctttggagg gtggtgccgc tgctttggct 240

gttggatccg gtcaagctgc tcaattcctg gctattgctg gtttggctca cactggtgac 300

aacgtcatct ccacctcttt cttgtacggt ggaacttaca accaattcaa ggtcgccttc 360

aagagactgg gaattgaatc cagatttgtc catggtgatg acccagctga attcgagaga 420

ctgatcgatg ataagaccaa ggccatctac gttgagtcca ttggtaaccc aaagtacaat 480

attccagatt ttgaggctct cgcagagctt gcccacaagc acggtatccc attagttgtt 540

gacaacacct ttggtgccgg tggttactac gttagaccaa tcgagcttgg tgctgacatc 600

gtcacccact ccaccactaa gtggatcaat ggtcacggta acaccatcgg tggtgttgtc 660

gttgactctg gtaagttccc atggaaagac tacccagaga agtaccctca attctccaag 720

ccatctgagg gttaccacgg tttgatcttg aatgacgcct ttggaccagc tgccttcatt 780

ggtcacttga gaactgaact gctaagagat ttgggtcctg cttcaagtcc attcggtaac 840

ttcttgaaca taatcggttt ggagacctta tctctgagag ctgagagaca cgctgagaat 900

gctttgaagc tggccaaata cttggaaacc tctccatacg tcagctgggt ctcttaccct 960

```
ggtttggagt ctcacgacta ccacgaggcc gctaagaagt acttgaagaa cggtttcggt      1020

gctgtattgt cttttggagt caaggatcat ggcaagccag cgctcactcc cttcgaagag      1080

gctggtccta aggttgtaga ctccctgaag gttttctcca acttggctaa cgttggtgac      1140

tccaagtctt tgatcattgc tccttactac actactcacc aacagttgtc tcacgaggag      1200

aagctggctt ccggtgtcac caaggactct atccgtgttt ctgtcggaac agagttcatc      1260

gatgatctta ttgcagacct tgaacaggcc tttgcccttg tttacgagga ggcaaacaca      1320

aagttgtga                                                              1329


<210>  27
<211>  1506
<212>  DNA
<213>  Pichia pastoris

<400>  27
atgtcagaca acaaccaacc tttgccaccg gtttccaact ccattctgga gcacattgga        60

aagactccac ttgttaaact taacagaata cccaaaattc ttaatttgaa accacaggtt       120

tatgctaaag ttgaactgtt caactcaggt ggttccatca aggaccgtat tgctctgaga       180

atggttgagc aagctgagaa agagggtaga atcaagccag ggtacacgct aattgagcca       240

acgtcaggta acactggtat tggtctggca ttagtaggtg cagtcaaggg ctacagaact       300

attatcactc ttccagagaa aatgtctaat gaaaaagttg ctgtgctgaa agctctcggg       360

gctgaaatta taagaactcc aaccgaggct gcttgggatt ccccagagtc tcacattggt       420

gttgcaagaa ggttggagaa agaaattcca aatgctgtaa tcttagacca gtatggtaat       480

gttaacaatc cagatgccca tttctacacc actggtgcgg aaatctggga tcagactaat       540

ggtaaagtta cccacgttgt tgctggtgca ggaactggag gtacgattac aggtattgcc       600

aaatttttga agtctaaaaa ccctaatatt caaattatcg gtgctgatcc tcatggttcc       660

attctagctc ttcctgaatc attgaacact agtaatgagc aatacaaagt tgaaggtatt       720

gggtatgact ttatcccaga agttttggat agagaaattg tggacacttg gtacaaaaca       780

gatgataagg attccttcaa gctggccagg caattgattt ctgaagaagg aattctagtt       840

ggaggatctt ccggctctgc gctttctgct gctgttaaaa taatcaatga tgccaagctt       900

gatgaaagtg ctgtagttgt tgtcgtttgt ccagacagta tcagatctta tctaaccaag       960

ttcgctgatg atgactggat gaaactcaat ggattcactg aagacgagcc tgcacctctc      1020

aaaaggaagt ccagcttttc aaaggacact ctgtcgtcct atacgatcaa agatttggcg      1080

ctcaaacctg tggtctctgt caccattgac gattcaattg aatccacaat taatatcctt      1140

caaaccaagg gttttgatca gttaccagtg ttgaagaacg ataagcttgc tggtattgtc      1200

actctgtctc aattgttacg attactttct aataagaaga ttcatcttac ttccaaggta      1260
```

gaatctgcct actttgattt ttccaagctt gaaaactttg aaaaatctta caacattaac 1320

aaggagtcaa caaacaagag aagagaatac cagaagatca ctactagcac cacgctggcc 1380

aaattgaaca agttttttga gaccagttca gctgctatag tagaaaatga taagggtcaa 1440

ccgatccaca tcgtttccaa ggttgatctg ctttctttct tagcctccaa aggagagttt 1500

aattaa 1506

<210> 28
<211> 1071
<212> DNA
<213> Pichia pastoris

<400> 28
atgaagaaag caggtgtttt gggcgcaacc ggaagtgttg gccaacgttt tatactttta 60

ctaagcgatc atccagagtt tgagttgtct gttctcggag cttcctctcg ctctgctgga 120

aagaggtatg aagatgccgt cgtctggaag caaactgatc ttttgcctga gtccgcaaga 180

aatatcattg ttcaagagtg caaagctgag gctttcaagg actgtgacat tatcttcagt 240

gggttagacg ccgattatgc tggagaaatt gaaaaggaat ttgtttctag tggtttagct 300

gttgtctcta acgcaaagaa ctacagaaga gaggcagacg ttcctttgat cgttcctatt 360

gtcaatccag agcatctgga aattgttgaa cagaaagtta agtctggaca aaagggattc 420

caaatttgca tttctaactg ttccactgct gggctcgttg ctcctttgaa acctctggtg 480

gagaagtttg gaccaattga cctcctgaca actactactt tacaggctat ttccggagcc 540

ggattttctc caggtgttcc aggaatagat atattagaca atattgtacc attcatcagt 600

ggtgaagaag agaaaatgga atgggagacc agaaagattt tgggaggact ctccgaggat 660

aagatggaat gcagacttgt tagtgaagac gcaatgaaag tttccgctca atgtaataga 720

gttcctgtaa ttgatggtca tactgagtgc atttccctga gattcgaacg aagacccccct 780

ccaagtgttg aggcagtcaa gcaatgtctt agagactacg tttgtgaggc cagcaaattg 840

ggctgtccat ccgctcccaa gaacaccatt cacgttttgg aacaaaatga ccgaccacag 900

ccaaggttag accgtgtacg tgataatggg tttggagtta gcgtcggcag aatcagagag 960

gatccagtgt tagatttcaa gatggtagta ttgtcccaca acacaatcat cggagctgct 1020

ggttctggag tactcattgg agagatcctg ttgaaaaagg gtctcattta a 1071

<210> 29
<211> 540
<212> PRT
<213> Sphingopyxis macrogoltabida

<400> 29

Met Lys Glu His Gln Cys Arg Gly Gly Arg Ala Ser Pro Ala Ala Pro
1 5 10 15

```
Ala Thr Trp Leu Ala Arg Ile Ser Val Ser Arg Gly Ala Ser Ala Ile
            20                  25                  30

Ala Trp Thr Phe Met Leu Gly Ala Thr Ala Ile Pro Val Ala Ala Gln
        35                  40                  45

Thr Asp Asp Pro Lys Leu Val Arg His Thr Gln Ser Gly Ala Val Glu
    50                  55                  60

Gly Val Glu Gly Asp Val Glu Thr Phe Leu Gly Ile Pro Phe Ala Ala
65                  70                  75                  80

Pro Pro Val Gly Asp Leu Arg Trp Arg Pro Pro Ala Pro Pro Arg Ala
                85                  90                  95

Trp Ala Gly Thr Arg Asp Gly Arg Arg Phe Ala Pro Asp Cys Ile Gly
            100                 105                 110

Asn Glu Arg Leu Arg Glu Gly Ser Arg Ala Ala Gly Thr Ser Glu Asp
        115                 120                 125

Cys Leu Tyr Leu Asn Ile Trp Ser Pro Lys Gln Val Gly Lys Gly Gly
    130                 135                 140

Leu Pro Val Met Ile Trp Val Tyr Gly Gly Gly Phe Ser Gly Gly Ser
145                 150                 155                 160

Gly Ala Val Pro Tyr Tyr Asp Gly Ser Ala Leu Ala Gln Lys Gly Val
                165                 170                 175

Val Val Val Thr Phe Asn Tyr Arg Ala Gly Ile Leu Gly Phe Leu Ala
            180                 185                 190

His Pro Ala Leu Ser Lys Glu Ser Pro Asn Gly Val Ser Gly Asn Tyr
        195                 200                 205

Gly Leu Leu Asp Met Leu Ala Ala Phe Lys Trp Val Gln Asn Asn Ile
    210                 215                 220

Arg Glu Phe Gly Gly Asp Pro Asn Arg Val Thr Val Phe Gly Glu Ser
225                 230                 235                 240

Ala Gly Ala Ser Ala Leu Gly Leu Leu Leu Thr Ser Pro Leu Ser Glu
                245                 250                 255

Ser Ala Phe Asn Gln Ala Ile Leu Gln Ser Pro Gly Leu Ala Arg Pro
```

260 265 270

Leu Ala Thr Leu Ser Glu Ser Glu Ala Asn Gly Leu Glu Leu Gly Ala
275 280 285

Asp Ile Ser Ala Leu Arg Arg Ala Asp Ala Gly Glu Leu Thr Lys Ile
290 295 300

Ala Gln Ser Arg Ile Pro Met Ser Arg Gln Phe Thr Lys Pro Arg Pro
305 310 315 320

Met Gly Pro Ile Leu Asp Gly Tyr Val Leu Arg Thr Leu Asp Val Asp
325 330 335

Ala Phe Ala Lys Gly Ala Phe Arg Lys Ile Pro Val Leu Val Gly Gly
340 345 350

Asn Ala Asp Glu Gly Arg Ala Phe Thr Asp Arg Leu Pro Val Lys Thr
355 360 365

Val Leu Glu Tyr Arg Ala Tyr Leu Thr Glu Gln Phe Gly Asp Glu Ala
370 375 380

Asp Ala Trp Glu Arg Cys Tyr Pro Ala Asn Ser Asp Ala Asp Val Pro
385 390 395 400

Ala Ala Val Ala Arg Leu Phe Gly Asp Ser Gln Phe Asn Asn Gly Ile
405 410 415

Glu Leu Leu Ser Ala Ala Phe Ala Lys Trp Arg Thr Pro Leu Trp Arg
420 425 430

Tyr Arg Phe Thr Gly Ile Pro Gly Ala Gly Arg Arg Pro Ala Thr His
435 440 445

Gly Asp Glu Ile Pro Tyr Val Phe Ala Asn Leu Gly Pro Ser Ser Val
450 455 460

Ser Met Phe Gly Ser Leu Glu Gly Gly Ala Gly Ala Ser Asp Ile Lys
465 470 475 480

Leu Ala Thr Glu Met Ser Ala Ala Trp Val Ser Phe Ala Val His Gly
485 490 495

Val Pro Asp Gln Gly Thr Lys Ser His Trp Pro Arg Phe Glu Arg Arg
500 505 510

Gly Glu Ile Met Thr Phe Gly Ser Gln Val Gly Ser Gly Glu Gly Leu
515 520 525

Gly Val Ser Pro Ser Lys Ala Cys Gln Pro Ser Lys
530 535 540

<210> 30
<211> 303
<212> PRT
<213> artificial

<220>
<223> synthetic construct (HyHEL, HC)

<400> 30

Met Arg Phe Pro Ser Ile Phe Thr Ala Val Leu Phe Ala Ala Ser Ser
1 5 10 15

Ala Leu Ala Ala Pro Val Asn Thr Thr Thr Glu Asp Glu Thr Ala Gln
20 25 30

Ile Pro Ala Glu Ala Val Ile Gly Tyr Ser Asp Leu Glu Gly Asp Phe
35 40 45

Asp Val Ala Val Leu Pro Phe Ser Asn Ser Thr Asn Asn Gly Leu Leu
50 55 60

Phe Ile Asn Thr Thr Ile Ala Ser Ile Ala Ala Lys Glu Glu Gly Val
65 70 75 80

Ser Leu Glu Lys Arg Asp Val Gln Leu Gln Glu Ser Gly Pro Ser Leu
85 90 95

Val Lys Pro Ser Gln Thr Leu Ser Leu Thr Cys Ser Val Thr Gly Asp
100 105 110

Ser Ile Thr Ser Asp Tyr Trp Ser Trp Ile Arg Lys Phe Pro Gly Asn
115 120 125

Arg Leu Glu Tyr Met Gly Tyr Val Ser Tyr Ser Gly Ser Thr Tyr Tyr
130 135 140

Asn Pro Ser Leu Lys Ser Arg Ile Ser Ile Thr Arg Asp Thr Ser Lys
145 150 155 160

Asn Gln Tyr Tyr Leu Asp Leu Asn Ser Val Thr Thr Glu Asp Thr Ala
165 170 175

Thr Tyr Tyr Cys Ala Asn Trp Asp Gly Asp Tyr Trp Gly Gln Gly Thr

```
            180                 185                 190

Leu Val Thr Val Ser Ser Ala Ser Thr Lys Gly Pro Ser Val Phe Pro
        195                 200                 205

Leu Ala Pro Ser Ser Lys Ser Thr Ser Gly Gly Thr Ala Ala Leu Gly
    210                 215                 220

Cys Leu Val Lys Asp Tyr Phe Pro Glu Pro Val Thr Val Ser Trp Asn
225                 230                 235                 240

Ser Gly Ala Leu Thr Ser Gly Val His Thr Phe Pro Ala Val Leu Gln
                245                 250                 255

Ser Ser Gly Leu Tyr Ser Leu Ser Ser Val Val Thr Val Pro Ser Ser
            260                 265                 270

Ser Leu Gly Thr Gln Thr Tyr Ile Cys Asn Val Asn His Lys Pro Ser
        275                 280                 285

Asn Thr Lys Val Asp Lys Arg Val Glu Pro Lys Ser Cys Asp Lys
    290                 295                 300


<210>  31
<211>  299
<212>  PRT
<213>  artificial

<220>
<223>  synthetic construct (HyHEL, LC)

<400>  31

Met Arg Phe Pro Ser Ile Phe Thr Ala Val Leu Phe Ala Ala Ser Ser
1               5                   10                  15

Ala Leu Ala Ala Pro Val Asn Thr Thr Thr Glu Asp Glu Thr Ala Gln
            20                  25                  30

Ile Pro Ala Glu Ala Val Ile Gly Tyr Ser Asp Leu Glu Gly Asp Phe
        35                  40                  45

Asp Val Ala Val Leu Pro Phe Ser Asn Ser Thr Asn Asn Gly Leu Leu
    50                  55                  60

Phe Ile Asn Thr Thr Ile Ala Ser Ile Ala Ala Lys Glu Glu Gly Val
65                  70                  75                  80

Ser Leu Glu Lys Arg Asp Ile Val Leu Thr Gln Ser Pro Ala Thr Leu
                85                  90                  95
```

Ser Val Thr Pro Gly Asn Ser Val Ser Leu Ser Cys Arg Ala Ser Gln
            100                 105                 110

Ser Ile Gly Asn Asn Leu His Trp Tyr Gln Gln Lys Ser His Glu Ser
        115                 120                 125

Pro Arg Leu Leu Ile Lys Tyr Ala Ser Gln Ser Ile Ser Gly Ile Pro
    130                 135                 140

Ser Arg Phe Ser Gly Ser Gly Ser Gly Thr Asp Phe Thr Leu Ser Ile
145                 150                 155                 160

Asn Ser Val Glu Thr Glu Asp Phe Gly Met Tyr Phe Cys Gln Gln Ser
                165                 170                 175

Asn Ser Trp Pro Tyr Thr Phe Gly Gly Gly Thr Lys Leu Glu Ile Lys
            180                 185                 190

Arg Thr Val Ala Ala Pro Ser Val Phe Ile Phe Pro Pro Ser Asp Glu
        195                 200                 205

Gln Leu Lys Ser Gly Thr Ala Ser Val Val Cys Leu Leu Asn Asn Phe
    210                 215                 220

Tyr Pro Arg Glu Ala Lys Val Gln Trp Lys Val Asp Asn Ala Leu Gln
225                 230                 235                 240

Ser Gly Asn Ser Gln Glu Ser Val Thr Glu Gln Asp Ser Lys Asp Ser
                245                 250                 255

Thr Tyr Ser Leu Ser Ser Thr Leu Thr Leu Ser Lys Ala Asp Tyr Glu
            260                 265                 270

Lys His Lys Val Tyr Ala Cys Glu Val Thr His Gln Gly Leu Ser Ser
        275                 280                 285

Pro Val Thr Lys Ser Phe Asn Arg Gly Glu Cys
    290                 295

<210> 32
<211> 312
<212> PRT
<213> artificial

<220>
<223> synthetic construct (SDZ-Fab, HC)

<400> 32

Met Arg Phe Pro Ser Ile Phe Thr Ala Val Leu Phe Ala Ala Ser Ser
1 5 10 15

Ala Leu Ala Ala Pro Val Asn Thr Thr Thr Glu Asp Glu Thr Ala Gln
20 25 30

Ile Pro Ala Glu Ala Val Ile Gly Tyr Ser Asp Leu Glu Gly Asp Phe
35 40 45

Asp Val Ala Val Leu Pro Phe Ser Asn Ser Thr Asn Asn Gly Leu Leu
50 55 60

Phe Ile Asn Thr Thr Ile Ala Ser Ile Ala Ala Lys Glu Glu Gly Val
65 70 75 80

Ser Leu Glu Lys Arg Glu Val Gln Leu Val Gln Ser Gly Gly Gly Leu
85 90 95

Val Gln Pro Gly Gly Ser Leu Arg Leu Ser Cys Ala Ala Ser Gly Phe
100 105 110

Thr Phe Ser His Tyr Trp Met Ser Trp Val Arg Gln Ala Pro Gly Lys
115 120 125

Gly Leu Glu Trp Val Ala Asn Ile Glu Gln Asp Gly Ser Glu Lys Tyr
130 135 140

Tyr Val Asp Ser Val Lys Gly Arg Phe Thr Ile Ser Arg Asp Asn Ala
145 150 155 160

Lys Asn Ser Leu Tyr Leu Gln Met Asn Ser Leu Arg Ala Glu Asp Thr
165 170 175

Ala Val Tyr Phe Cys Ala Arg Asp Leu Glu Gly Leu His Gly Asp Gly
180 185 190

Tyr Phe Asp Leu Trp Gly Arg Gly Thr Leu Val Thr Val Ser Ser Ala
195 200 205

Ser Thr Lys Gly Pro Ser Val Phe Pro Leu Ala Pro Cys Ser Arg Ser
210 215 220

Thr Ser Glu Ser Thr Ala Ala Leu Gly Cys Leu Val Lys Asp Tyr Phe
225 230 235 240

Pro Glu Pro Val Thr Val Ser Trp Asn Ser Gly Ala Leu Thr Ser Gly
245 250 255

```
Val His Thr Phe Pro Ala Val Leu Gln Ser Ser Gly Leu Tyr Ser Leu
            260                 265                 270

Ser Ser Val Val Thr Val Pro Ser Ser Ser Leu Gly Thr Lys Thr Tyr
        275                 280                 285

Thr Cys Asn Val Asp His Lys Pro Ser Asn Thr Lys Val Asp Lys Arg
    290                 295                 300

Val Glu Ser Lys Tyr Gly Pro Pro
305                 310


<210>  33
<211>  299
<212>  PRT
<213>  artificial

<220>
<223>  synthetic construct (SDZ-Fab, LC)

<400>  33

Met Arg Phe Pro Ser Ile Phe Thr Ala Val Leu Phe Ala Ala Ser Ser
1               5                   10                  15

Ala Leu Ala Ala Pro Val Asn Thr Thr Thr Glu Asp Glu Thr Ala Gln
            20                  25                  30

Ile Pro Ala Glu Ala Val Ile Gly Tyr Ser Asp Leu Glu Gly Asp Phe
        35                  40                  45

Asp Val Ala Val Leu Pro Phe Ser Asn Ser Thr Asn Asn Gly Leu Leu
    50                  55                  60

Phe Ile Asn Thr Thr Ile Ala Ser Ile Ala Ala Lys Glu Glu Gly Val
65                  70                  75                  80

Ser Leu Glu Lys Arg Ala Ile Gln Leu Thr Gln Ser Pro Ser Ser Leu
                85                  90                  95

Ser Ala Ser Val Gly Asp Arg Val Ile Leu Thr Cys Arg Ala Ser Gln
            100                 105                 110

Gly Val Ser Ser Ala Leu Ala Trp Tyr Gln Gln Lys Pro Gly Lys Ala
        115                 120                 125

Pro Lys Leu Leu Ile Tyr Asp Ala Ser Ser Leu Glu Ser Gly Val Pro
    130                 135                 140
```

Ser Arg Phe Ser Gly Ser Gly Ser Gly Pro Asp Phe Thr Leu Thr Ile
145 150 155 160

Ser Ser Leu Gln Pro Glu Asp Phe Ala Thr Tyr Phe Cys Gln Gln Phe
165 170 175

Asn Ser Tyr Pro Leu Thr Phe Gly Gly Gly Thr Lys Leu Glu Ile Lys
180 185 190

Arg Thr Val Ala Ala Pro Ser Val Phe Ile Phe Pro Pro Ser Asp Glu
195 200 205

Gln Leu Lys Ser Gly Thr Ala Ser Val Val Cys Leu Leu Asn Asn Phe
210 215 220

Tyr Pro Arg Glu Ala Lys Val Gln Trp Lys Val Asp Asn Ala Leu Gln
225 230 235 240

Ser Gly Asn Ser Gln Glu Ser Val Thr Glu Gln Asp Ser Lys Asp Ser
245 250 255

Thr Tyr Ser Leu Ser Ser Thr Leu Thr Leu Ser Lys Ala Asp Tyr Glu
260 265 270

Lys His Lys Val Tyr Ala Cys Glu Val Thr His Gln Gly Leu Ser Ser
275 280 285

Pro Val Thr Lys Ser Phe Asn Arg Gly Glu Cys
290 295

<210> 34
<211> 37
<212> DNA
<213> artificial

<220>
<223> synthetic construct

<400> 34
gatccctgca ggatgtctgc aagtacttac agttttg 37

<210> 35
<211> 42
<212> DNA
<213> artificial

<220>
<223> synthetic construct

<400> 35
gatcggccga ggcggcctca ctgatgtttc actaaactct tt 42

<210> 36
<211> 38
<212> DNA
<213> artificial

<220>
<223> synthetic construct

<400> 36
gatccctgca ggatgagagt taataaattt attcgtcc 38

<210> 37
<211> 42
<212> DNA
<213> artificial

<220>
<223> synthetic construct

<400> 37
gatcggccga ggcggcctca tttatacttt tgcatgaact tc 42

<210> 38
<211> 32
<212> DNA
<213> artificial

<220>
<223> synthetic construct

<400> 38
gatccctgca ggatggcaaa aactttggaa ag 32

<210> 39
<211> 37
<212> DNA
<213> artificial

<220>
<223> synthetic construct

<400> 39
gatcggccga ggcggcctca agaatgagct tcagcaa 37

<210> 40
<211> 32
<212> DNA
<213> artificial

<220>
<223> synthetic construct

<400> 40
gatccctgca ggatggcttc tccccaagat at 32

<210> 41

<211> 38
<212> DNA
<213> artificial

<220>
<223> synthetic construct

<400> 41
gatcggccga ggcggcccta gtatagcaga cgggtgac 38

<210> 42
<211> 35
<212> DNA
<213> artificial

<220>
<223> synthetic construct

<400> 42
gatccctgca ggatgttcat tcaaaacgat catgt 35

<210> 43
<211> 41
<212> DNA
<213> artificial

<220>
<223> synthetic construct

<400> 43
gatcggccga ggcggcccta attcttgaga ttacgacgtt c 41

<210> 44
<211> 31
<212> DNA
<213> artificial

<220>
<223> synthetic construct

<400> 44
gatccctgca ggatggagtt caagaaaccc g 31

<210> 45
<211> 40
<212> DNA
<213> artificial

<220>
<223> synthetic construct

<400> 45
gatcggccga ggcggcctta ccacaagctg ttggataata 40

<210> 46
<211> 32
<212> DNA
<213> artificial

<220>
<223> synthetic construct

<400> 46
gatccctgca ggatgtccgt aagaaatgcc ac 32

<210> 47
<211> 38
<212> DNA
<213> artificial

<220>
<223> synthetic construct

<400> 47
gatcggccga ggcggcctca gttgttttct ggacgtag 38

<210> 48
<211> 34
<212> DNA
<213> artificial

<220>
<223> synthetic construct

<400> 48
gatccctgca ggatgcctat gctaaaggac ccct 34

<210> 49
<211> 42
<212> DNA
<213> artificial

<220>
<223> synthetic construct

<400> 49
gatcggccga ggcggcctta tattttacca tctctccttg ca 42

<210> 50
<211> 34
<212> DNA
<213> artificial

<220>
<223> synthetic construct

<400> 50
gatccctgca ggatgagctc tctttacatg ccct 34

<210> 51
<211> 40
<212> DNA
<213> artificial

<220>
<223> synthetic construct

<400> 51
gatcggccga ggcggcccta tgactgatgc gtatggattt 40

<210> 52
<211> 36
<212> DNA
<213> artificial

<220>
<223> synthetic construct

<400> 52
gatccctgca ggatgggtga aaattgttac actatc 36

<210> 53
<211> 41
<212> DNA
<213> artificial

<220>
<223> synthetic construct

<400> 53
gatcggccga ggcggcccta tttatttggt ttggattctc t 41

<210> 54
<211> 37
<212> DNA
<213> artificial

<220>
<223> synthetic construct

<400> 54
gatccctgca ggatgtcttc aattggtaac gaatata 37

<210> 55
<211> 42
<212> DNA
<213> artificial

<220>
<223> synthetic construct

<400> 55
gatcggccga ggcggcccta tttctttcct aagctagtgg ca 42

<210> 56
<211> 32
<212> DNA
<213> artificial

<220>
<223> synthetic construct

<400> 56
gatccctgca ggatgccttc tcacttcgac ac 32

<210> 57
<211> 38
<212> DNA
<213> artificial

<220>
<223> synthetic construct

<400> 57
gatcggccga ggcggcctca caactttgtg tttgcctc 38

<210> 58
<211> 33
<212> DNA
<213> artificial

<220>
<223> synthetic construct

<400> 58
gatccctgca ggatgtcaga caacaaccaa cct 33

<210> 59
<211> 40
<212> DNA
<213> artificial

<220>
<223> synthetic construct

<400> 59
gatcggccga ggcggcctta attaaactct cctttggagg 40

<210> 60
<211> 33
<212> DNA
<213> artificial

<220>
<223> synthetic construct

<400> 60
gatccctgca ggatgaagaa agcaggtgtt ttg 33

<210> 61
<211> 40
<212> DNA
<213> artificial

<220>
<223> synthetic construct

<400> 61
gatcggccga ggcggcctta aatgagaccc tttttcaaca 40